(19) Europäisches Patentamt
European Patent Office
Office européen des brevets

(11) EP 1 939 197 A1

(12) EUROPEAN PATENT APPLICATION

(43) Date of publication:
**02.07.2008 Bulletin 2008/27**

(21) Application number: **06026739.0**

(22) Date of filing: **22.12.2006**

(51) Int Cl.:
***C07D 473/06*** *(2006.01)* ***C07D 473/04*** *(2006.01)*
***A61K 31/495*** *(2006.01)* ***A61P 25/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **SCHWARZ PHARMA AG**
**40789 Monheim (DE)**

(72) Inventor: **The designation of the inventor has not yet been filed**

(74) Representative: **Hildebrand, Steven**
**Schwarz Pharma AG**
**Alfred-Nobel-Straße 10**
**D-40789 Monheim (DE)**

(54) **8-ethinylxanthine derivatives as selective A2A receptor antagonists**

(57) The present invention relates to novel 8-ethinylxanthirtes of formula (I), pharmaceutical compositions comprising such compounds, methods for preparing such compounds and methods for using these compounds, alone or in combination with other therapeutic agents for the alleviation, prevention and/or treatment of central nervous system (CNS) diseases, disorders and conditions, such as but not limited to Parkinson's disease.

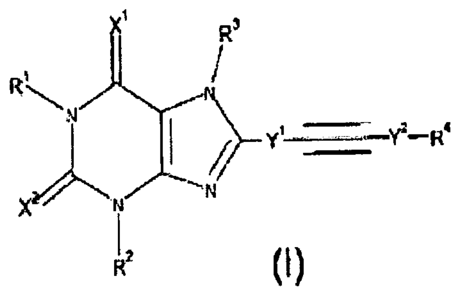

(I)

EP 1 939 197 A1

## Description

Adenosine receptors represent a subclass of the group of purine nucleotide and nucleoside G protein-coupled receptors known as purinoceptors; the main pharmacologically distinct adenosine receptor subtypes are known as $A_1$, $A_{2A}$, $A_{2B}$, and $A_3$. The dominant adenosine receptor subtypes in the brain are $A_1$ and $A_{2A}$. While the $A_1$ adenosine receptor subtype is found throughout the brain in high density, the distribution of the $A_{2A}$ receptor is more restricted; it is found in high density in the striatum (caudate-putamen, nucleus accumbens, olfactory tubercule), where it is co-colocalized with the dopamine D2 receptor on striatopallidal output neurons. The discrete localization of the $A_{2A}$ receptor within the striatum and its ability to functionally antagonize the actions of the D2 receptor has led to the suggestion of the potential utility of $A_{2A}$ receptor antagonists for the symptomatic treatment of Parkinson's disease (PD).

The first compounds that were identified as adenosine receptor antagonists were the naturally occurring xanthines, caffeine (1,3,7-trimethylxanthine) and theophylline (1,3-dimethylxanthine, Daly et al., Cell. Mol. Neurobiol., 1983, 3, 67). These xanthines have long been known to reverse motor deficits in a variety of PD models. However, they are non-selective and of moderate potency.

A variety of synthetic substitutions on the xanthine moiety led the chemists of Kyowa-Hakko to discover that introduction of the styryl group in the 8 position of xanthines was critical in achieving compounds endowed with selective $A_{2A}$ receptor antagonistic proper ties (Ongini et al., Trends Pharmacol. Sci., 1996, 17, 364; Shimada et al., J. Med. Chem., 1992, 36, 2343; Muller et al., Curr. Pharm. Des., 1996, 2, 501; Baraldi et al., Curr. Med. Chem., 1995, 2, 707). The results of this effort was the discovery of the structurally related compounds KF17837, (*E*)1,3-dipropy(-8-(3,4-dimethoxystyryl)-7-methyl-xanthine, and its analog KW6002 (istradefylline), (*E*)1,3-diethyl-8-(3,4-dimethoxystyryl)-7-methyl-xanthine, whose pharmacological characteristics have been studied extensively. Despite having similar *in vitro* profiles, these two structurally similar xanthines appeared to have dramatically different *in vivo* potencies, as measured by the attenuation of haloperidol-induces catalepsy in mice, with KW6002 being clearly more potent. This divergence in *in vivo* activity may be due to differences in pharmacokinetics, pharmacodynamics, metabolism, and/or bioavailability (Kiec-Kononowicz et al., Pure and Appl. Chem., 2001, 73, 1411). KW6002 was chosen-by Kyowa-Hakko as a drug development candidate and has shown potency in a recently completed Phase II Clinical trial (now in Phase III trials) as a novel treatment for PD (Hauser et al., Neurology, 2003, 61, 297; Weiss et al., Neurology, 2003, 61, 101).

Further work on 8-substituted xanthines involved replacement of the styryl phenyl group with a heterocycle or replacement of the styryl double bond with its aza analogue (Del Giudice et al., Eur. J. Med. Chem., 1996, 31, 59), These compounds are also selective $A_{2A}$ receptor blockers.

Another approach used 3,7-dimethyl-1-propargylxanthine (DMPX) as a starting point for the development of $A_{2A}$ selective xanthines. In particular, 8-(*m*-Bromostyryl)-DPMX was found to be very potent and highly selective for $A_{2A}$ adenosine receptors (Muller et al., J. Med. Chem., 1997,40,4396).

However, the presence of the double bonds on the 8-position makes all these compounds photosensitive (Muller et al., Curr. Pharm. Des., 1996, 2, 501; Ongini et al., Trends Pharmacol. Sci., 1996, 17, 364). For example, short exposure of dilute solutions to daylight produces an equilibrium mixture of the *E*/Z isomers, where only the *E* form (while possibly being present as the minor component in the stable mixture) possesses high $A_{2A}$ receptor affinity. In order to avoid the complicating factor of isomerization, which is hard to prevent under normal laboratory conditions, configurationally stable analogs of 8-syrylxanthines were synthesized. Thereby, 8-(phenylethynyl)-DPMX was found as configurationally stable $A_{2A}$ selective antagonist exhibiting a $K_i$ value at the $A_{2A}$ adenosine receptor of 300 nM and a more than 10-fold selectivity versus the $A_1$ adenosine receptor (Muller et al., Eur. J. Med. Chem., 1997, 32, 709). However, 8-(phenylethynyl)-DPMX seems not to be active in the Irwin Tests and in catalepsy *in vivo* models (such as e.g. Reserpine, CGS-21680). Another - even more serious - drawback of the described $A_{2A}$ selective xanthine derivates, including the configurationally stable 8-(phenylethynyl)-DPMX (Muller et al., Drugs of the Fut., 2000, 25(10), 1043)-is their high lipophilicity and low water solubility, which limits their use in *in vivo* studies. To increase water solubility, polar groups were in troduced into the phenyl ring, as in *p*-sulfostyryl-DPMX (Muller et al., Bioorg. Med. Chem., 1998, 6, 707). These modifications generally led to a drop in $A_{2A}$ receptor affinity of the compounds. An alternative approach was to prepare water-soluble prodrugs, which contained polar groups that would be split off after *in vivo* application (Muller et al., Drug Dev. Res., 1998, 45, 190; Sauer et al., J. Med. Chem., 2000, 43, 440). MSX-3 was developed as a phosphoric acid ester prodrug of MSX-2 (3-(3-hydroxypropy))-8-(*m*-methoxystyryl)-1-propargylxanthine), which is a potent and selective $A_{2A}$ receptor antagonist soluble in water (Sauer et al., J. Med. Chem., 2000, 43, 440). However, the presence of the double bonds on the 8-position makes MSX-3 (and MSX-2) photosensitive and lead therefore to the problems mentioned above. In addition, the compounds are not only unstable in solution, but also in the solid state, where they undergo light-induced dimerization forming cyclobutane derivatives that show largely reduced receptor affinity and selectivity (Hockemeyer et al., 2004, 69, 3308).

It is an object of the present invention to provide novel 8-ethinylxanthines. It is another object of the present invention to provide new 8-ethinylxanthines which will be more useful as $A_{2A}$ receptor antagonist while avoiding the disadvantages of photosensitivity of known xanthine compounds. Another object is to provide $A_{2A}$ receptor antagonists

exhibiting a high selectivity versus the $A_1$ adenosine receptor.

**Detailed Description**

According to the present invention novel 8-ethinylxanthines are provided, which are represented by the general formula (I),

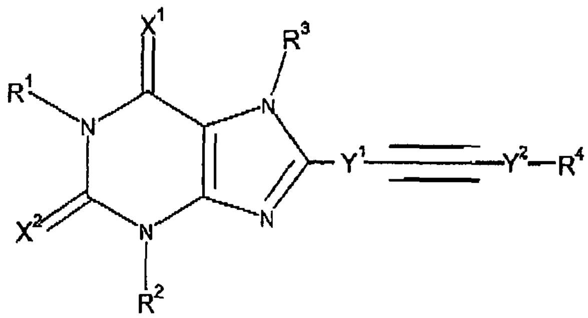

wherein,

- $X^1$, $X^2$ are independently S, O or ($C_1$-$C_6$)-alkyl,
- $Y^1$, $Y^2$ are independently a direct bond or ($C_1$-$C_3$)-alkyl,
- $R^1$ and $R^3$ are each independently hydrogen, optionally substituted heterocyclyl with 3 to 18 ring atoms, optionally substituted aryl, optionally substituted heteroaryl with 5 to 18 ring atoms, -C(O)$R^5$, -C(O)O$R^5$-O$R^5$, -OC(O)$R^5$,
  or
  ($C_1$-$C_{10}$)-alkyl, ($C_2$-$C_{10}$)-alkenyl, ($C_2$-$C_{10}$)-alkinyl, ($C_3$-$C_{16}$)-cycloalkyl, each of which can optionally be substituted in one or more places, in the same way or differently, with halogen, hydroxyl, cyano, amino, nitro, optionally substituted ($C_3$-$C_{18}$)-cycloalkyl, optionally substituted heterocyclyl with 3 to 18 ring atoms, optionally substituted aryl, optionally substituted heteroaryl with 5 to 18 ring atoms, -OC(O)$R^5$ or -C(O)$R^5$, -O$R^5$,
- $R^2$ hydrogen, optionally substituted heterocyclyl with 3 to 18 ring atoms, optionally substituted aryl, optionally substituted heteroaryl with 5 to 18 ring atoms, -N$R^6R^7$, -O$R^8$, -C(O)$R^5$, -C(O)O$R^8$,
  or
  ($C_1$-$C_{10}$)-alkyl, ($C_2$-$C_{10}$)-alkenyl, ($C_2$-$C_{10}$)-alkinyl, ($C_3$-$C_{18}$)-cycloalkyl, each of which can optionally be substituted in one or more places, in the same way or differently with halogen, hydroxyl, cyano, nitro, optionally substituted ($C_3$-$C_{18}$)-cycloalkyl, optionally substituted heterocyclyl with 3 to 18 ring atoms, optionally substituted aryl, optionally substituted heteroaryl with 5 to 18 ring atoms, -N$R^6R^7$, -O$R^6$, -C(O)$R^5$ or -C(O)O$R^8$,
- $R^4$ is an optionally substituted heterocyclyl with 3 to 18 ring atoms, optionally substituted ($C_3$-$C_{18}$)-cycloalkyl or optionally substituted heteroaryl with 5 to 18 ring atoms,
  or
  an aryl optionally substituted with one or more groups selected from the group consisting of halogen, amino, nitro, cyano, -O$R^9$,-C(O)$R^9$, -OC(O)$R^6$, optionally substituted heterocyclyl with 3 to 18 ring atoms, optionally substituted ($C_3$-$C_{18}$)-cycloalkyl, optionally substituted aryl or optionally substituted heteroaryl with 5 to 18 ring at oms,
  or
  an aryl optionally substituted with ($C_1$-$C_{10}$)-alkyl, ($C_2$-$C_{10}$)-alkenyl, ($C_2$-$C_{10}$)-alkinyl, ($C_3$-$C_{16}$)-cycloalkyl, each of which can optionally be further substituted in one or more places, in the same way or differ ently with halogen, hydroxy, cyano, amino, nitro, ($C_1$-$C_8$)-alkyl, ($C_1$-$C_6$)-alkoxy, ($C_1$-$C_6$)-alkoxy-($C_1$-$C_6$)-alkoxy, heterocyclyl with 3 to 18 ring atoms, ($C_3$-$C_{18}$)-cycloalkyl, aryl, a heteroaryl with 5 to 18 ring atoms, -C(O)$R^5$, or -OC(O)$R^5$,
  with the proviso, that
  if the aryl is a phenyl, the phenyl is at least once substituted with a group other than hydrogen,
- $R^5$ is a hydrogen, ($C_1$-$C_6$)-alkyl, ($C_2$-$C_6$)-alkenyl, ($C_2$-$C_6$)-alkinyl,
  or

$(C_1-C_6)$-alkyl substituted in one or more places, in the same way or differently, with hydroxyl, $(C_1-C_6)$-alkoxy, aryl or $-NR^6R^7$,

$R^6$ and $R^7$ are independently hydrogen, $(C_1-C_6)$-alkyl, $(C_2-C_6)$-alkenyl or $(C_2-C_6)$-alkinyl,

$R^8$ hydrogen, $(C_1-C_{10})$alkyl, $(C_2-C_{10})$-alkenyl, $(C_2-C_{10})$-alkinyl,

or

$(C_1-C_6)$-alkyl substituted in one or more places, in the same way or differently, with hydroxyl, $(C_1-C_8)$-alkoxy, aryl or $-NR^6R^7$,

or

optionally substituted $(C_3-C_{18})$-cycloalkyl, optionally substituted heterocyclyl with 3 to 18 ring atoms, optionally substituted aryl, optionally substituted heteroaryl with 5 to 18 ring atoms or carbohydrate,

or

formyl, optionally substituted $(C_1-C_{10})$-alkylcarbonyl, optionally substituted $(C_3-C_{18})$-cycloalkylcarbonyl, optionally substituted arylcarbonyl, optionally substituted heteroarylcarbonyl in which the heteroaryl part has 5 to 18 ring atoms, optionally substituted heterocyclylcarbonyl in which the heterocyclyl part has 3 to 18 ring atoms, hydroxycarbonyl, hydroxyl-$(C_1-C_6)$alkylcarbonyl, $(C_1-C_{10})$-alkoxycarbonyl, optionally substituted aryloxycarbonyl, benzoylacyl, benzoylglycyl, optionally substituted amino acid residue,

or

is selected from the group

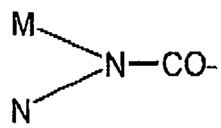

or

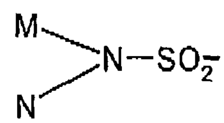

wherein M and N independently represent hydrogen, $(C_1-C_{10})$-alkyl, optionally substituted aryl, or phenoxy $(C_1-C_6)$-alkyl and wherein M and N may form a ring together with the amine nitrogen,

or

an ester moiety of inorganic acids,

or

an ester moiety of ascorbic acid,

or

$-SiR_k,R_j,R_u$, wherein $R_k$, $R_j$, $R_u$ are independently selected from $(C_1-C_6)$-alkyl or aryl,

$R^9$ hydrogen, $(C_{10}-C_{10})$-alkyl, $(C_2-C_{10})$-alkenyl, $(C_2-C_{10})$-alkinyl,

or

$(C_1-C_6)$-alkyl substituted in one or more places, in the same way or differently, with hydroxyl, $(C_1-C_6)$-alkoxy or $-NR^6R^7$,

or

optionally substituted $(C_3-C_{18})$-cycloalkyl, optionally substituted heterocyclyl with 3 to 18 ring atoms or carbohydrate,

or

formyl, optionally substituted $(C_1-C_{10})$-alkylcarbonyl, optionally substituted $(C_3-C_{18})$-cycloalkyloarbonyl, optionally substituted heterocyclylcarbonyl in which the heterocyclyl part has 3 to 18 ring atoms, hydroxycarbonyl, hydroxyl-$(C_1-C_6)$-alkylcarbonyl, $(C_1-C_{10})$-alkoxycarbonyl or an optionally substituted amino acid residue,

or

is selected from the group

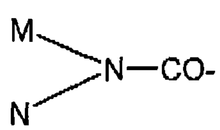

or

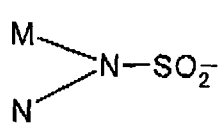

wherein M and N independently represent hydrogen or $(C_1-C_{10})$-alkyl, and wherein M and N may form a

non-aromatic ring together with the amine nitrogen,
or
an ester moiety of inorganic acids,
or
an ester moiety of ascorbic acid,
or
$-SiR_k,R_j,R_u$, wherein $R_k$, $R_j$, $R_u$ are independently selected from ($C_1$-$C_6$)-alkyl or aryl,

or pharmaceutically acceptable salts, isomers, diastereomers or enantiomers thereof.

Suitable salts of the compounds of formula (I) usually have a pharmaceutically acceptable anion or cation. Suitable pharmaceutically acceptable acid addition salts of the compounds of formula (I) are salts of inorganic acids such as hydrochloric acid, hydrobromic, phosphoric, metaphosphoric, nitric and sulfuric acid, and of organic acids such as, for example, acetic acid, benzenesulfonic, benzoic, citric, ethanesulfonic, fumaric, gluconic, glycolic, isethionic, lactic, lactobionic, maleic, malic, methanesulfonic, succinic, p-toluenesulfonic and tartaric acid. Suitable pharmaceutically acceptable basic salts are ammonium salts, alkali metal salts (such as sodium and potassium salts), alkaline earth metal salts (such as magnesium and calcium salts) and salts of trometamol (2-amino-2-hydroxymethyl-1,3-propanediol), diethanolamine, lysine or ethylenediamine.

Salts with a pharmaceutically unacceptable anion, such as, for example, trifluoro acetate, likewise belong within the framework of the invention as useful intermediates for the preparation or purification of pharmaceutically acceptable salts and/or for use in nontherapeutic, for example in vitro, applications.

The compounds of formula (I) may exist in the form of their E-isomers, their Z-isomers, mixtures of E- and Z-isomers, wherein one of the isomers, may be enriched. Preferred compounds of formula (I) are the E-isomers. The isomeric forms may be obtained by known methods.

Further, the compounds of formula (I) may be in the form of their racemates, enantiomer-enriched mixtures and pure enantiomers and to their diastereomers and mixtures thereof in the case that the compound of formula (I) comprises one or more centers of asymmetry. The isomeric forms may be obtained by known methods, even if not expressly described.

The term "alkyl" (alone or in combination with another term(s)) means a substituted or unsubstituted straight or branched chain saturated hydrocarbyl substituent preferably containing from 1 to about 10 carbon atoms ($C_1$-$C_{10}$-alkyl), more preferably from 1 to about 8 carbon atoms ($C_1$-$C_6$-alkyl), even more preferably from 1 to about 6 carbon atoms ($C_1$-$C_6$-alkyl), and most preferably from 1 to 3 carbon atoms ($C_1$-$C_3$-alkyl). The alkyl groups include methyl, ethyl, propyl, isopropyl, butyl, isobutyl, tertiary butyl, amyl, hexyl, and the like. Further, alkyl groups also include halogenated alkyl groups up to perhalogenation, e.g. trifluoromethyl, if not indicated otherwise.

The terms "alkenyl" and "alkinyl" include straight and branched chain radicals of up to 10 carbon atoms, preferably 2 to 8 carbon atoms, more preferably 2 to 8 carbon atoms, most preferably 2 to 8 carbon atoms, and even more preferably from 1 to about 6 carbon atoms wherein the hydrocarbon chain comprises at least one carbon to carbon double bond (in the case of "alkenyl") respectively at least one carbon to carbon triple bond (in the case of "alkinyl"). Examples of "alkenyl" substituents include ethenyl (vinyl), 2-propenyl, 3-propenyl, 1,4-pentadienyl, 1,4-butadienyl, 1-butenyl, 2-butenyl, 3-butenyl, pentenyl, hexenyl, and octenyl. Examples of "alkynyl" substituents include ethynyl, 2-propynyl, 3-propynyl, 1-butynyl, 2-butynyl, 3-butynyl, pentynyl, hexynyl, and octynyl.

The term "alkoxy" (alone or in combination with another term(s)) refers to -O-alkyl and means a straight or branched chain alkoxy substituent preferably containing from 1 to about 10 carbon atoms ($C_1$-$C_{10}$-alkoxy), more preferably from 1 to about 6 carbon atoms ($C_1$-$C_6$-alkoxy), even more preferably from 1 to about 3 carbon atoms ($C_1$-$C_3$-alkoxy), The alkoxy groups include methoxy, ethoxy, propoxy, butoxy, isobutoxy, tert-butoxy, pentoxy, hexoxy and the like. Further, alkoxy groups include halogenated alkoxy groups up to per halogenation, if not indicated otherwise.

The term ""alkoxyalkoxy"" refers to an alkoxy group substituted with at least one another alkoxy group.

The term "halogen" includes fluoro, chloro, bromo, and iodo.

The term "amino" denotes a nitrogen moiety having two hydrogen substituents attached to the nitrogen atom.

The term "cycloalkyl" when used alone or in combination with another term(s) means a cycloalkyl group containing from 3 to 18 ring carbon atoms ($C_3$-$C_{18}$-cycloalkyl), preferably from 3 up to 10 ring carbon atoms ($C_3$-$C_{10}$-cycloalkyl) and more preferably from 3 up to 6 ring atoms ($C_3$-$C_6$-cycloalkyl). The cycloalkyl groups may be monocyclic, bicyclic, tricyclic, or polycyclic. Examples of single-ring cycloalkyls include cyclopropyl, cyclobutyl, cyclopentyl, and cyclohexyl. A cycloalkyl alternatively may be a fused, bridged or spirocyclic ring system of 2 or 3 rings such as, for example, norbonyl, decalinyl, bicycloheptanyl, adamantyl, and norpinanyl, and the rings may be fused.

"Heterocyclyl" as defined herein contains at least one heteroatom in the cyclic structure, preferably one, two, three or four heteroatoms. The at least one heteroatom may be independently selected from sulfur, nitrogen and oxygen. Furthermore, the ring optionally can be interrupted by one or more -(CO)-, -(CS)-, or $-SO_2-$ groups. Only those combinations are meant, however, that are useful from the viewpoint of one skilled in the art. The heterocyclic groups contemplated by the present invention include saturated and partially saturated heterocyclic groups. The heterocyclics may be monocyclic, bicyclic, tricyclic or polycyclic and may be fused rings. The heterocyclics also include the so-called benzoheterocyclics. The binding valence is at a carbon atom or at a nitrogen atom and preferably at the carbon atom. The heterocycloalkyl group comprises 3 to 18 ring atoms, preferably 3 to 10 ring atoms, more preferably 3 to 7 ring atoms. A heterocyclyl ring with 3 ring atoms is for example aziridinyl, A heterocyclyl with 4 ring atoms is for example azetidinyl. Heterocyclyl with 5 ring atoms encompasses for example the rings: pyrrolidinyl, imidazolidinyl and pyrazolidinyl. Heterocyclyl rings with 6 ring atoms encompasses for example the rings: piperidinyl, piperazinyl, morpholinyl and thiomorphinyl. Heterocyclyl rings with 7 ring atoms encompasses for example the rings: azepanyl, [1,3]-diazepanyl and [1,4]-diazepanyl. Prefered are heterocycyl rings with 5 or 6 ring atoms,

The term "aryl", when used alone or in combination with other term(s), refers to an aromatic group which contains from 6 up to 18 ring carbon atoms ($C_6$-$C_{18}$-aryl), preferably from 6 up to 10 ring carbon atoms ($C_6$-$C_{10}$-aryl), and includes polynuclear aromatics. The aryl groups may be monocyclic, bicyclic, tricyclic or polycyclic and may be fused rings. A polynuclear aromatic compound as used herein, is meant to encompass bicyclic and tricyclic fused aromatic ring systems containing from 10-18 ring carbon atoms. Aryl groups include phenyl and polynuclear aromatics e.g., naphthyl, anthracenyl, phenanthrenyl, azulenyl and the like. The aryl group also includes groups such as ferrocenyl. A preferred aryl group is phenyl.

The term "benzoyl" denotes an acyl group of the formula $-CO-C_6H_5$ wherein the phenyl ring.

The term "heteroaryl" as employed in the present invention refers to monocyclic or bicyclic aromatic groups containing 1 to 3, preferably 1 or 2, more preferably 1 heteroatom, especially N and/or O and/or S. The heteroaryl group contains 5 to 18 ring atoms, preferably from 5 to 14 ring atoms, more preferably from 5 to 10 ring atoms Preferably, the "heteroaryl" group is bound via a carbon ring atom. It is possible, that the heteroaryl groups in the meaning of the present invention comprise one aromatic and one non-aromatic ring such as e.g. benzodioxole. Specific examples of heteroaryl substituents include 6-membered ring substituents such as pyridinyl, pyrazinyl, pyrimidinyl, pyridazinyl, and 1,3,5-, 1,2,4-, and 1,2,3-triazinyl; 5-membered ring substituents such as thienyl, imidazolyl, furanyl, pyrazolyl, oxazolyl, isoxazolyl, thiazolyl, 1,2,3-, 1,2,4-, 1,2,5-, or 1,3,4-oxadiazolyl and isothiazolyl; 6/5-membered fused ring substituents such as benzothiofu ranyl, isobenzothiofuranyl, benzisoxazolyl, benzoxazolyl, purinyl, and anthranilyl; and 6/6-membered fused rings such as quinolinyl, isoquinolinyl, cinnolinyl, and quinazolinyl.

In the compounds according to the present invention the term "($C_3$-$C_{18}$)-cycloalkylcarbonyl" denotes a group R-C(=O)-, wherein R is an $C_3$-$C_{18}$ cycloalkyl group as defined hereinbefore. The term "arylcarbonyl" denotes a group R-C(=O)-, wherein R is an aryl group as defined hereinbefore. Other carbonyl comprising groups are defined analogously.

The term "carbohydrate" denotes the residue of a polyhydroxyaldehyde or polyhydroxyketone of the formula $C_nH_{2n}O_n$ or $C_n(H_2O)_n$, wherein n ≥3, preferably 5-6, and corresponding carbohydrate groups are, for example, described in Aspinal, The Polysaccarides, New York: Academic Press 1982, 1983. A preferred carbohydrate group in the compounds according to the present invention is a glucosyl, in particular a 1-β-D-glucopyranosyl group.

The term "amino add residue" denotes the residue of a naturally occurring or synthetic amino acid. Particularly preferred amino acid residues are selected from the group consisting of glycyl, valyl, isoleucyl, phenylalanyl, prolyl, seryl, threonyl, methionyl, hydoxyprolyl. The amino acid residue may be substituted by a suitable group. Examples are benzyl glycyl and N-acetylglycyl.

Suitable ester moieties of inorganic acids may be derived from inorganic acids such as sulfuric acid and phosphoric acid.

The alkyl, ($C_3$-$C_{18}$)-cycloalkyl, heterocyclyl with 3 to 18 ring atoms, aryl, heteroaryl with 5 to 18 ring atoms, ($C_3$-$C_{18}$)-cycloalkylcarbonyl, arylcarbonyl, heteroarylcarbonyl in which the heteroaryl part has 5 to 18 ring atoms, heterocyclylcarbonyl in which the heterocyclyl part has 3 to 18 ring atoms, ($C_1$-$C_{10}$)-alkylcarbonyl, aryloxycarbonyl, amino acid residue mentioned above may be optionally substituted. Suitable substituents are for example halogen substituents such as fluorine, chlorine, bromine, iodine, or trifluoromethyl groups, amino, nitro, cyano, hydroxyl, $=CH_2$, ($C_1$-$C_{10}$)-alkyl, ($C_1$-$C_6$)-alkoxy, aryl, halogenated aryl, heteroaryl, halogenated heteroaryl, heterocyclyl, halogenated heterocylyl, cycloalkyl, halogenated cycloalkyl etc. The definition of the term "suitable substituents" in case of the above mentioned molecules which comprise a ring structure (such as e.g. heterocyclcyl, heteroaryl, aryl etc.) also include interruptions of the rings by one ore more -(CO)-, -(CS)-, or -($SO_2$)-groups.

The number of substituents may be from 1 to 6, preferably 1 or 4, more preferably 1, 2 or 3.

As used herein, the term "$C_1$-$C_{10}$", as used throughout this text e.g. in the context of the definitions of "$C_1$-$C_{10}$-alkyl" and "$C_1$-$C_{10}$-alkoxy", is to be understood as meaning an alkyl or alkoxy group having a finite number of carbon atoms of 1 to 12, i.e. 1, 2, 3, 4, 5, 6, 7, 8, 9, 10, 11 or 12 carbon atoms. It is to be understood further that said term "$C_1$-$C_{10}$" is to be interpreted as any subrange comprised therein, e.g. $C_1$-$C_{10}$, $C_2$-$C_{10}$, $C_3$-$C_{10}$, $C_4$-$C_{10}$, $C_5$-$C_{10}$, $C_6$-$C_{10}$, $C_1$-$C_2$, $C_1$-$C_3$, $C_1$-$C_4$, $C_1$-$C_5$, $C_1$-$C_6$, $C_1$-$C_7$, $C_1$-$C_8$, $C_1$-$C_9$, $C_1$-$C_{10}$; preferably $C_1$-$C_2$, $C_1$-$C_3$, $C_1$-$C_4$, $C_1$-$C_5$, $C_1$-$C_6$; more preferably $C_1$-$C_3$.

Similarly, as used herein, the term "$C_2$-$C_{10}$", as used throughout this text e.g. in the context of the definitions of "$C_2$-$C_{10}$-alkenyl", is to be understood as meaning an alkenyl group having a finite number of carbon atoms of 2 to 10,

i.e. 2, 3, 4, 5, 6, 7, 8, 9 or 10 carbon atoms. It is to be understood further that said term "$C_2$-$C_{10}$" is to be interpreted as any subrange comprised therein, e.g. $C_2$-$C_8$, $C_2$-$C_6$, $C_3$-$C_5$, $C_3$-$C_4$, $C_2$-$C_3$, $C_2$-$C_4$, $C_2$-$C_5$.

All area information of the application not explicitly cited here is defined analogously to the above areas of $C_1$-$C_{10}$" or "$C_2$-$C_{10}$".

In the general formula (I) $X^1$ stands for S, O or ($C_1$-$C_6$)-alkyl and $X^2$ stands for is S, O, Halogen, ($C_1$-$C_6$)-alkyl, -$OR^5$ or -$SR^5$,

In a preferred embodiment of the present invention $X^1$ and $X^2$ according to formula (I) are independently S, O or ($C_1$-$C_3$)-alkyl. In an even more preferred embodiment $X^1$ and $X^2$ are independently O.

In the general formula (I) $Y^1$, $Y^2$ are independently a direct bond or ($C_1$-$C_3$)-alkyl In a preferred embodiment of the present invention $Y^1$ and $Y^2$ according to formula (I) are independently a direct bond.

In the general formula (I) $R^1$ stands for are each independently hydrogen, optionally substituted heterocyclyl with 3 to 18 ring atoms, optionally substituted aryl, optionally substituted heteroaryl with 5 to 18 ring atoms, -C(O)$R^5$, -C(O)$OR^6$, -$OR^5$, -OC(O)$R^5$, or ($C_1$-$C_{10}$)-alkyl, ($C_2$-$C_{10}$)-alkenyl, ($C_2$-$C_{10}$)-alkinyl, ($C_3$-$C_{18}$)-cycycloalkyl, each of which can optionally be substituted in one or more places, in the same way or differently, with halogen, hydroxyl, cyano, amino, nitro, optionally substituted ($C_3$-$C_{18}$)-cycloalkyl, optionally substituted heterocyclyl with 3 to 18 ring atoms, optionally substituted aryl, optionally substituted heteroaryl with 5 to 18 ring atoms, -OC(O)$R^5$ or -C(O)$R^5$, -$OR^5$.

Those compounds of general formula (I), in which $R^1$ is hydrogen, or ($C_1$-$C_8$)-alkyl, ($C_2$-$C_8$)-alkenyl, ($C_2$-$C_8$)-alkinyl, ($C_3$-$C_6$)-cycloalkyl, each of which can optionally be substituted in one or more places, in the same way or differently, with halogen, hydroxy, cyano, amino, nitro, ($C_3$-$C_6$)-cycloalkyl, heterocyclyl with 3 to 7 ring atoms, aryl, heteroaryl with 5 to 10 ring atoms, -OC(O)$R^6$ or -C(O)$R^6$, are preferred.

More preferably, $R^1$ of general formula (I) stands for hydrogen, ($C_1$-$C_6$)-alkyl, allyl, propargyl, or a ($C_1$-$C_6$)-alkyl substituted with one or more cyano or ($C_3$-$C_6$)-cycloalkyl groups.

Even more preferably, $R^1$ of general formula (I) stands for a ($C_1$-$C_4$)-alkyl, cyanomethyl, allyl, propargyl or a cyclobutylmethyl group.

More preferably, $R^1$ of general formula (I) stands for an ethyl, allyl or propargyl group.

$R^1$ of general formula (I) can also preferably stand for a propargyl or methyl group.

Most preferably, $R^1$ of general formula (I) stands for a propargyl group.

In the general formula (I) $R^2$ stands for hydrogen, optionally substituted heterocyclyl with 3 to 18 ring atoms, optionally substituted aryl, optionally substituted heteroaryl with 5 to 18 ring atoms, -$NR^6R^7$, -$OR^8$, -C(O)$R^5$, -C(O)$OR^8$, or ($C_1$-$C_{10}$)-alkyl, ($C_2$-$C_{10}$)-alkenyl, ($C_2$-$C_{10}$)-alkinyl, ($C_3$-$C_{18}$)-cycloalkyl, each of which can optionally be substituted in one or more places, in the same way or differently with halogen, hydroxyl, cyano, nitro, optionally substituted ($C_3$-$C_{18}$)-cycloalkyl, optionally substituted heterocyclyl with 3 to 18 ring atoms, optionally substituted aryl, optionally substituted heteroaryl with 5 to 18 ring atoms, -$NR^6R^7$, -$OR^8$, -C(O)$R^5$ or -C(O)$OR^8$.

Preferably $R^2$ of general formula (I) stands for is a hydrogen, amino, ($C_1$-$C_6$)-alkyl-N-, ($C_1$-$C_6$)-alkyl-C(O)O-, or ($C_1$-$C_8$)-alkyl, ($C_2$-$C_8$)-alkenyl, ($C_2$-$C_8$)-alkinyl, ($C_3$-$C_6$)-cycloalkyl, each of which can optionally be substituted in one or more places, in the same way or differently with hydroxyl, halogen, cyano. nitro, -CO(OH), -C(O)H, ($C_3$-$C_6$)-cycloalkyl, heterocyclyl with 3 to 7 ring atoms, aryl, heteroaryl with 5 to 10 ring atoms, -$NR^6R^7$, or -$OR^8$.

$R^2$ of general formula (I) is a hydrogen or an allyl, propargyl, amino, ($C_1$-$C_4$)-alkyl-C(O)O-, or a ($C_1$-$C_6$)-alkyl-group optionally substituted with one or more groups selected from the group consisting of hydroxyl, halogen, cyano, -$NR^6R^7$, -C(O)OH, -C(O)H, ($C_1$-$C_3$)-alkoxy, ($C_1$-$C_3$)-alkoxy-($C_1$-$C_3$)-alkoxy, ($C_1$-$C_3$)-alkyl-C(O)O-, a heterocyclyl with 3 to 6 ring atoms, phosphate ester, or carbohydrate ester.

More preferably $R^2$ of general formula (I) stands for is hydrogen or a methyl, ethyl, allyl, bromethyl, amino, cyano-($C_2$-$C_3$)-alkyl, propargyl, butyl-C(O)O-, butyl-C(O)O-methyl-, methyl-C(O)O-propyl-, $R^6R^7$N-($C_2$-$C_3$)-alkyl-, epoxide-ethyl, 1,3-dioxo-1,3-dihydro-2H-isoindol-ethyl, dioxolane-propyl, morpholine-ethyl, $(OH)_2$OP(O)-propyl, H-C(O)-ethyl, HO-C(O)-ethyl, hydroxyl-($C_2$-$C_3$)-alkyl, dihydroxypropyl, glucosyl-O-propyl, methoxyethoxy-ethyl-, or a trihydroxypentyl-group.

$R^2$ of general formula (I) stands more preferably for is a hydrogen or an amino, methyl, ethyl, allyl, bromethyl, cyanomethyl, propargyl, butyl-C(O)O-, butyl-C(O)O-methyl, methyl-C(O)O-propyl-, $(CH_3)_2$-N-($C_2$-$C_3$)-alkyl, epoxide-ethyl, 3-dioxo-1,3-dihydro-2H-isoindolethyl, dioxolane-propyl, morpholine-ethyl, $(OH)_2$OP(O)-propyl, H-C(O)-ethyl, HO-C(O)-ethyl, hydroxyethyl, hydroxypropyl, or a dihydroxypropyl group.

Even more preferably $R^2$ of general formula (I) stands for a hydrogen, methyl, ethyl, allyl, bromethyl, propargyl, hydroxyethyl, hydroxypropyl, dihydroxypropyl, $(CH_3)_2$-N-ethyl, $(OH)_2$OP(O)-propyl, or a dioxolane-propyl group.

Even more preferably $R^2$ of general formula (I) stands for a hydrogen, methyl, ethyl, allyl, propargyl, hydroxyethyl or hydroxypropyl group.

Most preferably $R^2$ of general formula (I) is hydrogen, methyl, ethyl, or allyl.

Most preferred are also compounds according to general formula (I) wherein $R^2$ is a methyl, ethyl or hydroxypropyl group; in particular wherein $R^2$ stands for a ethyl or a hydroxypropyl group.

In the general formula (I) $R^3$ stands for are each independently hydrogen, optionally substituted heterocyclyl

with 3 to 18 ring atoms, optionally substituted aryl, optionally substituted heteroaryl with 5 to 18 ring atoms, -C(O)$R^5$, -C(O)O$R^5$, -O$R^5$, -OC(O)$R^5$, or ($C_1$-$C_{10}$)-alkyl, ($C_2$-$C_{10}$)-alkenyl, ($C_2$-$C_{10}$)-alkinyl, ($C_3$-$C_{18}$)-cycloalkyl, each of which can optionally be substituted in one or more places, in the same way or differently, with halogen, hydroxyl, cyano, amino, nitro, optionally substituted ($C_3$-$C_{18}$)-cycloalkyl, optionally substituted heterocyclyl with 3 to 18 ring atoms, optionally substituted aryl, optionally substituted heteroaryl with 5 to 18 ring atoms, -OC(O)$R^5$ or -C(O)$R^5$, -O$R^5$.

Those compounds of general formula (I), in which $R^3$ is hydrogen, or ($C_1$-$C_8$)-alkyl, ($C_2$-$C_8$)-alkenyl, ($C_2$-$C_8$)-alkinyl, ($C_3$-$C_6$)-cycloalkyl, each of which can optionally be substituted in one or more places, in the same way or differently, with halogen, hydroxy, cyano, amino, nitro, ($C_3$-$C_6$)-cycloalkyl, heterocyclyl with 3 to 7 ring atoms, aryl, heteroaryl with 5 to 10 ring atoms, -OC(4)$R^6$ or -C(O)$R^6$, are preferred.

Preferably $R^3$ of general formula (I) stands for hydrogen, ($C_1$-$C_8$)-alkyl, propargyl, or a ($C_1$-$C_3$)-alkyl group substituted with one or more groups selected from the group consisting of hydroxyl, halogen, cyano, phenyl, ($C_3$-$C_6$)-cycloalkyl, -OC(O)$R^6$ and -C(O)$R^6$.

More preferably $R^3$ of general formula (I) stands for hydrogen or a ($C_1$-$C_5$)-alkyl, hydroxyethyl, propargyl, methyl-C(O)-methyl, phenyl-methyl, cyclobutylmethyl, or butyl-C(O)O-methyl-group.

More preferably $R^3$ of general formula (I) stands for a methyl, ethyl or progargyl group.

Even more preferably $R^3$ of general formula (I) is a methyl or progargyl group.

Most preferably $R^3$ of general formula (I) is a methyl group.

In the general formula (I) $R^4$ stands for optionally substituted heterocyclyl with 3 to 18 ring atoms, optionally substituted ($C_3$-$C_{18}$)-cycloalkyl or optionally substituted heteroaryl with 5 to 18 ring atoms, or an aryl optionally substituted with one or more groups selected from the group consisting of halogen, amino, nitro, cyano, -O$R^8$, -C(O)$R^8$, -OC(O)$R^8$ optionally substituted heterocyclyl with 3 to 18 ring atoms, optionally substituted ($C_3$-$C_{18}$)-cycloalkyl, optionally substituted aryl or optionally substituted heteroaryl with 5 to 18 ring atoms, or an aryl optionally substituted with ($C_1$-$C_{10}$)-alkyl, ($C_2$-$C_{10}$)-alkenyl, ($C_2$-$C_{10}$)-alkinyl, ($C_3$-$C_{18}$)-cycloalkyl, each of which can optionally be further substituted in one or more places, in the same way or differently with halogen, hydroxy, cyano, amino, nitro, ($C_1$-$C_8$)-alkyl, ($C_1$-$C_6$)-alkoxy, ($C_1$-$C_6$)-alkoxy-($C_1$-$C_6$)-alkoxy, heterocyclyl with 3 to 18 ring atoms, ($C_3$-$C_{18}$)-cycloalkyl, aryl, a heteroaryl with 5 to 18 ring atoms, -C(O)$R^5$, or-OC(O)$R^5$, with the proviso, that if the aryl is a phenyl, the phenyl is at least once substituted with a group other than hydrogen.

In the general formula (I) $R^4$ stands for preferably for heterocyclyl with 3 to 7 ring atoms or a heteroaryl with 5 to 10 ring atoms, or an aryl optionally substituted with one or more groups selected from the group consisting of halogen, amino, nitro, cyano, -O$R^5$, -C(O)$R^6$, -OC(O)$R^6$, ($C_3$-$C_6$)-cycloalkyl, aryl, heterocyclyl with 3 to 7 ring atoms or a heteroaryl with 5 to 10 ring atoms or an aryl optionally substituted with ($C_1$-$C_8$)-alkyl, ($C_2$-$C_8$)-alkenyl, ($C_2$-$C_8$)-alkinyl, ($C_3$-$C_6$)-cycloalkyl, each of which can optionally be further substituted in one or more places, in the same way or differently with halogen, hydroxy, cyano, amino, nitro, ($C_1$-$C_8$)-alkyl, ($C_1$-$C_6$)-alkoxy, ($C_1$-$C_6$)-alkoxy-($C_1$-$C_6$)-alkoxy, ($C_3$-$C_6$)-cycloalkyl, heterocyclyl with 3 to 7 ring atoms, aryl, -C(O)$R^6$, or -OC(O)$R^6$.

Preferably $R^4$ of general formula (I) stands for a heterocyclyl with 5 ring atoms selected from the group consisting of furyl, thienyl, pyrrolyl, pyrrolinyl, pyrrolidinyl, dioxolanyl, oxazolyl, thiazolyl, imidazolyl, imidazolinyl, pyrazolyl, pyrazolinyl, pyrazolidinyl, isoxazolyl, isothiazolyl, oxadiazolyl, triazolyl and thiadiazolyl, or a phenyl substituted with $R^a$ and $R^b$, wherein $R^a$ and $R^b$ are connected to adjacent carbonate atoms of the phenyl ring and form together a carbocyclic or heterocyclic ring from 3 to 6 atoms, or an aryl optionally substituted with one or more groups selected from the group consisting of ($C_1$-$C_4$)-alkyl-, halogenyl, nitro, amino, trifluormehyl-, -O$R^5$ and -OC(O)$R^6$.

$R^4$ of general formula (I) stands more preferably for a thiophene or benzodioxole, or a phenyl substituted with one or more groups selected from the group consisting of methyl-, halogen, nitro, amino, trifluormethyl-, -OC(O)$CH_3$ and -O$R^5$.

More preferably $R^4$ of general formula (I) stands for a benzodioxole, or a phenyl substituted with one or more groups selected from the group consisting of methyl-, halogen, nitro, amino, trifluormethyl, -OC(O)$CH_3$ and -O$R^6$.

More preferably $R^4$ of general formula (I) stands for phenyl substituted with one or more groups selected from the group consisting of methyl, halogen, -OC(O)$CH_3$ and -O$R^5$.

Even more preferably $R^4$ of general formula (I) stands for a phenyl substituted with one or more groups selected from the group consisting of methyl, and methoxy.

Even more preferably $R^4$ of general formula (I) stands for a phenyl substituted with one or more groups discussed herein at meta- and/or para-position. Even more preferably at meta- and para-position.

Even more preferably $R^4$ of general formula (I) stands for a phenyl substituted with one or more methoxy groups or a meta-methylphenyl group.

Even more preferred is a compound wherein $R^4$ of general formula (I) stands for phenyl substituted with one or more methoxy groups, particularly preferred is meta-methoxyphenyl, or 3,4-dimethoxyphenyl.

Most preferred is a compound wherein $R^4$ of general formula (I)stands for phenyl substituted with two methoxy groups, particularly preferred is 3,4-dimethoxyphenyl.

In the general formula (I) $R^5$ stands for a hydrogen, ($C_1$-$C_6$)-alkyl, ($C_2$-$C_6$)-alkenyl, ($C_2$-$C_6$)-alkinyl, or

$(C_1-C_6)$-alkyl substituted in one or more places, in the same way or differently, with hydroxyl, $(C_1-C_6)$-alkoxy, aryl or $-NR^6R^7$.

$R^5$ in the general formula (I) stands preferably for a hydrogen, $(C_1-C_6)$-alkyl, $(C_2-C_6)$-alkenyl, $(C_2-C_6)$-alkinyl, or $(C_1-C_6)$-alkyl substituted in one or more places, in the same way or differently, with hydroxyl, aryl or $-NR^6R^7$.

Preferably $R^5$ of general formula (I) stands fora hydrogen, $(C_1-C_4)$-alkyl, phenyl-$(C_1-C_3)$-alkyl, hydroxyl-$(C_1-C_4)$-alkyl, $R^6R^7$-N-$(C_1-C_4)$-alkyl, or allyl.

More preferably $R^5$ of general formula (I) stands for hydrogen, methyl, ethyl, hydroxyl-$(C_2-C_3)$-alkyl, $(CH_3)_2$-N-$(C_2-C_3)$-alkyl, phenylmethyl or allyl.

Even more preferably $R^5$ of general formula (I) stands fora hydrogen, methyl, ethyl, allyl, hydroxyl-$(C_2-C_3)$-alkyl, or $(CH_3)_2$-N-$(C_2-C_3)$-alkyl.

Most preferably $R^5$ of general formula (I) stands for is a methyl, ethyl, allyl, or hydroxylethyl.

In the general formula (I) $R^6$ and $R^7$ stands independently from each other for hydrogen, $(C_1-C_6)$-alkyl, $(C_2-C_6)$-alkenyl or $(C_2-C_6)$-alkinyl.

Preferably $R^6$ and $R^7$ of general formula (I) stand independently from each other for hydrogen or a $(C_1-C_6)$-alkyl group.

More preferably, $R^6$ and $R^7$ of general formula (I) stand independently from each other for hydrogen or a $(C_1-C_3)$-alkyl group.

Most preferably $R^6$ and $R^7$ of general formula (I) stand independently from each other for hydrogen or a methyl group.

In the general formula (I) $R^8$ stands for hydrogen, $(C_1-C_{10})$-alkyl, $(C_2-C_{10})$alkenyl, $(C_2-C_{10})$-alkinyl, or $(C_1-C_6)$-alkyl substituted in one or more places, in the same way or differently, with hydroxyl, $(C_1-C_6)$-alkoxy, aryl or $-NR^6R^7$, or optionally substituted $(C_3-C_{18})$-cycloalkyl, optionally substituted heterocyclyl with 3 to 18 ring atoms, optionally substituted aryl, optionally substituted heteroaryl with 5 to 18 ring atoms or carbohydrate, or formyl, optionally substituted $(C_1-C_{10})$-alkylcarbonyl, optionally substituted $C_3-C_{16}$)-cycloalkylcarbonyl, optionally substituted arylcarbonyl, optionally substituted heteroaryl carbonyl in which the heteroaryl part has 5 to 18 ring atoms, optionally substituted heterocyclylcarbonyl in which the heterocyclyl part has 3 to 18 ring atoms, hydroxycarbonyl, hydroxyl-$(C_1-C_6)$-alkylcarbonyl, $(C_1-C_{10})$-alkoxycarbonyl, optionally substituted aryloxycarbonyl, benzoylacyl, benzoylglycyl, optionally substituted amino acid residue, or is selected from the group

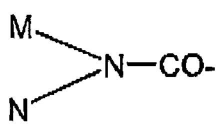

or

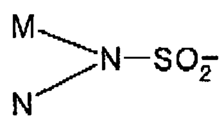

wherein M and N independently represent hydrogen, $(C_1-C_{10})$-alkyl, optionally substituted aryl, or phenoxy$(C_1-C_6)$-alkyl and wherein M and N may form a ring together with the amine nitrogen, or an ester moiety of inorganic acids, or an ester moiety of ascorbic acid, or-$SiR_k,R_j,R_u$, wherein $R_k$, $R_j$, $R_u$ are independently selected from $(C_1-C_6)$-alkyl or aryl.

Preferably $R^8$ of the general formula stands for hydrogen, $(C_1-C_6)$-alkyl, $(C_1-C_6)$-alkoxy-$(C_1-C_6)$-alkyl, $(C_3-C_6)$-cycloalkyl, benzyl, allyl or carbohydrate, or formyl, $(C_1-C_6)$-alkylcarbonyl, $(C_3-C_6)$-cycloalkylcarbonyl, arylcarbonyl, or hydroxycarbonyl, $(C_1-C_6)$-alkoxycarbonyl, aryloxycarbonyl, benzoylacyl, benzoylglycyl, amino acid residue, or is selected from the group

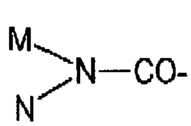

or

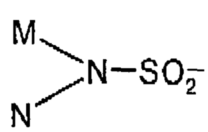

wherein M and N independently represent hydrogen, $(C_1-C_6)$-alkyl, aryl, benzyl, or phenoxy$(C_1-C_6)$-alkyl and wherein M and N may form a ring from 3 to 6 atoms together with the amine nitrogen, or an ester moiety of inorganic acids, or an ester moiety of ascorbic acid, or $-SiF_k,R_j,R_u$, wherein $R_k$, $R_j$, $R_u$ are independently selected from $(C_1-C_4)$-alkyl or aryl.

Other preferred compounds according to general formula (I) are those, wherein

$R^4$ is heterocyclyl with 3 to 7 ring atoms or a heteroaryl with 5 to 10 ring atoms, or an aryl optionally substituted

with one or more groups selected from the group consisting of halogen, amino, nitro, cyano, -$OR^5$, -C(O)$R^6$, -OC(O)$R^6$, ($C_3$-$C_6$)-cycloalkyl, aryl, heterocyclyl with 3 to 7 ring atoms or a heteroaryl with 5 to 10 ring atoms or an aryl optionally substituted with ($C_1$-$C_8$)-alkyl, ($C_2$-$C_8$)-alkenyl, ($C_2$-$C_8$)-alkinyl, ($C_3$-$C_6$)-cycloalkyl, each of which can optionally be further substituted in one or more places, in the same way or differently with halogen, hydroxy, cyano, amino, nitro, ($C_1$-$C_8$)-alkyl, ($C_1$-$C_6$)-alkoxy, ($C_1$-$C_6$)-alkoxy-($C_1$-$C_6$)-alkoxy, ($C_3$-$C_6$)-cycloalkyl, heterocyclyl with 3 to 7 ring atoms, aryl, -C(O)$R^6$, or -OC(O)$R^6$,

$R^5$ is a hydrogen, ($C_1$-$C_6$)-alkyl, ($C_2$-$C_6$)-alkenyl, ($C_2$-$C_6$)-alkinyl, or ($C_1$-$C_6$)-alkyl substi tuted in one or more places, in the same way or differently, with hydroxyl, aryl or -$NR^6R^7$,

$R^6$ and $R^7$ are independently hydrogen or a ($C_1$-$C_6$)-alkyl group, or pharmaceutically acceptable salts, isomers, diastereomers or enantiomers thereof.

Other preferred compounds of general formula (I) are whose, wherein

$R^1$ is hydrogen, ($C_1$-$C_6$)-alkyl, allyl, propargyl, or a ($C_1$-$C_6$)-alkyl substituted with one or more ($C_3$-$C_6$)-cycloalkyl groups,

$R^2$ is a hydrogen or an allyl, propargyl, amino, ($C_1$-$C_4$)-alkyl-C(O)O-, or a ($C_1$-$C_6$)-alkyl-group optionally substituted with one or more groups selected from the group consisting of hydroxyl, halogen, cyano, -$NR^6R^7$, -C(O)OH, -C(O)H, ($C_1$-$C_3$)-alkoxy, ($C_1$-$C_3$)-alkoxy-($C_1$-$C_3$)-alkoxy, ($C_1$-$C_3$)-alkyl-C(O)O-, a heterocyclyl with 3 to 6 ring atoms, phosphate ester, or carbohydrate ester,

$R^3$ is a hydrogen, ($C_1$-$C_8$)-alkyl, propargyl, or a ($C_1$-$C_3$)-alkyl group substituted with one or more groups selected from the group consisting of hydroxyl, halogen, cyano, phenyl, ($C_3$-$C_6$)-cycloalkyl, -OC(O)$R^6$ and -C(O)$R^6$,

$R^4$ is a heterocyclyl with 5 ring atoms selected from the group consisting of furyl, thienyl, pyrrolyl, pyrrolinyl, pyrrolidinyl, dioxolanyl, oxazolyl, thiazolyl, imidazolyl, imidazolinyl, pyrazolyl, pyrazolinyl, pyrazolidinyl, isoxazolyl, isothiazolyl, oxadiazolyl, triazolyl and thiadiazolyl, or a phenyl substituted with $R^a$ and $R^b$, wherein $R^a$ and $R^b$ are connected to adjacent carbonate atoms of the phenyl ring and form together a carbocyclic or heterocyclic ring from 3 to 6 atoms, or an aryl optionally substituted with one or more groups selected from the group consisting of ($C_1$-$C_4$)-alkyl, halogenyl, nitro, amino, trifluormethyl, -$OR^5$ and -OC(O)$R^6$,

$R^5$ is a hydrogen, ($C_1$-$C_4$)-alkyl, phenyl-($C_1$-$C_3$)-alkyl, hydroxyl-($C_1$-$C_4$)-alkyl, $R^6R^7$-N-($C_1$-$C_4$)-alkyl, or allyl,

$R^6$ and $R^7$ are independently hydrogen or a ($C_1$-$C_3$)-alkyl group,or pharmaceutically acceptable salts, isomers, diastereomers or enantiomers thereof.

More preferred compounds of general formula (I) are whose, wherein

$R^1$ is a ($C_1$-$C_4$)-alkyl, allyl, propargyl or a cyclobutylmethyl group

$R^2$ is hydrogen or a methyl, amino, ethyl, allyl, bromethyl, cyano-($C_2$-$C_3$)-alkyl, propargyl, butyl-C(O)O-, butyl-C(O)O-methyl-, methyl-C(O)O-propyl-, $R^6R^7$N-($C_2$-$C_3$)-alkyl-, epoxide-ethyl, 1,3-dioxo-1,3-dihydro-2H-isoindol-ethyl, dioxolane-propyl, morpholine-ethyl, $(OH)_2$OP(O)-propyl, H-C(O)-ethyl, HO-C(O)-ethyl, hydroxyl-($C_2$-$C_3$)-alkyl, dihydroxypropyl, glucosyl-O-propyl, methoxyethoxy-ethyl-, or a trihydroxypentyl-group,

$R^3$ is hydrogen or a ($C_1$-$C_5$)-alkyl, hydroxyethyl, propargyl, methyl-C(O)-methyl, phenyl-methyl, cyclobutylmethyl, or butyl-C(O)O-methyl-group,

$R^4$ is a thiophene or benzodioxole, or a phenyl substituted with one or more groups selected from the group consisting of methyl-, halogen, nitro, amino, trifluormethyl-, -OC(O)$CH_3$ and -$OR^5$,

$R^5$ is a hydrogen, methyl, ethyl, hydroxyl-($C_2$-$C_3$)-alkyl, $(CH_3)_2$-N-($C_2$-$C_3$)-alkyl, phenyl-methyl or allyl,

$R^6$ and $R^7$ are independently hydrogen or a methyl group, or pharmaceutically acceptable salts, isomers, diastereomers or enantiomers thereof.

More preferred compounds of general formula (I) are whose, wherein

$R^2$ is a hydrogen or an amino, methyl, ethyl, allyl, bromethyl, cyanomethyl, propargyl, butyl-C(O)O-, butyl-C(O)O-methyl, methyl-C(O)O-propyl-, $(CH_3)_2$-N-($C_2$-$C_3$)-alkyl, epoxide-ethyl, 3-dioxo-1,3-dihydro-2H-isoindol-ethyl, dioxolane-propyl, morpholine-ethyl, $(OH)_2$OP(O)-propyl, H-C(O)-ethyl, HO-C(O)-ethyl, hydroxyethyl, hydroxypropyl, or a dihydroxypropyl group,

$R^4$ is a benzodioxole, or a phenyl substituted with one or more groups selected from the group consisting of methyl-, halogen, nitro, amino, trifluormethyl, -OC(O)$CH_3$ and -$OR^5$, or pharmaceutically acceptable salts, isomers, diastereomers or enantiomers thereof.

Other more preferred compounds of general formula (I) are whose, wherein

$R^1$ is a ethyl, allyl or propargyl group,
$R^3$ is a methyl, ethyl or progargyl group,
$R^4$ is phenyl substituted with one or more groups selected from the group consisting of methyl, halogen, $-OC(O)CH_3$ and $-OR^5$,
$R^5$ is a hydrogen, methyl, ethyl, allyl, hydroxyl-($C_2$-$C_3$)-alkyl, or $(CH_3)_2$-N-($C_2$-$C_3$)-alkyl, or pharmaceutically acceptable salts, isomers, diastereomers or enantiomers thereof.

Other more preferred compounds of general formula (I) are whose, wherein

$R^1$ is a propargyl group,
$R^2$ is a hydrogen, methyl, ethyl, allyl, propargyl, hydroxyethyl or hydroxypropyl group,
$R^3$ is a methyl or progargyl group,
$R^5$ is a methyl, ethyl, allyl, or hydroxylethyl, or pharmaceutically acceptable salts, isomers, diastereomers or enantiomers thereof.

Even more preferred compounds of general formula (I) are whose, wherein

$R^1$ is a propargyl group,
$R^2$ is a methyl, ethyl or hydroxypropyl group,
$R^4$ is a phenyl substituted with one or more methoxy groups or a meta- methylphenyl group, or pharmaceutically acceptable salts, isomers, diastereomers or enantiomers thereof.

Even more preferred compounds of general formula (I) are whose, wherein

$R^1$ is a propargyl group,
$R^3$ is a methyl group,
$R^4$ is phenyl substituted with one or more methoxy groups, or pharmaceutically acceptable salts, isomers, diastereomers or enantiomers thereof.

Most preferred compounds of general formula (I) are whose, wherein

$R^1$ is a propargyl group,
$R^2$ is an ethyl or hydroxypropyl group"
$R^4$ is phenyl substituted with two methoxy groups,

or pharmaceutically acceptable salts, isomers, diastereomers or enantiomers thereof.

Also object of the present invention are those compounds which result when possible, preferable, more preferable and most preferable meanings of the substituents are combined.

Additionally preferred compounds are those which results when exemplified meanings of the substituents are combined.

In one embodiment the present invention relates to pharmaceutical compositions comprising compounds according to formula (I), wherein $R^1$, $R^2$, $R^3$ and $R^4$ has the meanings shown in table 1.

Most preferred is a compound selected from the group consisting of: 3-Ethyl-8-(3,4-dimethoxyphenylethynyl)-7-methyl-1-prop-2-ynyl-3,7-dihydropurine-2,6 dione. This compound induces a potent hyperlocomotion in the Irwin Test. This compound also significantly reduced catalepsy time in acute CGS-21680 and Reserpine-induced catalepsy models. These models are e.g. described by Ferré S. et al., Neurosci. Let., 1991, 130, 162; Ferré S. et al., Neuroscience, 1992, 51, 501; Kafka S.H. et al., Eur. J. Pharma col., 1996, 295, 147; Rimondini R. et al., Neuropsychopharmacology, 1997, 17, 82 and are herein incorporated by reference.

Most preferred is also a compound compound selected from the group consisting of: 3-(3-Hydroxypropyl)-8-(3,4-dimethoxyphenylethynyl)-7-methyl-1-prop-2-ynyl-3,7-dihydropurine-2,6-dione. This compound induces a potent hyperlocomotion in the Irwin Test. This compound also reduced catalepsy time in acute CGS-21680 and Reserpine-induced catalepsy models.

Most preferred is also a compound compound selected from the group consisting of: 8-[(3,4-Dimethoxyphenyl) ethynyl]-7-methyl-1-prop-2-ynyl-3,7-dihydro-1H-purine-2,6-dione.

Intermediate products used for the production of a compound according to general formula (I) of the invention are those of general formula (II),

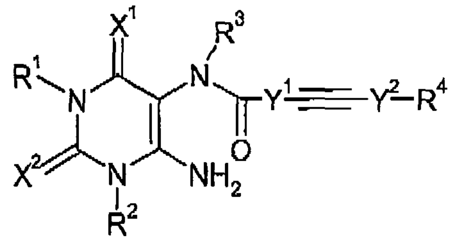

in which $X^1$, $X^2$, $Y^1$, $Y^2$, $R^1$, $R^2$, $R^3$ and $R^4$ have the meanings indicated in one of the claims 1 to 20, as well as their or pharmaceutically acceptable salts, isomers, diastereomers or enantiomers.

Preferably $R^2$ or $R^3$ of general formula (II) stands for a hydrogen. In an even more preferred embodiment of the invention $R^2$ and $R^3$ of general formula (II) stand for hydrogen.

Another embodiment of the present invention is a taumeric form of formula (I) according to formula (III),

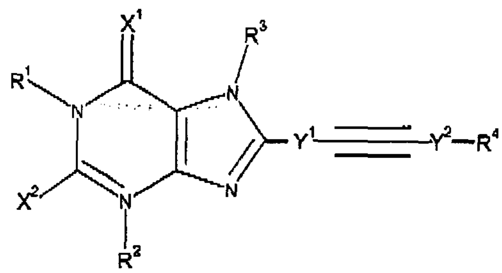

wherein, $X^1$, $Y^1$, $Y^2$, $R^1$, $R^2$, $R^3$ and $R^4$ have the meanings indicated in one of the claims 1 to 20 and wherein $X^2$ is halogen, $(C_1\text{-}C_6)$-alkyl, $-OR^5$ or $-SR^5$, and

$R^5$ is a hydrogen, $(C_1\text{-}C_6)$-alkyl, $(C_2\text{-}C_6)$-alkenyl, $(C_2\text{-}C_6)$-alkinyl, or $(C_1\text{-}C_6)$-alkyl substituted in one or more places, in the same way or differently, with hydroxyl, $(C_1\text{-}C_6)$-alkoxy, aryl or $-NR^6R^7$, or pharmaceutically acceptable salts, isomers, diastereomers or enantiomers thereof.

$R^5$ in the general formula (III) stands preferably for hydrogen, allyl, propargyl, or $(C_1\text{-}C_6)$-alkyl optionally substituted with $(C_1\text{-}C_3)$-alkoxy.

More preferably $R^5$ of general formula (III) stands for $(C_1\text{-}C_3)$-alkyl.

The tautomeric a taumeric form of formula (I) according to formula (III) can be produced according to the methods known to the skilled person in the art, such as e.g. by Muller et al., Synthesis, 1998, 1428. This document is herein incorporated by reference.

The compounds of formula (I) can be used as pharmaceutical compounds based on their selective antagonistic activity relative to the $A_{2A}$ adenosine receptor. The compounds according to general formula (I) exhibit more than 3-fold, preferably a more than 5-fold, more preferably a more than 10-fold, even more preferably a more than 50-fold, most preferably a more than 100-fold. Selectivity versus the $A_1$ adenosine receptor is desired e.g. due potential negative effects of the $A_1$ adenosine receptor on cardiac and kidney functions and in the central nervous system.

Based on their profile of action, the compounds according to the invention are suitable for preventing or treating diseases such as Parkinson's disease (PD), catalepsy, dystonia, dyskinetic syndrome, restless legs syndrome, migraine, pain, dementia, neurodegenerative disorders, alcohol withdrawal and/or ischemic conditions such as e.g. stroke or cardiac ischemia. Preferably the compounds are used to treat PD.

Subjects of this invention are also pharmaceutical composition comprising a compound according to general formula (I). The pharmaceutical composition is useful for preventing or treating the above-cited diseases. In a preferred embodiment of the invention, the pharmaceutical composition comprises additional suitable pharmaceutically acceptable carriers.

Suitable pharmaceutically acceptable carriers depend on the pharmaceutical form and are known by a person skilled in the art.

As used herein, "pharmaceutically acceptable carriers" includes any and all solvents and solvent mixtures,

dispersion media, complexation agents, surface active excipients, solid carriers, coatings, antibacterial and antifungal agents, isotonic and absorption delaying agents for pharmaceutically active substances and mixtures thereof, as well known in the art.

Examples for pharmaceutically acceptable carriers are selected from the group consisting of gelatine, lactose, sugar alcohols, e.g. mannitol, starch, e.g. corn starch, magnesium stearate, talc, vegetable oil, microcrystalline cellulose, carboxymethyl-cellulose polysorbate, sodium lauryl sulphate, colloidal silicon oxide, copolyridone, water, buffered aqueous solutions, ethanol, polyalkylene glycols, preferably polyethylene glycols, e.g. PEG 400, propylene glycol, Tween® 80 (i.e. PEG (20) sorbitol monooleate), DMSO, mixtures of water and co-solvents, e.g. aqueous solutions comprising alcohols like ethanol and/or polyalkylene glycols like polyethylene glyol, complexation agents like cyclodextrines, e.g. α-cyclodextrine, (α-CD) or hydroxypropyl-β-cyclodextrine (HP-β-CD), surface tants like anionic, kationic, non-ionic and amphoteric surfactants, salts of bile acids or lipids, e.g. animal or vegetable phospholipids, esters of polyols like glycerol and/or polyethylene glycol with fatty acids, micelles forming agents, and oils like corn oil, or mixtures of two or more of the components mentioned before.

Further suitable pharmaceutically acceptable carriers as well as suitable additives which may be used in the compositions of the present invention are mentioned below.

In one embodiment the present invention relates to pharmaceutical compositions of the present invention forming in aqueous media lipid-based drug delivery systems (DDS). Said pharmaceutical compositions comprise at least one surfactant beside the at least one compound of formula (I) or salt thereof. Suitable surfactants are mentioned above- The lipid-based drug delivery systems may form the following structures:

- micelles, microemulsions, emulsions (i.e. simple self-assembly structures of lipids and surfactants)
- liposomes (i.e. dispersed closed bilayer assembleys of a lamellar phase in water)
- nanoparticles of non-lamellar phases (e.g. cubic, hexagonal, sponge).

Preferably , the lipid-based drug delivery systems can form micelles, microemulsions or emulsions. The HLB-value (hydrophil-lipophil-balance) of suitable surfactants or surfactant mixtures for the formation of micelles, microemulsions or emulsions is in general of from 8 to 18, preferably 10 to 18, more preferably 12 to 16. The lipid-based drug delivery systems form an SEDDS (self-emulsifying drug delivery system) or an SMEDDS (self-microemulsifying drug delivery system). SEDDS and SMEDDS are mixtures, ideally isotropic, of oil(s) (i.e. lipid(s), e.g. a compound of formula (I) or salts thereof, at least one surfactant, optionally at least one co-surfactant and optionally at least one co-solvent, which emulsify spontaneously to produce fine oil-in-water emulsions when introduced into an aqueous phase under gentle agitation. The gentle agitation may be for example provided by gastric mobility. Such self-emulsifying drug delivery systems or self-microemulsifying drug delivery system are preferably used.

The pharmaceutical compositions may comprise further excipients and/or additives. Suitable further excipients and/or additives are mentioned before and below.

The compounds of formula (I) or the pharmaceutical composition may be administered in a convenient manner, such as by oral, intravenous, intramuscular, intrathecal or subcutaneous routes. Enteral, parenteral or oral administration is preferred. Most preferred is oral administration.

The compound of formula (I) may be orally administered, for example, with an inert diluentor with an assimilable edible carrier or it may be enclosed in capsules, or it may be compressed into tablets, or it may be incorporated directly into the food of the diet. For oral therapeutic administration, the active compound of formula (I) may be incorporated with excipients and used in the form of ingestible tablets, buccal tablets, troches, capsules, elixirs, suspensions, syrups, wafers, pills, soft gel caps, powders, solutions, dispersions, liquids and the like. Such compositions and preparations should contain at least 1 % of active compound of formula (I). The percentage of the compositions and preparations may, of course, be varied and may conveniently be between about 5 to about 80 % of the weight of the unit.

The tablets, troches, pills, capsules and the like may also contain the following: A binder such as gum tragacanth, acacia, corn starch or gelatine; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier.

Various other materials may be present as coatings or otherwise modify the physical form of the dosage unit. For instance, tablets, pills, or capsules may be coated with shellac, sugar or both. A syrup or elixir may contain the compound of formula (I), sucrose as a sweetening agent, methyl and propylparabens as preservatives, a dye and flavouring such as cherry or orange flavour.

Of course, any material used in preparing any dosage unit form should be pharmaceutically pure and substantially non-toxic in the amounts employed.

In one embodiment of the invention the compound of formula (I) is included in a capsule. The capsule can be a hard or soft shell capsule. The capsule can be made from any suitable film forming material comprising e.g. gelatine,

cellulose derivatives, pullulan or other glucans, polyvinyl alcohol, pectin, modified starches, such as starch ethers and oxidized starch, more particularly hydroxyethylated starch (HES) or hydroxypropylated starch (HPS) - alone or mixtures thereof and if appropriate in a mixture with a setting system or further components. The cellulose derivatives used for the manufacture of capsules include, but are not limited to, hydroxypropyl cellulose, hydroxyethyl cellulose, hydroxypropyl methyl cellulose, hydroxymethyl cellulose, methylcellulose, ethyl cellulose, cellulose acetate, cellulose acetate phthalate, cellulose acetate trimelliate, hydroxypropylmethyl cellulose phthalate, hydroxypropylmethyl cellulose succinate, carboxymethyl cellulose sodium, and mixtures thereof. Preferred cellulose derivatives are hydroxypropyl cellulose, hydroxyethyl cellulose, hydroxypropyl methyl cellulose, hydroxymethyl cellulose, methylcellulose, and ethyl cellulose.

In addition, the compound of formula (I) may be incorporated into sustained-release preparations and formulations (retard compositions). For example, sustained release dosage forms are contemplated wherein the compound of formula (I) is bound to an ion exchange resin which, optionally, can be coated with a diffusion barrier coating to modify the release properties of the resin.

The compound of formula (I) may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the compound of formula (I) in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique.

It is especially advantageous to formulate the pharmaceutical compositions of the present invention in dosage unit form for ease of administration and uniformity of dosage. Dosage unit form as used herein refers to physically discrete units suited as unitary dosages for the mammalian subjects to be treated; each unit containing a predetermined quantity of the compound of formula (I) calculated to produce the desired therapeutic effect in association with the required pharmaceutical carrier. The specifics for the novel dosage unit forms of the invention are dictated by and directly dependent on (a) the unique characteristics of the compound of formula (I) and the particular therapeutic effect to be achieved, and (b) the limitations inherent in the art of compounding the compound of formula (I) for the treatment of diseases in patients having a disease condition in which bodily health is impaired.

The compound of formula (I) is compounded for convenient and effective administration in effective amounts with a suitable pharmaceutically acceptable carrier and optionally further suitable additives and excipients in dosage unit form as hereinbefore described. The dosage of the compound of formula (I) is depending on the way of administration, age and weight of the patient, kind and severeness of the disease to be treated, etc. The daily dosage calculated as entacapone is in general from 200 to 2000 mg/d preferably from 800 to 1800 mg/d, more preferably from 100 to 1600 mg/d. The daily dose may be administered in one single dosage unit per day or in two or more dosage units per day.

**Tables and Figures**

| | |
|---|---|
| Table 1: | Overview of compounds of the present invention |
| Table 2: | Data of radioligand assay 1; data of human receptors |
| Table 3: | Data of radioligand assay 1; data of rat receptors |
| Table 4: | Data of radioligand assay 2 including the $A_{2B}$ adenosine receptor (and partly with other ligands) |
| Table 5: | Data of the sodium chloride-shift-experiment |
| Table 6: | Data of functional assay concerning inhibition of $A_1$- and $A_{2A}$-adenosine receptors |

| | |
|---|---|
| Table 7: | Methods and behavioural parameters of the functional observational battery (FOB) / modified Irwin test |
| Table 8: | Methods and behavioural parameters of the short animal check (SAC) / modified Irwin test |
| Table 9: | Methods and behavioural parameters of the follow-up observation (FU) / modified Irwin test |
| Table 10: | Compound data / modified Irwin test |
| Figure 1: | Overview of the acute CGS-21680 induced catalepsy and acute Reserpine induced catalepsy tests (time schedule / application of compounds) |
| Figure 2: | CGS-21680 induced catalepsy time with control (vehicle/vehicle) and reference compound caffeine |
| Figure 3: | Reserpine induced catalepsy time with control (vehicle/vehicle) and reference compound apomorphine |
| Figure 4a & 4b: | Significant reduction of catalepsy time in acute CGS-21680 induced catalepsy model with test compound 1 (=3-Ethyl-8-(3,4-dimethoxyphenylethynyl)-7-methyl-1-prop-2-ynyl-3,7-dihydropurine-2,6 dione) |
| Figure 5a & 5b: | Significant reduction of catalepsy time in acute Reserpine induced catalepsy model with test compound 1 |
| Figure 6a & 6b: | Reduction of catalepsy time in acute CGS-21680 induced catalepsy model with test compound 2 (=3-(3-Hydroxypropyl)-8-(3,4-dimethoxyphenylethynyl)-7-methyl-1-prop-2-ynyl-3,7-dihydropurine-2,6-dione) |
| Figure 7a & 7b: | Significant reduction of catalepsy time in acute Reserpine induced catalepsy model with test compound 2 |

**Synthesis Scheme**

The compounds according to formula (I) of the present invention may be prepared by any process known by a skilled in the art. In preferred embodiments of the present invention the compounds according to formula (I) are prepared according to four different general methods (A to C)

*Method A*

According to this method an amide bond was formed by the reaction of step (a), followed by selective substitution at the N1-atom (step (b)). Subsequently, the ring is closed with dehydrating reagents (step (c)) followed by substitution at the N7-atom (step (e)). This method (except step c)) is described by Muller C.E. et al., Eur. J. Med. Chem. 1997, 32, 709-719 and is incorporated herein by reference.

The reaction is shown in the scheme below:

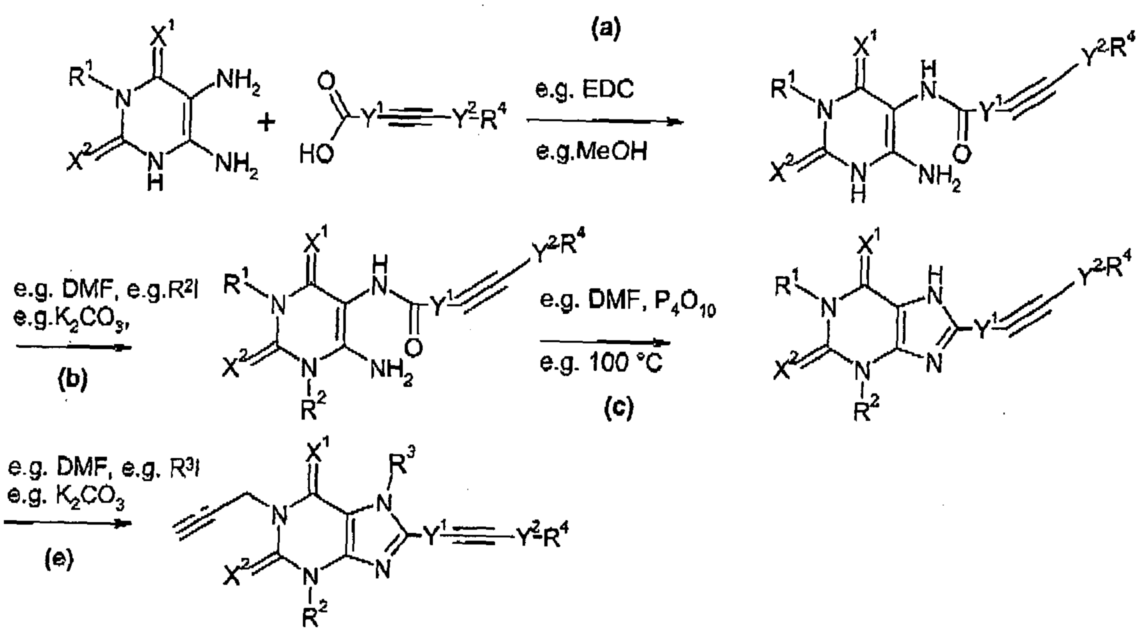

wherein $X^1$, $X^2$, $Y^1$, $Y^2$, $R^1$, $R^2$, $R^3$ and $R^4$ are defined as mentioned above.

Step (a): The reaction with the 5-amino group of the 5,6-diaminodihydropyrimidine derivatives with differently substituted substituted carboxylic acids can be conducted similarly to the process described by Muller C.E, et al., Eur. J. Med. Chem. 1997, 32. 709-719. For this amide bond forming step methanol or another suitable solvent in the presence of a condensation agent such as e.g. (dimethylaminopropyl)ethylcarbodiimide x HCl (EDC), other carbodiimides, or other suitable amide coupling reagents and methods can be used. The step can be conducted at room temperature and takes seldom longer than 3 hours.

Step (b) An additional substituent (e.g. an alkyl group) is added at position N1 of the intermediate obtained through step (a). The intermediate can be suspended in DMF or another preferably polar solvent. The reaction can e.g. be conducted at room temperature (up to 60°C) with $R^2$ substituted halogenides (e.g. iodide or bromide) in the presence of potas sium carbonate or another suitable base. When using other less reactive e.g. alkylating agents higher temperatures may be required. The intermediates obtained through this step can for example be precipitated with water optionally in the presence of sodium chloride and dichloromethane.

Step (c) The intermediate obtained through step (b) is dissolved (e.g. in DMF). The ring forming step occurs at a temperature of between 70°C - 130°C, preferably at 100°C with an excess of phosphorus pentoxide ($P_4O_{10}$) within a few minutes. Alternatively, other suitable conditions and/or dehydrating reagents can be used for the ring closure reaction.

Step (e) The addition of a substituent at position 7 of the intermediate obtained through step (c) is conducted in analogy to the reaction described under step (b). Further purification of the products may be conducted with column chromatography and/or crystalline transformation (e.g. with dichloromethane/petrol ether).

*Method B*

According to this method, the ring-closing step (a(1)) or alternatively (a(2)) directly follows the above described amide bond forming step (step (a) in method A). Substitution at position N3 and N7 can be done in one step (step (b)).

The reaction is shown in the scheme below:

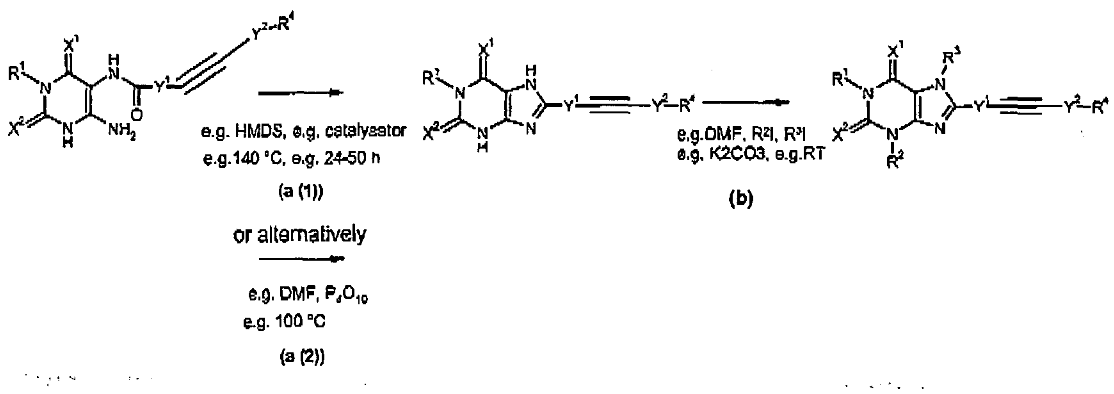


wherein $X^1$, $X^2$, $Y^1$, $Y^2$, $R^1$, $R^2$, $R^3$ and $R^4$ are defined as mentioned above.

Step (a (1)) The ring forming step can be conducted in analogy to described procedures using hexamethyldisilazane (HMDS) as condensing agent (Burbiel et al., 2006, Beilstein J. Org. Chem. 6, 1375). The intermediate obtained through step (b) is therefore suspended in HMDS or other silylating agents in the presence or absence of catalysts such as trimethylchlorosilane, p-toluenesulfonic acid, ammonium sulphate. The reaction takes place at various temperatures, preferably at 120°C -160°C, more preferably at 140°C within preferably 24-50 hours. The reaction can be accelerated by microwave heating. Other water detracting agents such as polyphosphoric acid trimethylsilylester (PPSE) or NaOH, dioxane/water can be used, However, decomposition of the reactant might occur. or alternatively

Step (a (2)) This step is similar to the Step (c) of method A.

Step (b) This step is performed in analogy to the steps (b) and (e) of method A.

*Method* C

Step (a (1)) or step (a (2)) from method B are used to close the ring. Afterwards the N7 position is selectively

substituted (step a) of method C). Optionally, the N7-substituted intermediate obtained through step a) can optionally be further purified by column chromatography. Step b) describes the substitution at N3.

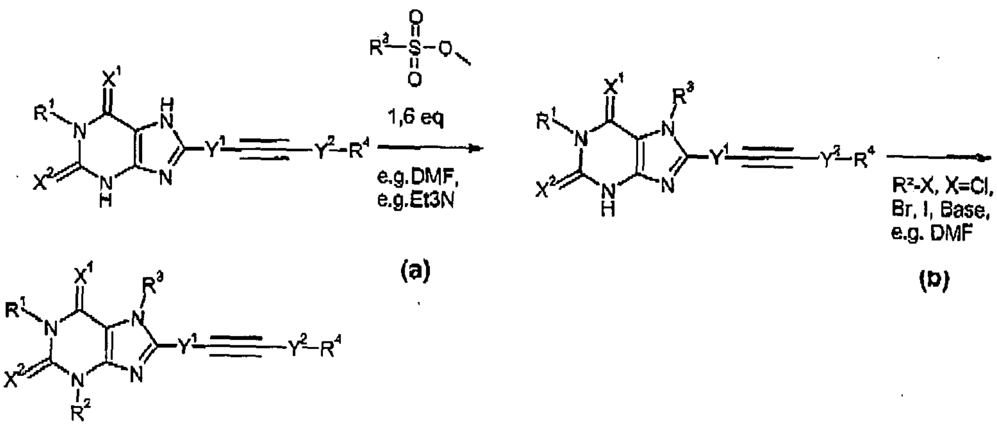


wherein $X^1$, $X^2$, $Y^1$, $Y^2$, $R^1$, $R^2$, $R^3$ and $R^4$ are defined as mentioned above.

Step (a) N7- selective addition of a subsistuent after the ring closing step described in method B (step (a (1)) or step (a (2))) occurs in the presence of 1.6 eq of substituted sulfonicacidmethylester (e.g. methylmesylate), a solvent such as DMF and a base, preferabl $Et_3N$, preferably at room temperature within 1 hour. The intermediate(s) obtained through this step can optionally be purified by column chromatography. Therefore, the intermediate reacts with an excess of pivaloyloxymethyl chloride, DMF, $K_2CO_3$ at room temperature for 1 ½ hours to different POM-substituted intermediates which can be easily separated by column chromatography (e.g. with petrol ether/acetic acid 7/3). Alternatively, other protecting groups can be introduced by suitable methods known to the skilled person in the art. The N3-POM group can then be cleaved off under basic conditions, e.g. with MeOH, THF, LiOH at room temperature within 3-4 hours.

Step (b) A substituent is then introduced at N3 of the intermediate obtained through step (a) with substituted halogenides (or compound with another suitable leaving group) in the presence of a base and DMF or another suitable, preferably polar solvent, at room or elevated temperatures.

*Starting Substances:*

If the production of the starting compounds is not described, the latter are known or can be produced in a way that is similar to known compounds or processes that are described here.

5,6-diaminodihydropyrimidine derivatives:

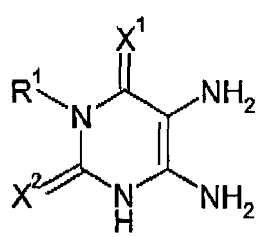

wherein $X^1$, $X^2$ and $R^1$ are defined as mentioned above.

E.g. 5,6-Diamino-3-propargyluracil can be synthesized as described in the literature (Muller, Tetrahedron Lett., 1991, 32, 6539; Muller et al., J. Med. Chem., 1997, 40, 4396, herein incorporated by reference). 1,3-Disubstituted 5,6-diaminouracil are synthe sized as previously described (e.g. Muller et al., J. Med. Chem. 1993, 36, 3341, herein incorporated by reference).

Substituted carboxylic acids:

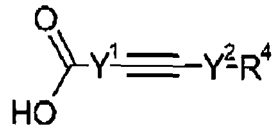

wherein $Y^1$, $Y^2$ and $R^4$ are defined as mentioned above.

The compounds can for example be synthesized as described below (method 1, 2 and 3). In method 1 alkynes are treated with butyl lithium at low temperatures followed by quenching with CO2. In method 2 carboxylic acids containing a double bond in the α-position are brominated followed by dehydrohalogenation leading to the formation of a triple bond. Method 3 uses a Wittig reaction starting from the aldehydes to yield the target acetylene carboxylic acids.

Phenylacetylenecarboxylic acid is commercially available. Other arylacetylencarboxylic acids can e.g. synthesized as follows:

**Method 1**

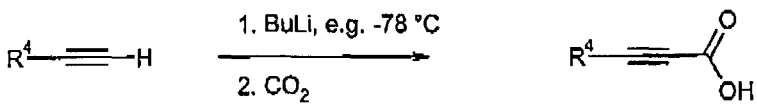


**Method 2**

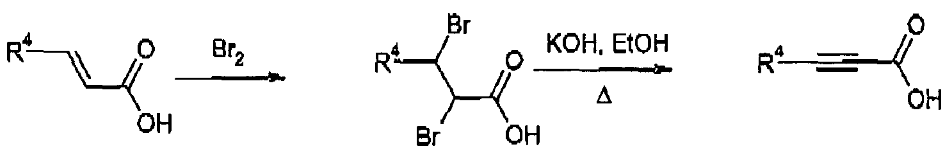


**Method 3**

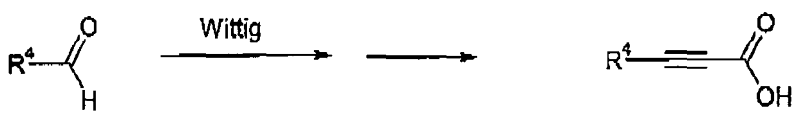


wherein $R^4$ is an optionally substituted aryl as mentioned above (see definition of $R^4$ for further explanation of the possible substituents).

Method 1) : Starting material: arylacetylene. Lithiation of the arylacetylen at low temperatures and reaction of the intermediate with solid carbon dioxyde.

Method 2) Starting material: cinnamic acid. Bromination of cinnamic acid and dehydrohalogenation with a strong base (e.g. potassium hydroxide).

Method 3) Starting material: aldehyde. Reaction with special Wittig-like reagents and subsequent catalysed eliminiation reaction with a base.

All three methods are known to the skilled person in the art. For the herein described compounds, method 1 and 2 were used.

Synthesis 1 of 3,4-dimethoxyphenylacetylenecarboxylic acid:

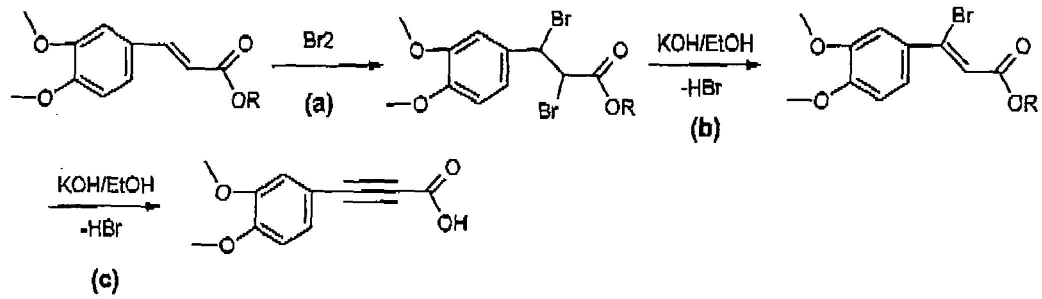


Synthesis 2 of 3,4-dimethoxyphonylacetylenecarboxylic acid:

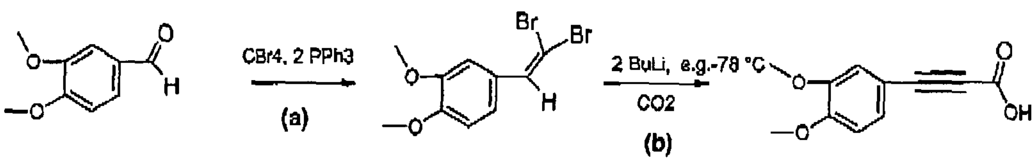


The present invention is illustrated by the following non-limiting examples.

**Examples**

**5.6-diamino-3-prop-2-ynyl-1H-pyrimidine-2,4-dione:** were prepared as described in the literature. Muller, Tetrahedron Lett., 1991, 32, 6539; Muller et al., J. Med. Chem., 1997, 40, 4396, Hockemeyer et al., 2004, J. Org. Chem. 69, 3308). These documents are herein incorporated by reference.

**3-arylpropynoic acids:** were prepared as described in the literature.

**Preparation of 3-arylpropynoic acid 6-amino-2,4-dioxo-3-prop-2-ynyl-1,2,3,4-tetrahydropyrimidin-5-yl amides (according to method A)**

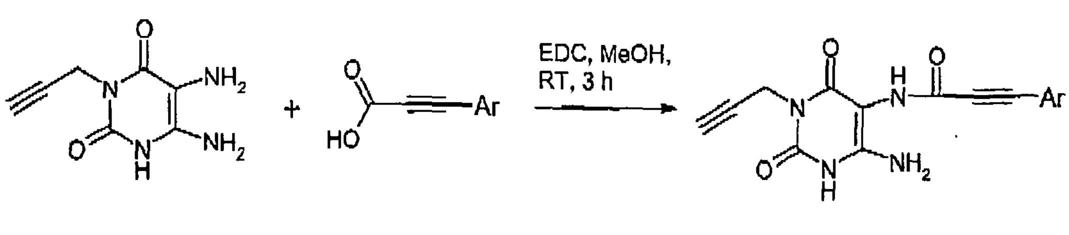


A mixture of 3 mmol of the corresponding 3-arylpropynoic acid, 3 mmol of freshly pre-A mixture of 3 mmol of the corresponding 3-arylpropynoic acid, 3 mmol of freshly prepared 5,6-diamino-3-prop-2-ynyl-1H-pyrimidine-2,4-dione and 3.2 mmol of (3-dimethylaminopropyl)ethylcarbodiimide hydrochloride (EDC) in 40 mL of methanol was stirred for 5 h at rt. The precipitate was filtered under reduced pressure and washed with 40 mL of methanol and dried at 70 ˚C. TLC analysis (eluent: CH2Cl2: methanol = 5:1 or 7:1) of the products showed two spots indicating two stable tautomers which can also be observed in the NMR spectra. Ar = Aryl.

**Preparation of 3-arylpropynoic acid 6-amino-1-methyl-2,4-dioxo-3-prop-2-ynyl-1,2,3,4-tetrahydropyrimidin-5-yl amides (according to method A)**

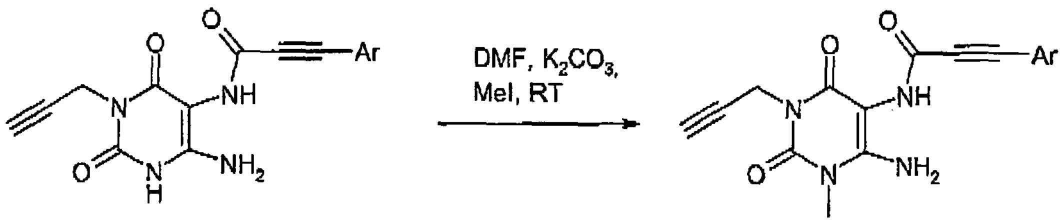


A solution of 1.5 mmol of the N1 unsubstituted 3-arylpropynoic acid 6-amino-2,4-dioxo-3-prop-2-ynyl-1,2,3,4-tetrahydropyrimidin-5-yl amide, 2 mmol of dry potassium carbonate and 5 mmol of methyl iodide in 5 mL of dry DMF was stirred at room temperature until no further starting material could be detected by TLC (eluent: $CH_2Cl_2$/methanol, 7:1). The TLC analysis showed two spots for the product tautomers. The product was precipitated by adding 40 mL of water, filtered under reduced pressure, washed with 50 mL of water and dried al 70 ˚C. Ar = Aryl.

**Formation of 8-arylethynyl-1-prop-2-ynyl-3,7-dihydropurine-2,6-diones and 8-arylethynyl-3-methyl-1-prop-2-ynyl-3,7-dihydropurine-2,6-diones (according to method A)**

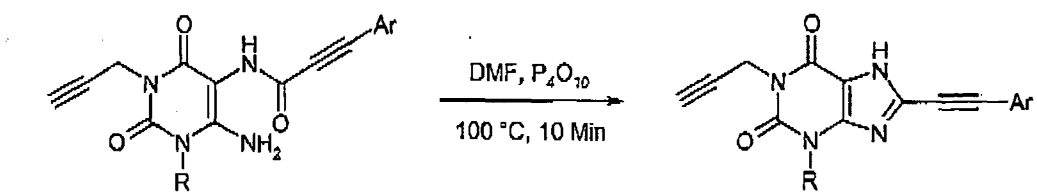


R = H or Me

To a solution of 1 mmol of the corresponding 3-arylpropynoic acid 6-amino-2,4-dioxo-3-prop-2-ynyl-1,2,3,4-tetrahydropyrimidin-5-yl amide or respectively of the N1-methylated derivative in 5 mL of DMF was added phosphorous pentoxyde (ca. 1.2 g, 8 mmol) under stirring. The stirred mixture was heated at 100 ˚C for 5 min and cooled to room temperature. The product was precipitated by adding water (30 mL), filtered under reduced pressure, washed thoroughly with water and dried at 70 ˚C. Ar = Aryl.

**Methylation of 8-Arylethynyl-3-methyl-1-prop-2-ynyl-3,7-dihydropurine-2,6-diones to 8-Arylethynyl-3,7-dimethyl-1-prop-2-ynyl-3,7-dihydropurine-2,6-diones (according to method A)**

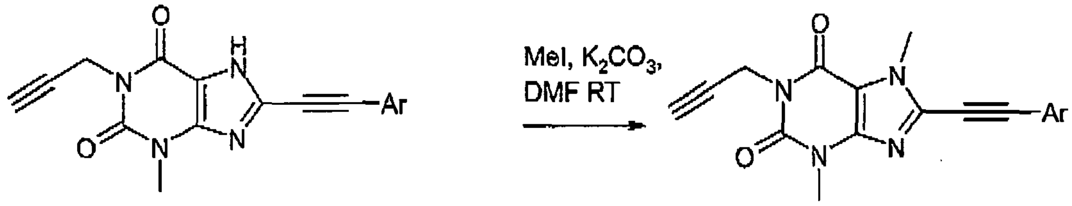

A suspension of 1 mmol of the corresponding 8-arylethynyl-3-mothyl-1-prop-2-ynyl-3,7-dihydropurine-2,6-dione, K2CO3 (1.2 mmol) and 2 mmol of methyl iodide in DMF (20 mL) was stirred at room temperature for 0.5 h (TLC control: eluent CH2Cl2/methanol, 9.5:0.5). Subsequently the product was precipitated by adding water (50 mL), filtered off under reduced pressure, washed with water (150 mL) and dried at 70˚C. Further purification was achieved, unless otherwise noted, by dissolving the crude product was in dichloromethane. After filtration and concentration the residue was dissolved in a minimum of dichloromethane and recrystallized by adding petroleum ether. Ar = Aryl.

**3,7-Dimethyl-8-(3-nitrophenylethynyl-1-prop-2-ynyl-3,7-dihydropurine-2,6-dione** For purification (Method A) the crude product was entirely dissolved in chloroform, crystallized by adding ether, filtered under reduced pressure and washed with ether: light yellow crystals (yield 85 %), m.p. 286-289 ˚C (dec).

**Alkylation of 8-Arylethynyl-3-methyl-1-prop-2-ynyl-3,7-dihydropurine-2,6-diones to 7-Alkyl-8-arylethynyl-3-methyl-1-prop-2-ynyl-3,7-dihydropurine-2,6-diones (accord ing to method A)**

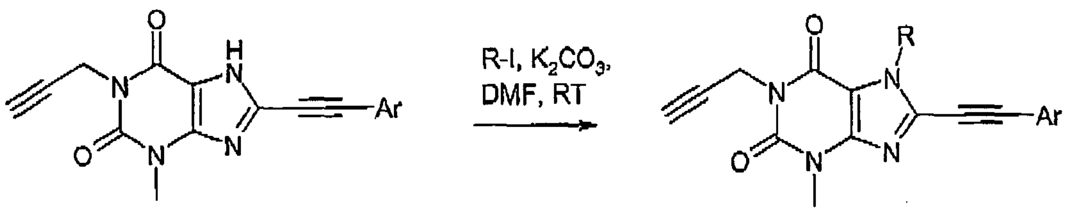


General procedure for reactive alkylation reagents (alkyl iodides)
To a suspension of 0.1 mmol of the corresponding arylethynyl-3-methyl-1-prop-2-ynyl-3,7-dihydropurine-2,6-dione and K2CO3 (0.2 mmol) in 2 mL of DMF were added 0.5 mmol of the corresponding alkyl halogenide. The mixture was stirred at room temperature until no further starting material could be observed by TLC analysis (eluent: dichloromethane/methanol, 9.5:0.5). Subsequently the product was precipitated by adding water (25 mL), filtered under reduced pressure and dried at 70 ˚C. Further purification was achieved by column chromatography on silica gel (eluent CH2Cl2/methanol, 9.5:0.5) and/or recrystallization from dichloromethane/petroleum ether. Ar = Aryl.

**Methylation of 8-Arylethynyl-1-prop-2-ynyl-3,7-dihydropurine-2,6-diones to 8. Arylethynyl-3,7-dimethyl-1-prop-2-ynyl-3,7-dihydropurine-2,6-diones** ((according to **method B)**

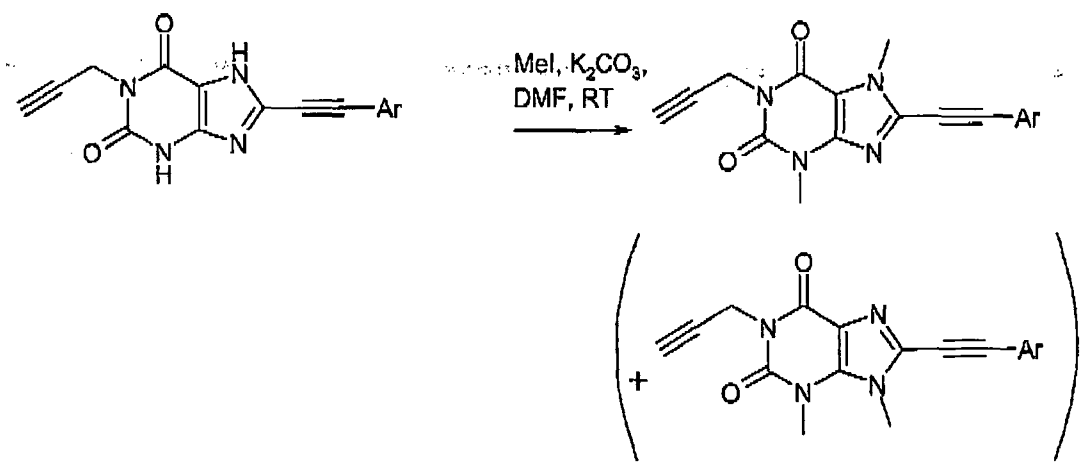


A suspension of 0.5 mmol of the corresponding 8-arylethynyl-1-prop-2-ynyl-3,7-dihydropurine-2,6-dione, K2CO3 (1.2 mmol) and 2 mmol of methyl iodide in DMF (15 mL) was stirred at room temperature for 0.5 h (TLC control:

eluent CH2Cl2/methanol, 9.5:0.5). Subsequently the product was precipitated by adding water (50 mL), filtered under reduced pressure, washed with water (150 mL) and dried at 70 °C. Further purification was achieved by column chromatography on silica gel (eluent CH2Cl2/methanol, 9.5:0.5) and subsequent recrystallization from dichloromethane/petroleum ether as described above. As side-procucts the corresponding arylethynyl-3,9-dimethyl-1-prop-2-ynyl-3,9-dihydropurine-2,6-diones could be isolated in low yields. Ar = Aryl.

**Other 1-Alkyl-8-arylethynyl-3,7-dimethyl-3,7-dihydropurine-2,6-diones (according to method A)**

All other 1-alkyl-8-arylethynyl-3,7-dimethyl-3,7-dihydropurine-2,6-diones bearing allyl, ethyl, propyl, butyl or cyclobutylmethyl substituents on the N1 position of the xanthine ring system were prepared analogously to the above mentioned procedures. Ar = Aryl.

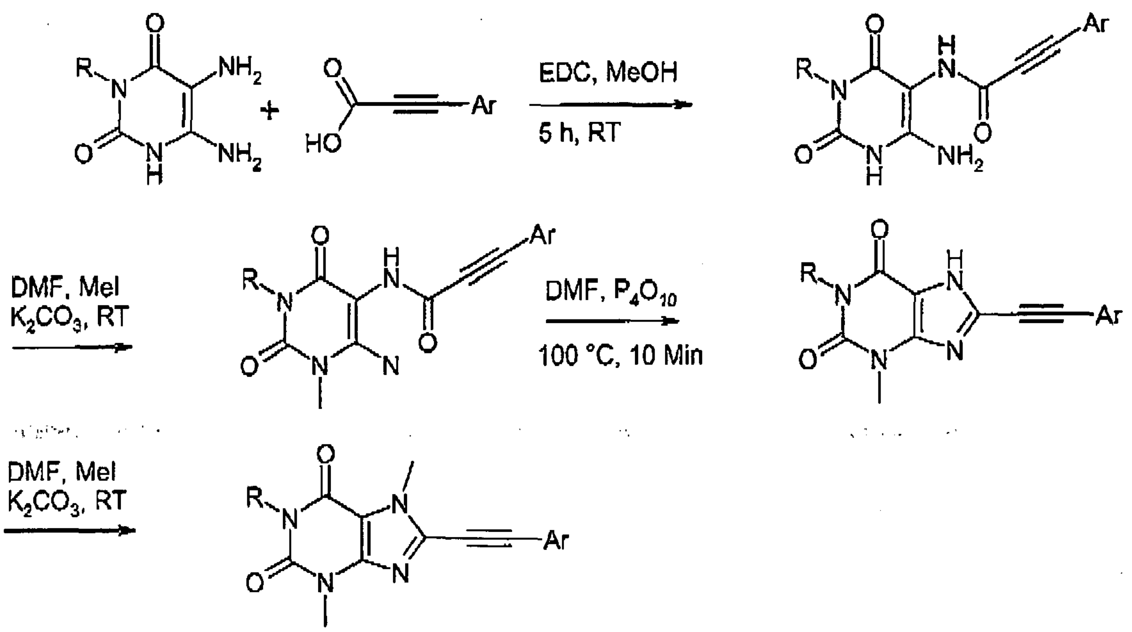


R = Ethyl, Propyl, Butyl, Allyl, Cyclobutylmethyl

**Preparation of 8-(3-Hydroxyphenylethynyl)-3,7-dimethyl-1-prop-2-ynyl-3,7-dihydropurine-2,6-dione**

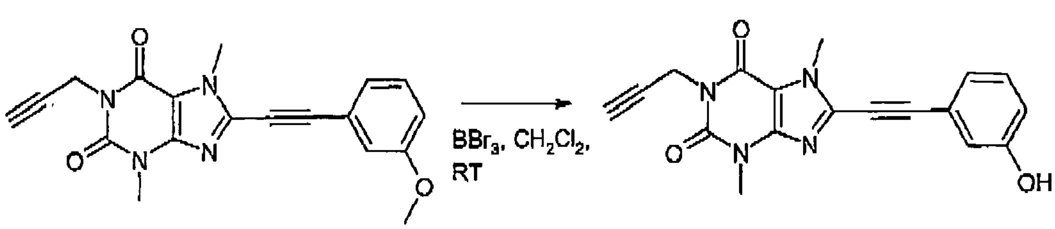


To a solution of 100 mg (0.28 mmol) 8-(3-methoxyphenylethynyl)-3,7-dimethyl-1-prop-2-ynyl-3,7-dihydropurine-2,6-dione in dichloromethane was added boron tribromide (0.5 mL, 5.3 mmol). After e few minutes precipitation of a yellow solid could be observed. The suspension was stirred for 6 h at room temperature until no starting material could be detected by TLC analyses, eluent: dichloromethane/methanol, 9.5:0.5. After hydrolysis with concentrated NaHCO3-solution (50 mL) the dichloromethane was removed under reduced pressure. The precipitate was filtered under reduced pressure, washed with water, dried at 70 °C and purified by column chromatography on silica gel (eluent: dichloromethane/methanol, 9.5:0.5 or 9:1): colorless crystals (yield: 76 %), m.p. 271 °C. Ar = Aryl.

**Alkylation of 8-(3-Hydroxyphenylethynyl)-3,7-dimethyl-9-prop-2-ynyl-3,7-dihydropurine-2,6-dione**

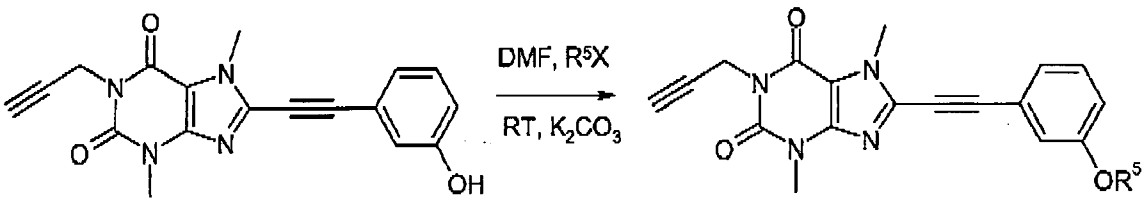


General procedure for reactive alkylation reagents (alkyle iodides)
To a suspension of 0.1 mmol of 8-(3-Hydroxyphenylethynyl)-3,7-dimethyl-1-prop-2-ynyl-3,7-dihydropurine-2,6-dione and K2CO3 (0.2 mmol) in 2 mL of DMF were added 0.5 mmol of the corresponding alkyle halogenide. The mixture was stirred at room temperature until no further starting material could be observed by TLC analysis (eluent: dichloromethane/methanol, 9.5:0.5). Subsequently the product was precipitated by adding water (25 mL), filtered under reduced pressure and dried at 70 °C. Further purification was achieved, unless otherwise noted, by column chromatography on silica gel (eluent CH2Cl2/methanol, 9.5:0.5) and/or recrystallization from dichloromethane/petroleum ether. $R^5$ = as mentioned above.

**Acylation of 8-(3-Hydroxyphenylethynyl)-3,7-dimethyl-1-prop-2-ynyl-3,7-dihydropurine-2,6-dione**

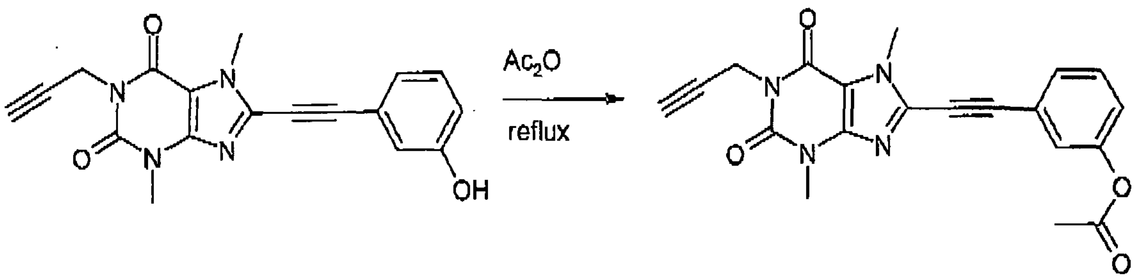


A mixture of 0.1 mmol of 8-(3-Hydroxyphenylethynyl)-3,7-dimethyl-1-prop-2-ynyl-3,7-dihydropurine-2,6-dione and 0.5 mL acetic anhydride was heated under reflux for 15 Min and subsequently hydrolyzed with concentrated NaHCO3-solution (50 mL). The product was extracted two times with 50 mL dichloromethane each. The organic extracts were dried over MgSO4 and filtered and concentrated under vacuum. The crystalline residue was treated with ether and filtered under reduced pressure yielding 8-(3-Acetoxyphenylethynyl)-3,7-dimethyl-1-prop-2-ynyl-3,7-dihydropurine-2,6-dione: off white crystals (yield > 95 %), m.p. 238 °C.

**8-Arylethynyl-1,3-diethyl-3,7-dihydropurine-2,6-diones**

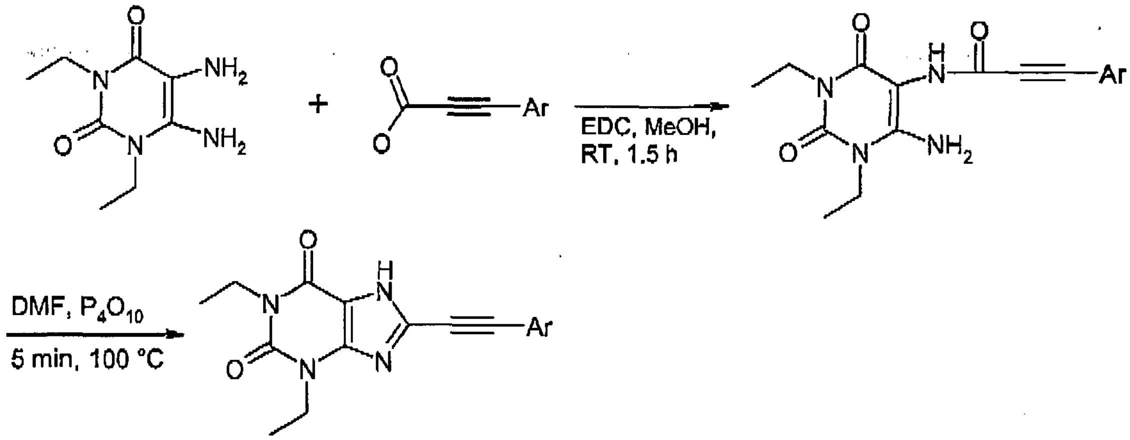


5,6-Diamino-1,3-diethyl-1H-pyrimidine-2,4-dione was prepared as described in the literature. The crude oily 5,6-diaminouracil (prepared from 2 mmol of the corresponding 6-amino-5-nitrosouracil) was dissolved in 15 mL of methanol. Subsequently 2 mmol of the corresponding arylpropynoic acid and 2.5 mmol of EDC were added. The reaction was controlled by TLC analysis (CH2CH2 : MeOH = 7:1). After reaction times of ca. 1.5 h 20 mL of brine and 20 mL of diluted hydrochloric acid was added to the clear solution (in order to dissolved unreacted diaminouracil) and extracted four times with 50 mL of CH2CH2 each. The combined organic extracts were washed with water (50 ml), finally with 50 mL of saturated NaHCO3-solution (in order to remove traces of unreacted carbonic acid), dried over MgSO4, filtered and concentrated under vacuum (the residue at least under 20 mbar at 60-70 ˚C in order to remove traces of water). The DMF containing oily residue was dissolved in additional 5 mL of DMF and treated with an excess of phosphorus pentaoxyde (ca. 1 g). The mixture was heated for 5 min under stirring at 100 ˚C, cooled to room temperature and diluted with water (ca. 30 ml). The precipitate was filtered under vacuum, washed with water (ca. 150 ml) and dried at 70 ˚C. Ar = Aryl.

**8-Arylethyny-1,3-diethyl-7-methyl-3,7-dihydropurine-2,6-diones**

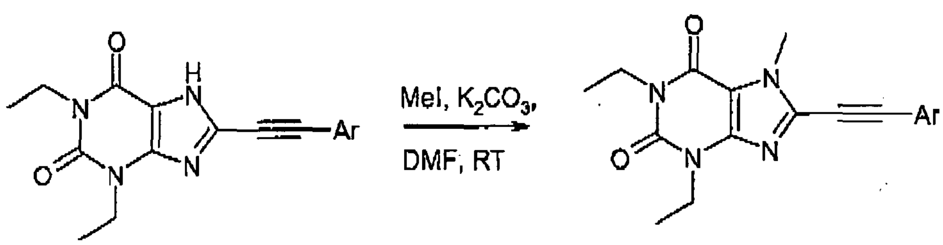


8-Arylethynyl-1,3-diethyl-7-methyl-3,7-dihydropurine-2,6-diones were prepared analogously to the formation of 8-arylethynyl-3-methyl-1-prop-2-ynyl-3,7-dihydropurine-2,6-diones starting from 8-aryfethynyl-3,7-dirnethyl-1-prop-2-ynyl-3,7-dihydropurine-2,6-diones as described above. Ar = Aryl.

**8-Arylethyny-3-(3-hydroxypropyl)-7-methyl-1-prop-2-ynyl-3,7-dihydropurine-2,6-diones**

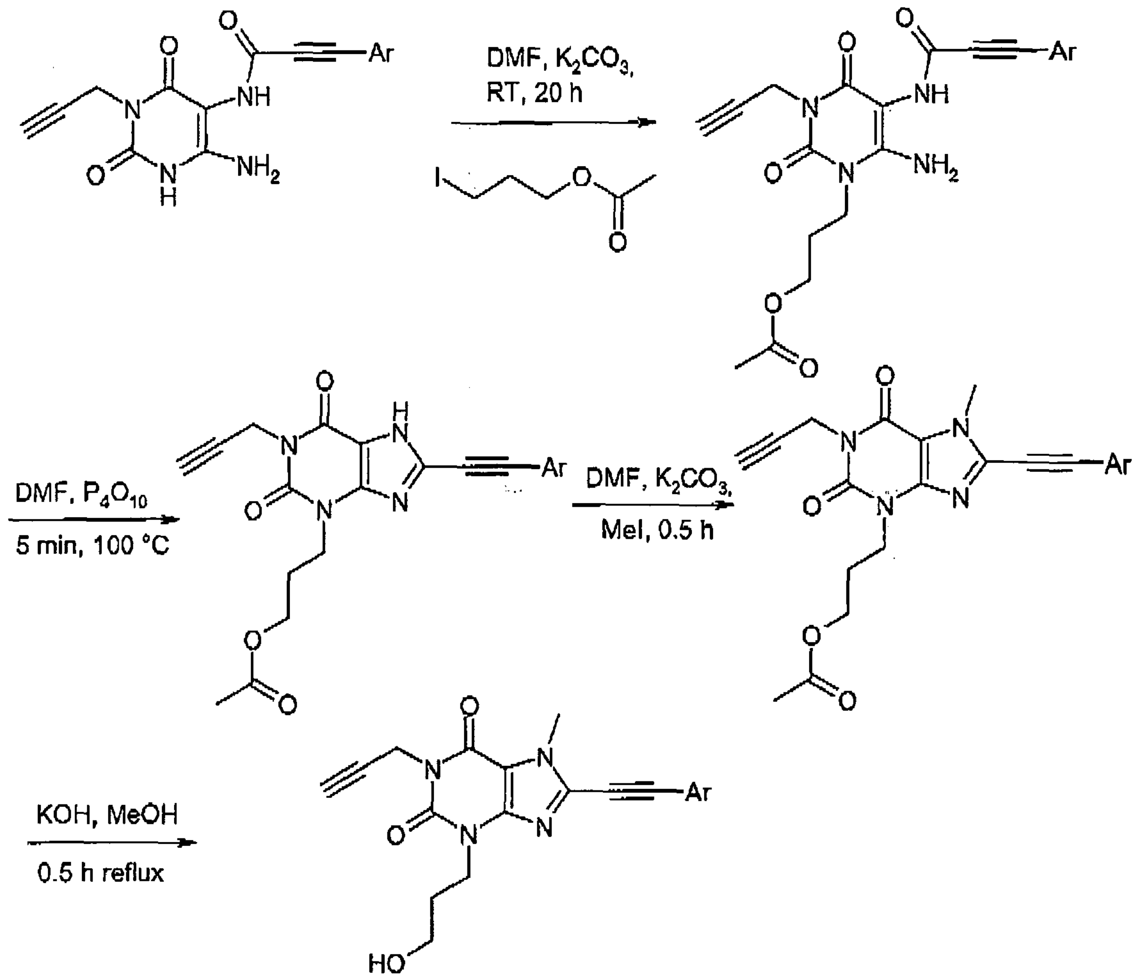


A mixture of the corresponding 3-arylpropynoic acid 6-amino-2,4-dioxo-3-prop-2-ynyl-1,2,3,4-tetrahydropyrimidin-5-yl amide (1 mmol), 250 mg K2CO3 (1.8 mmol), 4 mL of dry DMF and acetic acid-3-iodopropyl ester (0.8 g, 3.5 mmol) was stirred at room temperature until no starting material could be detected by TLC-analysis (CH2CH2 : MeOH = 7: 1). After reaction times of ca. 16-20 h 30 mL of brine was added and the mixture was extracted five times with 50 mL of CH2CH2 each. The combined organic extracts were dried over MgSO4, filtered and concentrated under vacuum (the residue at least under 20 mbar at 60-70 ˚C in order to remove traces of water). The DMF containing oily residue was dissolved in additional 5 mL of DMF and treated with an excess of phosphorus pentaoxyde (ca. 1 g). The mixture was heated for 5 min under stirring at 100 ˚C, cooled to room temperature and diluted with water (ca. 30 ml). In some cases the addition of diethyl ether (5-10 ml) was advantageous in order to dissolve unreacted alkyl iodide. The precipitate was filtered under vacuum, washed with water (ca. 150 ml) and dried at 70 ˚C or directly suspended in 8 mL of DMF and methylated with methyl iodide (0.5 ml) in the presence of K2CO3 (100 mg). After reaction times of less than 0.5 h (TLC-control, CH2CH2 : MeOH = 9:1) the product was precipitated by adding water (ca. 30 ml), filtered under vacuum, and washed with water. The precipitate was directly dissolved in a mixture of methanol (10 ml) and KOH (0.5 g) and refluxed for 0.5 h. After cooling to room temperature, the product was precipitated by adding water (ca. 30 ml), filtered under reduced pressure, washed with water (ca. 100 ml) and dried at 70˚C. Further purification was achieved by column chromatography on slica gel (CH2CH2 : MeOH = 9:1) and subsequent recrystallization from CH2CH2/petroleum ether. Ar = Aryl.

**Alkylation of 8-[(3,4-dimethoxyphenyl)ethynyl]- and 8-[(3-methoxyphonyl)ethynyl]-7-methyl-1-prop-2-ynyl-3,7-dihydropurine-2,6-dione derivatives**

**General Procedure:**

To a mixture of the corresponding 8-(phenylethynyl)-7-methyl-1-prop-2-ynylxanthine derivative (0.1 mmol) and K2CO3 (0.3 mmol) in DMF (3.5 mL) 0.3 mmol of the alkylation reagent was added. The mixture was stirred at room temperature or heated until a complete conversion could be detected by TLC (TLC-control: dichloromethane/methanol, 9.5 : 0.5). Subsequently the product was precipitated by adding water (30 ml), filtered under reduced pressure, washed with water and dried at 70 ˚C. Finally the products were crystallized from dichloromethane/petroleum ether, filtered under reduced pressure and washed with diethyl ether. A further purification was achieved by column chromatography on silica gel (eluent: dichloromethane/methanol, 9.5 : 0.5).

The compounds in table 1 are synthesized analogously to the above described processes.

**Biological Experiments**

*Radioligand Binding Assay 1:*

Rat brain preparations were used for $A_1$- and $A_{2A}$ adenosine receptor (AR) and human recombinant cell samples for $A_1$- and $A_{2A}$- and $A_3$ adenosine receptor radioligand binding studies. Affinity and selectivity was tested in displacement experiments with following radioligands: [$^3$H]CCPA for $A_1$-adenosine receptors, [$^3$H]MSX-2 for $A_{2A}$-receptors and [$^3$N] PSP-11 for $A_3$-receptors. Results of this experiments are depicted in table 2 (human data) and table 3 (rat data). Radioligand binding test systems are e.g. generally described by Muller et al., Curr. Pharm Des., 1996, 2, 501 and Weyler et al., ChemMedChem 2006, 1, 891. These documents are incorporated herein by reference.

*Radioligand Binding Assay 2; Receptor Binding Profile of $A_{2A}$ Receptor Antagonists including the* $A_{2B}$ adenosine receptor:

The assays were performed under the conditions described below. The literature references are also provided for each assay:

| | |
|---|---|
| Adenosine $A_1$: | |
| Source: | Human recombinant CHO cells |
| Ligand: | 1 nM [$^3$H] CCPA |
| Incubation Time/Temp.: | 60 min/22˚C |
| Non-Specific Ligand: | CPA (10 μM) |
| Method of Detection: | Scintillation Counting |

The adenosine $A_1$ radioligand binding assay used is further described in Rivkees S.A. et al., J. Biol. Chem., 1995, 270, 20485 and is incorporated herein by reference.

| | |
|---|---|
| Adenosine $A_{2A}$: | |
| Source: | Human recombinant HEK-293 cells |
| Ligand: | 6 nM [$^3$H] CGS 21680 |
| Incubation Time/Temp.: | 120 min./22˚C |
| Non-Specific Ligand: | NECA (10 μM) |
| Method of Detection; | Scintillation Counting |

The adenosine $A_{2A}$ radioligand binding assay used is further described in Luthin D.R. et al., Mol. Pharmacol., 1995, 47 and is incorporated herein by reference.

| | |
|---|---|
| Adenosine $A_{2B}$: | |
| Source: | Human recombinant HEK-293 cells |
| Ligand: | 0.5 nM [$^3$H] MRS 1754 |
| Incubation Time/Temp.: | 120 min./22˚C |

(continued)

| | |
|---|---|
| Adenosine $A_{2B}$: | |
| Non-Specific Ligand: | NECA |
| Method of Detection: | Scintillation Counting |

The adenosine $A_{2B}$ radioligand binding assay used is further described in Stehle J.H. et al., Mol. Endocrinol., 1992, 6, 384 and is incorporated herein by reference.

| | |
|---|---|
| Adenosine $A_3$: | |
| Source: | Human recombinant HEK-293 cells |
| Ligand: | 0.15 nM [$^{125}$I] AB-MECA |
| Incubation Time/Temp.: | 120 min./22°C |
| Non-Specific Ligand: | IB-MECA (1 μM) |
| Method of Detection: | Scintillation Counting |

The adenosine $A_3$ radioligand binding assay used is further described in Salvatore C.A. et al., Proc. Natl. Acad. Sci. USA, 1993, 90, 10365 and is incorporated herein by reference.

The specific ligand binding to the receptors is defined as the difference between the total binding and the non-specific binding determined in the presence of an excess of unlabelled ligand. The results are expressed as a percent of control specific binding and as a percent inhibition of control specific binding obtained in the presence of the test compounds. Results showing an inhibition higher than 50% are considered to represent significant effects of the test compounds.

The results of the binding assays are depicted in table 4 as the % inhibition of agonist binding at 1 μmol/l (≥50 % inhibition of agonist binding is commonly regarded as a robust signal). As can be seen from table 4, all compounds bind specifically to the $A_{2A}$-receptor.

*Sodium Chloride-Shift-Experiment:*

The compounds were tested in a sodium chloride-shift experiment of whether they functionally bind A2A adenosine receptors from rat striatum. In this experiment the Ki-value is measured with and without sodium chloride present (100 mM). The presence of a relatively high concentration of sodium chloride should not influence the affinity of the antagonists. However, the affinity of A2A AR agonists should be attenuated and therefore lead to an elevated $K_i$-value. As can be seen from table 5, the absence and presence of sodium chloride does not influence the $K_i$-value of the examined compounds. This confirms the A2A-antagonistic function of the compounds. (Gao et al, Biochem. Pharmacol., 2000, 60, 669, herein incorporated by reference).

*Functional Assay Concerning Inhibition of $A_1$- and $A_{2A}$-Adenosine Receptors :*

The following experiment evaluated the compounds of the invention for their functional antagonistic activity on two cell lines expressing the human recombinant adenosine receptors $A_1$ or $A_{2A}$. CHO-DUKX cells expressing recombinant human adenosine $A_1$ or $A_{2A}$ (named CHO-DUKX-SRE-Luci-A1-44 and CHO-DUKX-CRE-A2A-19, respectively) are prepared. These cell lines originated from CHO-DUKX (DSMZ: ACC 126) cells, and have the reporter-gene plasmid pSRE-Luci or pCRE-Luci (Biofrontera Pharmaceuticals Germany) stably integrated. CHO-DUKX-SRE-Luci-A1-44 and CHO-DUKX-CRE-A2A-19 cells are cultivated in DMEM/F12-Mix (Invitrogen, San Diego, CA, #31331-028) supplemented with 10 % heat-inactivated FBS (PAA Laboratories, Germany, #A15-649), 0.2 mg/ml Hygromycin B (Invitrogen, San Diego, CA, #1113347) and 0.4 mg/ml G418 (invitrogen, San Diego, CA, #10131-019). Cells are grown in a humidified chamber at 37°C, 5% CO2. cDNA encoding the respective human receptors was cloned from human mRNA preparations by RT-PCR with sequence-specific primers covering the start and stop codons, respectively, using high-fidelity Taq polymerases (Pfu, Stratagen; Pfx, Invitrogen). cDNA inserts were directionally subcloned into the expression vector pCineo (Promega) and sequenced. The deduced amino acid sequences were in accordance with those published in GenBank. Expression plasmids were introduced into eukaryotic cells harboring the luciferase reporter-gene driven by a SRE-based (CHO-DUKX-SRE) promoter element or CRE-based (CHO-DUKX-CRE) promoter element as indicated in the name of the cell line. The investigated receptor was shown to activate the respective reporter-gene in the selected cell line. Transfections were performed in 6-well plates using the Lipofectamine Plus reagent (Invitrogen, San Diego, CA) according to the instructions of the manufacturer. Two days after transfection cells were selected for G418 resistance

(0.4 mg/ml) and grown for 10 days. Cells were seeded into 96-well plates in a limited dilution of 2 cells per well. Two weeks later single colonies were split into three wells and tested for agonist responsiveness. The clonal cell lines used for this study exhibited the most robust signals in terms of fold induction and absolute signal intensity in relative light units (RLUs) and have been pharmacologically characterized.

The luciferase reporter assay was then used to measure concentration response curves. Cells were seeded in white 96-well microtiter plates (Becton Dickinson, Heidelberg, Germany, #353296) at a density of approx. 30,000 cells per well in growth medium, supplemented with 0.2 mg/ml hygromycin (Invitrogen, San Diego, CA) and 0.4 mg/ml G418 (Invitrogen, San Diego, CA). After 24 h, the growth medium was removed, the cells washed and incubated further with 90 μl medium lacking supplements and serum. Cells were starved under these conditions for 15-20 h prior to stimulation by the antagonist. Test compounds (test stock solution: 10 mM in DMSO, stock solutions were kept frozen at minus 20 °C in aliquots until use) were added to the cells after dilution in PBS from DMSO-stocks. Test items were serially diluted from stock (with PBS (Invitrogen, San Diego, CA, #14190-094, Lot #3091940) to the 10 x final concentrations. Since the stock solutions were prepared in 100 % DMSO, the final incubation medium contained DMSO at concentrations lower than 0.3 %. DMSO concentrations below 4 % did not influence the outcome of the experiment. With a multichannel pipette, 10 μl of a dilution of the test item was added to 90 μl of medium. Test items were tested in 14 different concentrations covering, after final dilution in the well, a concentration range from 10-13 M to 3x10-5 M. After a 5-minute incubation, the cells were stimulated for 4 h at 37°C with agonist at the respective calculated $EC_{80}$ concentrations (3.5 μM ADAC for the A1, 60,nM NECA for the $A_{2A}$ receptor), obtained from the concentration-response experiments of the receptor assays (data not shown). Subsequently, the medium was removed and the cells were lysed by the addition of 20 μl lysis buffer (25 mM Tris/HCl pH 7.8, 0.4 mM DTT, 0.4 mM CDTA, 2.5 % Glycerol, 0.25 % Triton X-100) and 30 μl of luciferase assay reagent (20 mM Tricine, 1.07 mM Mg(CO3)4 x Mg(OH)2 x 5H2O, 2.67 mM MgSO4 x 7H2O, 0.1 mM EDTA, 33.3 mM DTT, 0.27 mM CoA x 2H2O, 0.47 mM D-Luciferin, 0.53 mM ATP). After mixing, the luminescence of the solution was measured integrative for 3 s in an Ascent Fluoroskan FL (Labsystems, Helsinki, Finland). $IC_{50}$ values and the maximum antagonistic effect, Imax, were calculated from the concentration response curves of the compounds. The data were compared to the concentration response curves of CPX (8-Cyclopentyl 1,3-diprophylxanthine, Sigma, C-101), an $A_1$ receptor antagonist, and 5-Amino-7-(β-phenylethyl)-2-(8-furyl)pyrazolo(4,3-e)-1,2,4-triazolo(1,5-c)pyrimidine (Sigma, S-4568), an $A_{2A}$ receptor antagonist. Receptors were stimulated with 3.5 μM ADAC (adenosine amine congener, Sigma, A-111) for the $A_1$ and 60 nM N ECA (5-(N-ethylcarboxamido)-adenosine, Sigma, E-2387) for the $A_{2A}$ receptor.

The background signal (background = mean of diluent values; PBS was used as diluent) was subtracted from each data point. Curve fitting was performed to determine $IC_{50}$, $I_{max}$ and p values using ORIGIN (Microcal Software, Northhampton, MA, U.S.A.). The following model was applied:

$$y = \frac{[A1 - A2]}{1 + (x/x_0)^p} + A2$$

This model represents a four parametric logistic equation for the description of antagonist action. The parameters are initial value (A1), final value (A2), X at $Y_{50}$ ($X_0$), and power (p). To compare $I_{max}$ values of the test items with the $I_{max}$ of the corresponding antagonist $I_{max}$ % values were calculated as $I_{max}$ in % of the corresponding antagonist. $K_i$ values were calculated using the equation: $K_i = IC_{50}/(1+\text{used concentration of agonist}/EC_{50})$. The results are depicted in table 6. The compounds proof to be full antagonists at $A_{2A}$ adenosine receptors.

*Modified Irwin Test:*

In this study the compounds of the present invention were screened for basal behavioural, autonomic, neurological and toxicological side-effects in rats. For this purpose, a functional observational battery (FOB) in form of an Irwin test (Irwin S., Psychopharmacologia, 1968, 13, 222; 1968; Warburton D.M. Psychopharmacology, 2002, 163, 4 ; Haggerty G. C. et al., J. Amer. Coll Toxicol., 1991, 10, 677; Mattsson J.L. et al., J., Amer. Coll. Toxicol., 1996, 15, 239, all references are herein incorporated by reference) modified and validated by the Test Facility was used. Each test compound was tested at eight doses (0.01, 0.03, 0.1, 0.3, 1.0, 3.0, 10.0 and 30.0 mg/kg). All compounds were dissolved in DMSO (administration volume 1 ml/kg). Each dose group included three animals. Two vehicle groups (DMSO and Labrasol) served as negative controls. At the first day of each test week all test compound solutions necessary for the test (1-2 weeks) were prepared by means of one dilution series starting with the highest concentration. All test compounds dissolved in DMSO were administered in a volume of 1 ml/kg. DMSO-containing solutions were stored at -18°C. All

solutions were melted for administration at room temperature 16-20 hours before the start of the test.

Study design and time schedule:

| | | |
|---|---|---|
| 4-7 days: | | Acclimatization and handling of the animals |
| 1 day: | | Test day with |
| | (a) | Four repeated functional observational battery (FOB): 30 min, 60 min, 120 min, 180 min following compound administration |
| | (b) | Short animal check (SAC): 300 min following compound administration |
| 1 day: | | Follow-up observation (FU): 24h (1440 min) following compound administration. |

In all experimental parts, an experienced ethologist used lists of predefined methods and behavioural parameters in order to rate the behaviour of the animal (see table 7 for functional observational battery (FOB), table 8 for short animal check (SAC) and table 9 for follow-up observation (FU).

A statistical evaluation of the data was performed that enabled an assessment of the compound's effects on each parameter. Dose-response relationships are not necessarily linear. It was not clear a priori whether the response would be dose-dependent or whether low, medium or high dose effects would occur. Therefore, the data were correlated against a set of different numeric models of theoretical dose-response curves. For this kind of evaluation the measurement times were not differentiated. An average value over all measurement times was calculated for each animal. This value was taken as one measurement within the observation vector. The complete set of parameters measured for the animal (each one averaged over time) formed the observation vector. Each dose was then represented by three observation vectors (3 animals per dose). The effects of all doses were compared with vehicle (DMSO) as the observation vectors for the zero dose. A common pool of 24 animals for the zero dose was used for all compounds to be tested. For each test compound the obtained values for each parameter together with values from the pool of vehicle-treated (DMSO) animals were then correlated with theoretical dose-response models (analysis profiles). Since 8 doses were be used, there were 8 models, each assuming that the maximum compound effect occurred at one of these doses. Effects of other doses were assumed to be attenuated with a linearly descending slope of the theoretical curve. Correlation values and statistical significance of regression were then calculated. An error probability of $p<0.05$ (two-tailed testing) was taken as significance level. Non-significant regressions were neglected. In consequence, each parameter was assessed as not affected or affected by the compound in a dose-response relationship according to type 1 (low dose effect) up to type 8 (high dose effect). Dose response is maximal for the effect with the greatest absolute correlation coefficient. The results were then summarized and visualized in a result matrix for each test compound. The outcome of this study is depicted in compressed form in table 10 (0.01 - 30 mg/kg). In summary, the compounds of the present invention induce a potent hyperlocomotion.

*Acute CGS-21680 Induced Catalepsy and Acute Reserpine Induced Catalepsy Tests:*

Animals were administered with Reserpine or CGS-21680 according to methods known to the skilled person in the art. These models and procedures are e.g. described by Ferré S. et al., Neurosci. Let., 1991, 130, 162; Ferré S. et al., Neuroscience, 1992, 51, 501; Kafka S.H. et al., Eur. J. Pharmacol., 1996, 295, 147; Rimondini R. et al., Neuropsychopharma cology, 1997, 17, 82 and are herein incorporated by reference. In the acute CGS-21680 induced catalepsy model (CGS model) the test compounds (TC, compounds according to the present invention) and CGS-21880 (i.p. 2 mg/kg) were administered 60 minutes before the start of the catalepsy bar measurement and behavioural testing described below (see also figure 1). In the acute Reserpine induced catalepsy model (RES model resperine (subcutaneously, 3 mg/kg) was injected 24h prior to the test compound application. catalepsy bar measurement took place 60 minutes after test compound application (see figure 1). Test compounds were administered i.p. in DMSO in following doses (mg/kg) : 0.1, 0.3, 1, 3, 10.

Dimethylsulfoxide (DMSO) was used as vehicle for the test compounds and Reserpine. Reserpine was dissolved in DMSO in a concentration of 3 mg/ml. A 10% (w/v) Cyclodextrin solution was used as vehicle for CGS-21680. CGS 21680 was dissolved in 10% (w/v) Cyclodextrine in a concentration of 2 mg/ml.

Caffeine was used as a reference compound in the CGS-21680 induced catalepsy model. A caffeine solution was prepared with water for injection purposes to a concentration of 30 mg/ml. The solution was administered intraperitoneally in a volume of 1 ml/kg (30 mg/kg) 30 minutes before the beginning of testing and after CGS-21680 pre-treatment (1 hour prior to testing).

Apomorphine was used as a reference compound in the Reserpine induced catalepsy model. Apomorphine

injection solution was diluted with purified water to a concentration of 0.3 mg/ml. The solution was administered subcutaneously (s.c.) in a volume of 1 ml/kg (0.3 mg/kg) 20 minutes before the begin of testing after Reserpine pre-treatment (25 hours prior to testing).

The catalepsy bar after CGS-21680 administration was measured (CAT) as follows: The rat was placed with its forepaws on a log of wood. If it did not descend within 30 seconds, the trial ended and 30 seconds was taken as the time for that trial. If the animal descended faster than 1 second, the trial was regarded as invalid. If more than 20 trials were rated as invalid, the values of all uncompleted trials were noted as 0. The animal was tested until five trials had been completed and the time for each trial was noted. Only the values of the last three trials were used for the analysis. The following parameters were calculated: maximum time to descend (s) and median time to descend (s).

The catalepsy bar after Reserpine administration was measured (CAT) as follows: The rat was placed with its forepaws on a log of wood. If it did not descend within 30 seconds, the trial ended and 30 seconds was taken as the time for that trial. The animal was tested for three trials and the time for each trial was noted. The following parameters were calculated: maximum time to descend (s) and median time to descend (s).

Behavioural observation (BO) included the measurement of the body tone, inclined plane and grip strength.

Body tone:

The rat is taken in the hand of the observer and the animal's body tone is rated as normal, soft or hard. The rating is performed immediately after taking the animal from its home cage before performance of catalepsy testing. The following parameters were calculated: mean occurrence of hard body tone and mean occurrence of soft body tone.

Inclined plane:

The rat is horizontally placed to an inclined plane for a maximum time of 30 seconds. Time to reach bottom or top is measured. If the animal does not reach the bottom or top of the grid after 30 seconds inclined plane testing is stopped and 30 seconds are taken as result. The test is performed after catalepsy testing. Time to leave the inclined plane (s) was measured.

Grip strength:

The rat is put on a grip and the observer pulls the animal back on his tail. The strength (fore paws and hind paws) by which it holds on the grid is scored as grasping and pulling, grasping without pulling or no grasping. The test is performed after the inclined plane test. The following parameters were calculated: mean score of fore paw grip strength and mean score of hind paw grip strength.

All statistical tests were performed two tailed with a value of $p<0.05$ considered as significant.

To assess the effects of CGS-21680 and Reserpine pre-treatment compared to vehicle treated controls statistical comparisons between the test groups were performed using a U test statistic.

To assess the effects of test compounds on CGS-21680 and Reserpine pre-treated animals correlation analyses including animals from test groups and the respective test compound-treated groups were performed.

Additionally, parametric and non-parametric comparisons for the median catalepsy measure were performed to assess the reliability of the method. In case of parametric testing a GLM analyses was performed including animals from a control group (only vehicle treated animals) and the respective test compound-treated groups with dose as a categorial factor. In case of a significant result, a Dunnett's post hoc test was performed between the according test compound groups and a control group as reference group. In case of non-parametric testing a Kruskal-Wallis H-test was performed including animals from a control group and the respective test compound groups. In case of a significant result single U-test comparisons were performed between a control group and the groups treated with different doses of the test compound.

Figure 2 shows the CGS-21680 induced catalepsy time with control (vehicle/vehicle) and reference compound caffeine (mean $\pm$ SEM. N = 24). CGS-21680 compared to vehicle significantly increased rating of soft and decreased rating of hard body tone. Time leaving inclined plane and grip strength were unaffected. Compared to only CGS-21680-treated animals caffeine increased rating of hard and decreased rating of soft body tone almost to the level of only vehicle-treated animals. Caffeine significantly decreased median and maximum catalepsy values in CGS-21680 pre-treated animals to a level that was even lower than that of only vehicle-treated animals.

Figure 3 shows the Reserpine induced catalepsy time with control (vehicle/vehicle) and reference compound apomorphine (mean $\pm$ SEM. N = 24). Reserpine compared to vehicle significantly increased median and maximum catalepsy values. Compared to only Reserpine-treated animals apomorphine decreased median and maximum catalepsy values to a level that was still higher than that of only vehicle-treated animals.

Figure 4a and figure 4b show the significant reduction of catalepsy time in acute CGS-21680 induced catalepsy

model with test compound 1 (=3-Ethyl-8-(3,4-dimethoxyphenylethynyl)-7-methyl-1-prop-2-ynyl-3,7-dihydropurine-2,6 dione). Figure 5a and figure 5b show the significant reduction of catalepsy time in acute Reserpine induced catalepsy model with test compound 1 (=3-Ethyl-8-(3,4-dimethoxyphenylethynyl)-7-methyl-1-prop-2-ynyl-3,7-dihydropurine-2,6 dione).

Test compound 1 significantly increased hard body tone and decreased soft body tone, median and maximum catalepsy values after CGS-21680 treatment. Additionally test compound 1 led to a higher amount of grip strength. The effects were dose-dependent with a dose of 1 mg/kg and 10 mg/kg having the largest effects. Test compound 1 significantly reduced median catalepsy values after Reserpine treatment. The effects were dose-dependent with a dose of 1 mg/kg and 10 mg/kg having the strongest effects. Maximum catalepsy values were only weakly reduced by a dose of 1 mg/kg.

Figure 6a and figure 6b show the reduction of catalepsy time in acute CGS-21680 induced catalepsy model with test compound 2 (=3-(3-Hydroxypropyl)-8-(3,4-dimethoxyphenylethynyl)-7-methyl-1-prop-2-ynyl-3,7-dihydropurine-2,6-dione.

Figure 7a and figure 7b show the significant reduction of catalepsy time in acute Reserpine induced catalepsy model with test compound 2 (=3-(3-Hydroxypropyl)-8-(3,4-dimethoxyphenylethynyl)-7-methyl-1-prop-2-ynyl-3,7-dihydropurine-2,6-dione.

Test compound 2 significantly increased hard body tone (strongest effect at 0.1 mg/kg) and decreased soft body tone (strongest effect at 10 mg/kg) after CGS-21680 treatment. There were no significant effects on any other parameter measured after CGS-21680 treatment. Test compound 2 significantly decreased medium and maximum catalepsy values after Reserpine treatment. The effects were dose dependent with the highest dose having the strongest effects. Mean $\pm$ standard error of the mean (SEM) is indicated in figures 2, 3, 4b, 5b, 6b and 7b.

**Table 1:**

| $R^1$ | $R^2$ | $R^3$ | $R^4$ | Name | MW/ m.p.,/NMR |
|---|---|---|---|---|---|
| 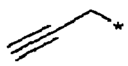 | $-CH_3$ | $-CH_3$ | 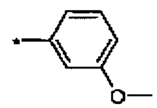 | 8-(3-Methoxyphenylethyn yl)-3,7-dimethyl-1-prop-2-ynyl-3,7-dihydropurine-2,6-dione | MW 348.4, colorless crystals, m.p. 234.7 °C. **$^1$H NMR** (500 MHz, $CDCl_3$): δ = 2.17 (t, J = 2.52 Hz, 1H, CCH, 1H), 3.59 (s, 3H, $OCH_3$), 3.81 (s, 3H, $N3CH_3$), 4.07 (s, 3H, $N7CH_3$), 4.79 (d, J = 2.52 Hz, 2H, $\underline{CH_2}$-CCH, 2H), 6.98 (ddd, J = 1.26/2.52 and 8.43 Hz, 1H, 5'H), 7.16 (dd, J = 1.26 and 2.52 Hz, 1 H, 2'H), 7.19 (dt, J = 1.26 and 7.56 Hz, 1H, 6'H), 7.29 (dd, J = 7.57 and 8.36 Hz, 1 H, 4'H) ppm. **$^{13}$C NMR** (125 MHz, $CDCl_3$): δ = 29.9, 30.6, 33.3, 55.4 ($OCH_3$), 70.6, 76.5, 78.5, 97.4, 107.8, 116.8, 116.9, 121.2, 124.6, 129.8, 136.1, 148.2, 150.7, 153.8,159.5 ppm. |
| 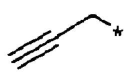 | $-CH_3$ | $-CH_3$ | 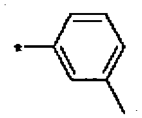 | 3,7-Dimethyl-8-(3-methylphenylethynyl)-1-prop-2-ynyl-3,7-dihydropurine-2,6-dione | MW 332.4, colorless crystals: **m.p.** 218.7 °C; $^1$H **NMR ($CDCl_3$);** 2.17 (t, J = 2.6 Hz, 1H), 2.36 (s, 3H), 3.59 (s, 3H), 4.07 (s, 3H), 4.78 (d, J = 2.6 Hz, 2H), 7.23-7.29 (m, 2H), 7.39-7.42 (m, 2H) ppm. **$^{13}$C NMR ($CDCl_3$):** 21.2, 29.9, 30.5, 33.2, 70.5, 76.5, 78.6, 97.8, 107.8, 120.1, 128.6, 129.3, 131.2, 132.6, 136.3, 138.5, 148.3, 150.7, 153.7 ppm. |
| 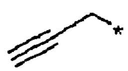 | $CH_3$ | $-CH_3$ | 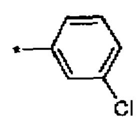 | 8-(3-Chlorophenylethynyl) -3,7-dimethyl-9-prop-2-ynyl-3,7-dihydropurine-2,6-dione | MW 352.8, colorless crystals: **m.p.** 216.6 °C; **$^1$H NMR ($CDCl_3$):** 2.17 (t, J = 2.3 Hz, 1H), 3.59 (s, 3H), 4.07 (s, 3H), 4.78 (d, J = 2.3 Hz, 2H), 7.31-7.35 (m, 1H), 7.40-7.43 (m, 1H), 7.46-7.49 (m, 1H), 7.57-7.58 (m, 1H) ppm. **$^{13}$C NMR ($CDCl_3$):** 29.9, 30.6, 33.3, 70.6, 77.7, 78.5, 95.6, 108.0, 122.0, 130.0, 130.2, 130.5, 131.9, 134.6, 135.6, 148.2, 150.6, 153.8 ppm. |

(continued)

| R¹ | R² | R³ | R⁴ | Name | MW/ m.p.,/NMR |
|---|---|---|---|---|---|
| 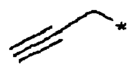 | $-CH_3$ | $-CH_3$ | 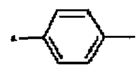 | 3,7-Dimethyl-8-(4-methylphenylethynyl) -1-prop-2-ynyl-3,7-dihydropurine-2,6-dione | MW 332.4, colorless crystals, m.p. 224.3 ˚C. **$^{1}$H NMR ($CDCl_3$):** 2.17 (t, J = 2.5 Hz, 1H), 2.38 (s, 3H), 3.59 (s, 3H), 4.06 (s, 3H), 4.78 (d, J = 2.5 Hz, 2H), 7.18-7.20 (m, 2H), 7.47-7.49 (m, 2H) ppm. **$^{13}$C NMR ($CDCl_3$):** 21.7, 29.9, 30.5, 33.2, 70.5, 76.3, 78.6, 97.9, 107.7, 117.2, 129.5, 132.1, 136.4, 140.9, 148.3, 150.7, 153.8 ppm. |
| 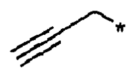 | $-CH_3$ | $-CH_3$ | 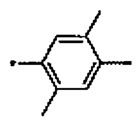 | 3,7-Dimethyl-8-(2,4,5-trimethylphenylethyn yl)-1-prop-2-ynyl-3,7-dihydropurine-2,6-dione | MW 360.4, colorless crystals, m.p. 262.2 ˚C. **$^{1}$H NMR ($CDCl_3$):** 2.17 (t, J = 2.2 Hz, 1 H), 2.21 (s, 3H), 2.24 (s, 3H), 2.44 (s, 3H), 3.59 (s, 3H), 4.06 (s, 3H), 4.78 (d, J = 2.2 Hz, 2H), 7.02 (s, 1 H), 7.33 (s, $^{1}$H) ppm. **$^{13}$C NMR ($CDCl_3$):** 19.1, 19.9, 20.2, 29.9, 30.5, 33.2, 70.5, 78.6, 79.7, 97.3, 107.7, 117.3, 131.2, 133.5, 134.3, 136.7, 138.3, 139.6, 148.3, 150.7, 153.7 ppm. |
| 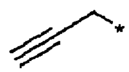 | $-CH_3$ | $-CH_3$ | 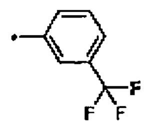 | 8-(3-Trifluoromethylphenyl ethynyl)-3,7-dimethyl-1-prop-2-ynyl-3,7-dihydropurine-2,6-dione | MW 386.3, colorless crystals: m.p.: 229.3 ˚C; **$^{1}$H NMR ($CDCl_3$):** 2.17 (t, J = 2.5 Hz, 1 H), 3.59 (s, 3H), 4.09 (s, 3H), 4.78 (d, J = 2.5 Hz, 2H), 7.52-7.56 (m, 1 H), 7.67-7.70 (m, 1 H), 7.76-7.78 (m, 1 H), 7.85 (s br, 1H) ppm. $^{13}$C **NMR ($CDCl_3$):** 29.9, 30.6, 33.4, 70.6, 78.1, 78.5, 95.4, 108.0, 121.4, 122.3, 124.4, 126.7, 126.75, 126.78, 128.86, 128.89, 128.92, 128.95, 129.4, 131.3, 131.6, 135.5, 148.2, 150.6, 153.8 ppm. |
| 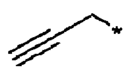 | $-CH_3$ | $-CH_3$ | 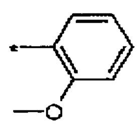 | 3-Methyl-8-(2-methoxyphenylethyn yl)-1-prop-2-ynyl-3,7-dihydropurine-2,6-dione | MW 348.4, colorless crystals, **m.p.** 282.3 ˚C. **$^{1}$H NMR ($CDCl_3$):** 2.17 (s, 1H), 3.59 (s, 3H), 3.91 (s, 3H), 4.09 (s, 3H), 4.78 (s, 2H), 6.91-6.6.97 (m, 3H), 7.38-7.41 (m, 1H), 7.52-7.54 (m, 1H) ppm. **$^{13}$C NMR ($CDCl_3$):** 29.9, 30.5, 33.2, 55.8, 70.5, 78.6, 80.9, 94.6, 107.8, 109.7, 110.7, 120.7, 131.8, 133.7, 136.7, 148.3, 150.7, 153.8, 160.9 ppm. |

(continued)

| $R^1$ | $R^2$ | $R^3$ | $R^4$ | Name | MW/ m.p.,/NMR |
|---|---|---|---|---|---|
| 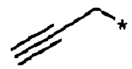 | $-CH_3$ | $-CH_3$ | 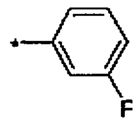 | 8-(3-Fluorophenylethynyl)-3,7-dimethyl-1-prop-2-ynyl-3,7-dihydropurine-2,6-dione | MW 336.3, colorless crystals: **m.p.** 220.6 °C. **$^1$H NMR ($CDCl_3$):** 2.17 (t, J = 2.2 Hz, 1H), 3.59 (s, 3H), 4.07 (s, 3H), 4.78 (d, J = 2.2 Hz, 2H), 7.13-7.17 (m, 1 H), 7.27-7.30 (m, 1 H), 7.34-7.39 (m, 2H) ppm. **$^{13}$C NMR ($CDCl_3$):** 29.9, 30.6, 33.3, 70.6, 77.5, 78.5, 95.8 (d), 108.0, 117.7 (d), 118.8 (d), 122.1 (d), 128.0 (d), 130.4 (d), 135.7, 148.2, 150.6, 153.8, 162.3 (d) ppm. |
| 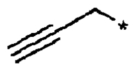 | $-CH_3$ | $-CH_3$ | 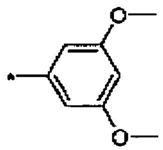 | 8-(3,5-Dimethoxyphenylethy nyl)-3,7-dimethyl-1-prop-2-ynyl-3,7-dihydropurine-2,6-dione | MW 378.4, colorless crystals, m.p. 228 °C. **$^1$H NMR ($CDCl_3$):** 2.17 (t, J = 2.5 Hz, 1H), 3.59 (s, 3H), 3.79 (s, 6H), 4.07 (s, 3H), 4.78 (d, J = 2.5 Hz, 2H), 6.53 (t, J = 2.2 Hz, 1 H), 6.72 (d, J = 2.2, 2H) ppm. **$^{13}$C NMR ($CDCl_3$):** 29.9, 30.6, 33.3, 55.4, 70.6, 76.2, 78.5, 97.5, 103.6, 107.8, 109.9, 121.5, 136.1, 148.2, 150.7, 153.8, 160.7 ppm. |
| 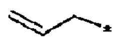 | $-CH_3$ | $-CH_3$ | 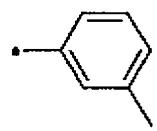 | 1-Alkyl-3,7-dimethyl-8-(3-methylphenylethynyl) -dihydropurine-2,6-dione | MW 334.4, colorless crystals, **m.p.** 157.8 °C. **$^1$H NMR ($CDCl_3$):** 2.35 (s, 3H, $CH_3$), 3.57 (s, 3H, $CH_3$), 4.06 (s, 3H, $NCH_3$), 4.62 (m, 2H), 5.22 (m, 2H), 5.91 (m, 1 H), 7.23-7.29 (m, 2H), 7.39-7.41 (m, 2H) ppm. **$^{13}$C NMR ($CDCl_3$):** 21.2, 29.8, 33.2, 43.4, 76.6, 97.6, 107.8, 117.7, 120.2, 128.6, 129.2, 131.1, 132.1, 132.6, 136.0, 138.5, 148.0, 151.1, 154.4 ppm. |
| 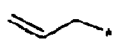 | $-CH_3$ | $-CH_3$ | 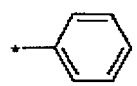 | 1-Allyl-3,7-dimethyl-8-phenylethynyl-dihydropurine-2,6-dione | MW 320.4, colorless crystals, **m.p.** 193.2 °C. **$^1$H NMR ($CDCl_3$) :** 3.57 (s, 3H, $CH_3$), 4.07 (s, 3H, $NCH_3$), 4.62 (m, 2H), 5.22 (m, 2H), 5.91 (m, 1 H), 7.36-7.45 (m, 3H), 7.58-7.60 (m, 2H) ppm. **$^{13}$C NMR ($CDCl_3$) :** 29.8, 33.2, 43.4, 76.9, 97.3, 107.9, 117.7, 120.24, 128.7, 130.2, 132.1, 132.2, 135.9, 148.0, 151.1, 154.4 ppm. |

(continued)

| $R^1$ | $R^2$ | $R^3$ | $R^4$ | Name | MW/ m.p.,/NMR |
|---|---|---|---|---|---|
| 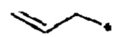 | $-CH_3$ | $-CH_3$ | 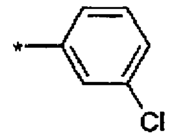 | 1-Allyl-8-(3-chlorophenylethynyl)-3,7-dimethyl-dihydropurine-2,6-dione | MW 354.8, colorless crystals, m.p. 193.0 °C. **$^1$H NMR ($CDCl_3$):** 3.57 (s, 3H, $CH_3$), 4.07 (s, 3H, $NCH_3$), 4.62 (m, 2H), 5.23 (m, 2H), 5.91 (m, 1H), 7.31-7.34 (m, 1 H), 7.40-7.42 (m, 1H), 7.47-7.49 (m, 1H), 7.57-7.58 (m, 1H) ppm. **$^{13}$C NMR ($CDCl_3$):** 29.8, 33.3, 43.4, 77.8, 95.5, 108.1, 117.8, 122.1, 129.9, 130.2, 130.5, 131.8, 132.1, 134.6, 135.4, 148.0, 151.1, 154.4 ppm. |
| 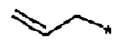 | $-CH_3$ | $-CH_3$ | 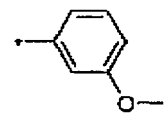 | 1-Allyl-8-(3-methoxyphenylethyn yl)-3,7-dimethyl-3,7-dihydropurine-2,6-dione | MW 350.4, colorless crystals, m.p. 172.3 °C. **$^1$H NMR ($CDCl_3$):** 3.58 (s, 3H, $NCH_3$), 3.81 (s, 3H, $OCH_3$), 4.07 (s, 3H, $NCH_3$), 4.62 (m, 2H), 5.23 (m, 2H), 5.91 (m, 1H), 6.96-6.99 (m, 1 H), 7.10-7.1 (m, 1H), 7.17-7.20 (m, 1H), 7.27-7.30 (m, 1H) ppm. **$^{13}$C NMR ($CDCl_3$):** 29.8, 33.2, 43.4, 55.4, 76.6, 97.2, 107.9, 116.7, 116.8, 117.7, 121.3, 124.6, 129.8, 132.1, 135.8, 148.0, 159.1, 154.4, 159.4 ppm. |
| 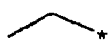 | $-CH_3$ | $-CH_3$ | 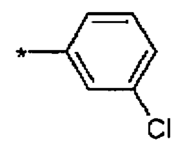 | 8-(3-Chlorophenylethynyl) -1-ethyl-3,7-dimethyl-3,7-dihydropurine-2,6-dione | MW 342.8, colorless crystals, m.p. 221.5 °C. **$^1$H NMR ($CDCl_3$):** 1.24 (t, J = 6.95 Hz, 3H, $CH_3$), 3.56 (s, 3H, $NCH_3$), 4.07 (q, J = 6.95 Hz, 2H), 4.07 (s, 3H, $NCH_3$), 7.31-7.34 (m, 1H), 7.40-7.41 (m, 1H), 7.47-7.48 (m, 1H), 7.57-7.58 (m, 1H) ppm. **$^{13}$C NMR ($CDCl_3$):** 13.3, 29.7, 33.2, 36.7, 77.9, 95.3, 108.2, 122.2, 129.9, 130.2, 130.4, 131.8, 134.6, 135.2, 147.9, 151.1, 154.6 ppm. |
| 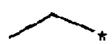 | $-CH_3$ | $-CH_3$ | 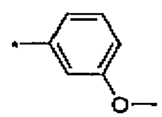 | 1-Ethyl-8-(3-methoxyphenylethyn yl)-3,7-dimethyl-3,7-dihydropurine-2,6-dione | MW 338.4, colorless crystals, m.p. 198.6 °C. **$^1$H NMR ($CDCl_3$):** 1.24 (t, J = 6.9 Hz, 3H, $CH_3$), 3.56 (s, 3H, $NCH_3$), 3.81 (s, 3H, $OCH_3$), 4.07 (s, 3H, $NCH_3$), 4.07 (q, J = 7.25 Hz, 2H, $CH_2$), 6.96-6.98 (m, 1 H), 7.09-7.11 (m, 1H), 7.17-7.19 (m, 1H), 7.27-7.30 (m, 1H) ppm. **$^{13}$C NMR ($CDCl_3$):** 13.3, 29.7, 33.6, 36.6, 55.4, 76.7, 97.1, 108.1, 116.7, 116.8, 121.4, 124.6, 129.8, 135.7, 147.9, 159.1, 154.6, 159.4 ppm. |

(continued)

| R¹ | R² | R³ | R⁴ | Name | MW/ m.p.,/NMR |
|---|---|---|---|---|---|
| 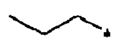 | $-CH_3$ | $-CH_3$ | 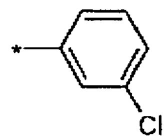 | 8-(3-Chlorophenylethynyl) -3,7-dimethyl-1-propyl-3,7-dihydropurine-2,6-dione | MW 356.8, colorless crystals, m.p. 183.2 °C. **$^1$H NMR ($CDCl_3$):** 0.95 (t, J = 7.25 Hz, 3H, $CH_3$), 1.65-1.69 (m, 2H, $CH_2$), 3.56 (s, 3H, $NCH_3$), 3.94-3.97 (m, 2H, $CH_2$), 4.07 (s, 3H, $NCH_3$), 7.31-7.34 (m, 1H), 7.39-7.42 (m, 1 H), 7.47-7.48 (m, 1 H), 7.57-7.58 (m, 1H) ppm. **$^{13}$C NMR ($CDCl_3$):** 11.3, 21.3, 29.7, 33.2, 43.1, 78.0, 95.4, 108.2, 122.2, 129.9, 130.2, 130.4, 131.8, 134.6, 135.2, 147.9, 151.3, 154.8 ppm. |
| 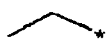 | $-CH_3$ | $-CH_3$ | 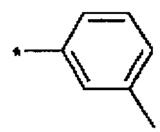 | 1-Ethyl-3,7-dimethyl-8-(3-methylphenylethynyl) -3,7-dihydropurine-2,6-dione | MW 322.4, colorless crystals, m.p. 166.1 °C. **$^1$H NMR ($CDCl_3$):** 1.24 (t, J = 6.95 Hz, 3H, $CH_3$), 2.36 (s, 3H, $CH_3$), 3.57 (s, 3H, $NCH_3$), 4.07 (s, 3H, $NCH_3$), 4.07 (q, J = 6.95 Hz, 2H, $CH_2$), 7.23-7.28 (m, 2H) 7.3-7.41 (m,2H) ppm. **$^{13}$C NMR($CDCl_3$):** 13.3, 21.2, 29.7, 33.2, 36.6, 76.7, 97.5, 108.0, 120.3, 128.6, 129.2, 131.1, 132.6, 135.8, 138.5, 147.9, 151.2, 154.6 ppm. |
| 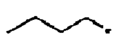 | $-CH_3$ | $-CH_3$ | 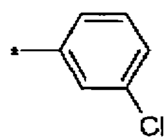 | 1-Butyl-8-(3-chlorophenylethynyl)-3,7-dimethyl-dihydropurine-2,6-dione | MW 370. 84, colorless crystals, m.p. 156.8 °C. **$^1$H NMR ($CDCl_3$):** 0.94 (t, J = 7.2 Hz, 3H, $CH_3$), 1.28 (m, 2H, $CH_2$), 1.61 (m, 2H, $CH_2$), 3.56 (s, 3H, $NCH_3$), 3.99 (t, J = 7.9 Hz, 2H, $CH_2$), 4.07 (s, 3H, $NCH_3$), 7.31-7.34 (m, 1H), 7.40-7.42 (m, 1 H), 7.47-7.48 (m, 1 H), 7.57 (s, 1H) ppm. **$^{13}$C NMR ($CDCl_3$):** 13.7, 20.2, 29.7, 30.1, 33.2, 41.4, 78.0, 95.4, 108.2, 122.2, 129.9, 130.2, 130.4, 131.8, 134.6, 135.2, 147.9, 151.3, 154.8 ppm. |
| 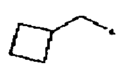 | $-CH_3$ | $-CH_3$ | 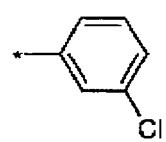 | 1-Cyclobutylmethyl-8-(3-chlorophenylethynyl)-3,7-dimethyl-dihydropurine-2,6-dione | MW 382.9, colorless crystals, **m.p.** 162.5 °C. **$^1$H NMR ($CDCl_3$):** 1.83 (m, 4H), 1.99 (m, 2H), 2.73 (m, 1 H), 3.56 (s, 3H, $NCH_3$), 4.06 (d, J = 7.55 Hz), 4.07 (s, 3H, $NCH_3$), 7.31-7.34 (m, 1H), 7.39-7.42 (m, 1 H), 7.47-7.49 (m, 1H), 7.57-7.58 (m, 1 H) ppm. **$^{13}$C NMR ($CDCl_3$) :** 18.3, 26.3, 29.8, 33.2, 34.5, 46.1, 78.0, 95.3, 108.1, 122.1, 129.9, 130,2, 130.4, 131.8, 134.6, 135.2, 147.9, 151.5, 155.0 ppm. |

(continued)

| $R^1$ | $R^2$ | $R^3$ | $R^4$ | Name | MW/ m.p.,/NMR |
|---|---|---|---|---|---|
| 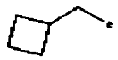 | $-CH_3$ | $-CH_3$ | 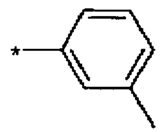 | 1-Cyclobutylmethyl-3,7-dimethyl-8-(3-methylphanylethynyl) -dihydropurine-2,6-dione | MW 362.4, colorless crystals, m.p. 129.8 °C. **$^1$H NMR ($CDCl_3$):** 1.83 (m, 4H), 1.99 (m, 2H), 2.35 (s, 3H, $CH_3$), 2.73 (m, 1H), 3.56 (s, 3H, $NCH_3$), 4.06 (d, J = 7.25 Hz), 4.06 (s, 3H, $NCH_3$), 7.23-7.28 (m, 2H), 7.39-41 (m, 2H) ppm. **$^{13}$C NMR ($CDCl_3$):** 18.3, 21.2, 26.3, 29.8, 33.2, 34.5, 46.1, 76.7, 97.5, 107.9, 120.3, 128.6, 129.2, 131.1, 132.6, 135.9, 138.5, 147.9, 151.6, 155.0 ppm. |
| 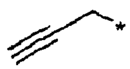 | $-CH_3$- | $-CH_3$ | 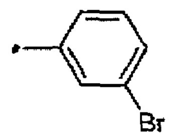 | 8-(3-Bromophenylethynyl) -3,7-dimethyl-1-prop-2-ynyl-3,7-dihydropurine-2,6-dione | MW 397.2, colorless crystals: m.p. 211.2 °C; **$^1$H NMR ($CDCl_3$):** 2.17 (t, J = 2.2 Hz, 1H), 3.59 (s, 3H), 4.07 (s, 3H), 4.78 (d, J = 2.2 Hz, 2H), 7.25-7.28 (m, 1H), 7.51-7.54 (m, 1 H), 7.56-7.58 (m, 1 H), 7.73-7.74 (m, 1 H) ppm. **$^{13}$C NMR ($CDCl_3$)**: 29.9, 30.6, 33.3, 70.6, 77.9, 78.5, 95.5, 108.0, 122.3, 122.5, 130.1, 130.6, 133.4, 134.7, 135.6, 148.2, 150.6, 153.8 ppm. |
| 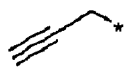 | $-CH_3$ | $-CH_3$ | 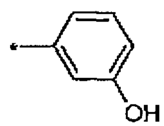 | 8-(3-Hydroxyphenylethyny l)-3,7-dimethyl-1-prop-2-ynyl-3,7-dihydropurine-2,6-dione | MW 334.3, colorless crystals, m.p. 271 °C; $^1$H NMR (DMSO-$d_6$): 3.08 (t, J = 2.2 Hz, 1 H), 3.42 (s, 3H), 3.98 (s, 3H), 4.60 (d, J = 2.2 Hz, 2H), 6.91-6.94 (m, 1H), 7.02-7.03 (m, 1H), 7.10-7.12 (m, 1H), 7.26-7.29 (m, 1H), 9.85 (s, 1H, OH) ppm. **$^{13}$C NMR (DMSO-$d_6$):** 29.6, 30.2, 33.2, 73.0, 76.9, 79.5, 96.6, 107.5, 118.2, 120.7, 122.9, 130.1, 130.4, 135.2,147.7,150.2,153.2,157.6 ppm. |
| 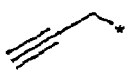 | $-CH_3$ | $-CH_3$ | -H | 3,7-Dimethyl-1-prop-2-ynyl-8-prop-1-ynyl-3,7-dihydropurine-2,6-dione | MW 256.3, colorless crystals, **m.p.** 251.5 **$^1$H NMR ($CDCl_3$):** 2.15 (t, J = 2.2 Hz, 1H), 2.16 (s, 3H, $CH_3$), 3.54 (s, 3H, $NCH_3$), 3.96 (s, 3H, $NCH_3$), 4.75 (d, J = 2.2 Hz, 2H) ppm. **$^{13}$C NMR ($CDCl_3$):** 4.6, 29.8, 30.5, 33.0, 68.1, 70.4, 78.6, 95.8, 107.2, 136.5, 148.0, 150.7, 153.7 ppm. |

(continued)

| $R^1$ | $R^2$ | $R^3$ | $R^4$ | Name | MW/ m.p.,/NMR |
|---|---|---|---|---|---|
| 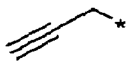 | $-CH_3$ | $-CH_3$ | 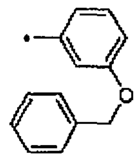 | 8-(3-Benzyloxyphenylethy nyl)-3,7-dimethy)-1-prop-2-ynyl-3,7-dihydropurine-2,6-dione | MW 424.5, colorless crystals (yield > 95 %), **m.p.** 197.5 °C. **$^1$H NMR ($CDCl_3$):** 2.17 (t, J =2.6 Hz, 1H), 3.59 (s, 3H), 4.07 (s, 3H), 4.78 (d, J =2.6 Hz, 2H), 5.07 (s, 2H), 7.04-7.42 (m, 9H) ppm. **$^{13}$C NMR ($CDCl_3$):** 29.9, 30.6, 33.3, 70.2 ($CH_2OPh$), 70.6, 76.6, 78.5, 97.3, 107.8, 117.7, 117.9, 121.3, 124.9, 127.4, 128.2, 128.7, 129.8, 136.1, 136.3, 148.2, 150.7, 153.8, 158.6 ppm. |
| 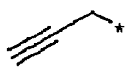 | $-CH_3$ | $-CH_3$ | 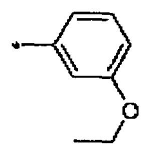 | 8-(3-Ethoxyphenylethynyl) -3,7-dimethyl-1-prop-2-ynyl-3,7-dihydropurine-2,6-dione | MW 362.4, colorless crystals (yield > 95 %). m.p. 204.2 °C. **$^1$H NMR (DMSO-*$d_6$*): ):** 1.33 (t, J = 6.9 Hz, 3H), 3.09 (t, J = 2.5 Hz, 1H), 3.43 (s, 3H), 4,00 (s, 3H), 4.07 (q, J = 6.9 Hz, 2H), 4.60 (d, J = 2.5 Hz, 2H), 7.07-7.09 (m, 1H), 7.23-7.26 (m, 2H), 7.36-7.39 (m, 1H)ppm. **$^{13}$C NMR (DMSO-*$d_6$*):** 14.7, 29.6, 30.3, 33.2, 63.6, 73.1, 77.2, 79.5, 96.4, 107.5, 117.2, 117.6, 120.9, 124.4, 130.4, 135.1, 147.7, 150.2, 153.2, 158.7 ppm. |
| 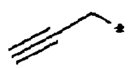 | $-CH_3$ | $-CH_3$ | 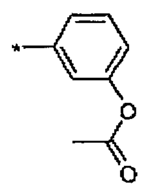 | 8-(3-Acetoxyphenylethyny I)-3,7-dimethyl-1 -prop-2-ynyl-3,7-dihydropurine-2,6-dione | MW 376.4, off white crystals, m.p. 238 °C. **$^1$H NMR ($CDCl_3$): ):** 2.17 (t, J = 2.5 Hz, 1H), 2.30 (s, 3H), 3.59 (s, 3H), 4.07 (s, 3H), 4.78 (d, J = 2.5 Hz, 2H), 7.16-7.18 (m, 1 H), 7.33-7.34 (m, 1H), 7.38-7-41 (m, 1H), 7.45-7.47 (m, 1H) ppm. **$^{13}$C NMR ($CDCl_3$):** 21.1, 29.9, 30.6, 33.3, 70.6, 77.4, 78.5, 96.2, 107.9, 121.6, 123.9, 125.2, 129.6, 129.8, 135.8, 148.2, 150.6, 150.7, 153.8, 169.0 ppm. |
| 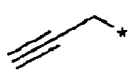 | $-CH_3$ | $-CH_3$ | 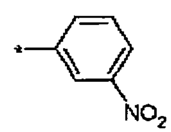 | 3,7-Dimethyl-8-(3-nitrophenylethynyl-1-prop-2-ynyl-3,7-dihydropurine-2,6-dione | MW 363.3, light yellow crystals, m.p. 286-289 °C (dec.). **$^1$H NMR ($CDCl_3$):** 2.18 (t, J = 2.2 Hz, 1H), 3.59 (s, 3H), 4.11 (s, 3H), 4.78 (d, J = 2.2 Hz, 2H), 7.59-7.62 (m, 1H), 7.89-7.92 (m, 1H), 8.27-8.30 (m, 1H), 8.44-8.45 (m, 1 H) ppm. **$^{13}$C NMR ($CDCl_3$):** 29.9, 30.6, 33.4, 70.6, 78.4, 78.9, 94.2, 108.2, 122.2, 124.8, 126.9, 129.9, 135.1, 137.5, 148.2, 150.6, 153.8 ppm. |

(continued)

| R¹ | R² | R³ | R⁴ | Name | MW/ m.p.,/NMR |
|---|---|---|---|---|---|
| 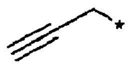 | $-CH_3$ | $-CH_3$ | 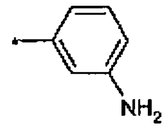 | 8-(3-Aminophenylethynyl)-3,7-dimethyl-1-prop-2-ynyl-3,7-dihydropurine-2,6-dione | MW 333.4, **$^1$H NMR (DMSO-$d_6$):** 3.05 (t, J = 2.5 Hz). 3.43 (s, 3H), 3.97 (s, 3H), 4.60 (d, J = 2.5 Hz, 2H), 677-6.80 (m, 1H), 6.88-6.90 (m, 2H), 7.13-7.17 (m, 1H) ppm. **$^{13}$C NMR (DMSO-$d_6$):** 29.6, 30.2, 33.1, 72.9, 76.4, 79.5, 97.3, 107.4, 117.3, 117.4, 120.1, 120.7, 135.3, 147.4, 147.7, 150.2, 153.1 ppm. |
| 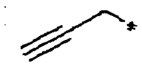 | $-CH_3$ | $-CH_3$ | 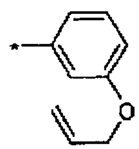 | 8-(3-Allyloxyphenytethynyl )-3,7-dimethyl-1-prop-2-ynyl-3,7-dihydropurine-2,6-dione | MW 374.4, colorless crystals (yield > 95 %), **m.p.** 189.2 °C. **$^1$H NMR ($CDCl_3$):** 2.17 (t, J = 2.2 Hz, 1H), 3.59 (s, 3H), 4.07 (s, 3H), 4.54 (m, 2H), 4.78 (d, J = 2.2 Hz, 2H), 5.29 (m, 1 H), 5.40 (m, 1H), 6.03 (m, 1H), 6.99-7.01 (m, 1H), 7.11-7.12 (m, 1H), 7.18-7.20 (m, 1 H), 7.27-7.30 (m, 1H) ppm. **$^{13}$C NMR ($CDCl_3$) :** 29.9, 30.6, 33.3, 68.9, 70.6, 76.5, 78.5, 97.3, 107.8, 117.6, 117.7, 118.0, 121.3, 124.8, 129.8, 132.6, 136.1, 148.2, 150.7, 153.8, 158.5 ppm. |
| 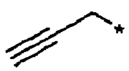 | $-CH_3$ | $-CH_3$ | 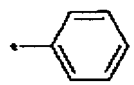 | 3,7-Dimethyl-8-phenylethynyl-1-prop-2-ynyl-3,7-dihydropurine-2,6- | MW 318.3, m.p.: 233 °C [Joe Hipp]; **$^1$H NMR ($CDCl_3$) :** 2.17 (t, J = 2.4 Hz, 1H), 3.59 (s, 3H), 4.07 (s, 3H), 4.78 (d, J = 2.4 Hz, 2H), 7.37-7.45 (m, 3H), 7.58-7.61 (m, 2H) ppm. **$^{13}$C NMR ($CDCl_3$):** 29.9, 30.6, 33.2, 70.6, 76.8, 78.5, 97.5, 107.8, 120.3, 128.7, 130.3, 132.1, 136.2, 148,2, 150.7, 153.8, ppm. |
| 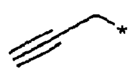 | 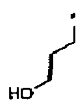 | $-CH_3$ | 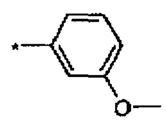 | 3-(3-Hydroxypropyl)-8-[3-methoxyphenylethyn yl]-7-methyl-prop-2-ynyl-3,7-dihydropurine-2,6-dione | MW 392.4, colorless crystals, **m.p.** 177 **°C. $^1$H NMR ($CDCl_3$):** 1.97 (m, 2H, $CH_2$), 2.18 (t, J = 2.5 Hz, 1H), 3.54 (m, 2H, $CH_2$), 3.81 (s, 3H, $CH_3$), 4.07 (s, 3H, $CH_3$), 4.28 (m, 2H, $CH_2$), 4.78 (d, J = 2.5 Hz, 2H), 6.98-6.99 (m, 1H), 7.00-7.09 (m, 1H), 7.10-7.19 (m, 1 H), 7.28-7.31 (m, 1 H) ppm. **$^{13}$C NMR ($CDCl_3$):** 30.6, 31.0, 33.3, 40.0, 55.4, 58.1, 70.7, 76.3, 78.3, 97.8, 107.8, 116.8, 117.1, 121.1, 124.7, 129.8, 136.2, 148.1, 150.9, 153.8, 159.5 ppm. |

(continued)

| $R^1$ | $R^2$ | $R^3$ | $R^4$ | Name | MW/ m.p.,/NMR |
|---|---|---|---|---|---|
| 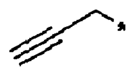 | $-CH_3$ | 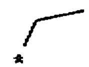 | 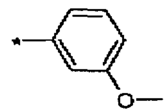 | 7-Ethyl-8-(3-methoxyphenylethyn yl)-3-methyl-1-prop-2-ynyl-3,7-dihydropurine-2,6-dione | MW 362.4, colorless crystals, m.p. 221.9 °C. **$^1$H NMR** ($CDCl_3$): 1.50 (t, J = 7.25 Hz, 3H, $CH_3$), 2.17 (t, J = 2.5 Hz, 1 H), 3.55 (s, 3H, $NCH_3$), 3.81 (s, 3H, $OCH_3$), 4.51 (q, J = 7.75 Hz, 2H, $NCH_2$), 4.78 (d, J = 2.5 Hz, 2H), 6.97-6.99 (m, 1 H), 7.09-7.10 (m, 1H), 7.17-7.19 (m, 1H), 7.28-7.31 (m, 1 H) ppm. **$^{13}$C NMR ($CDCl_3$):** 16-1, 29.9, 30.6, 42.1, 55.4, 70.5, 76-5, 79.5, 96.9, 107.1, 116.8, 116.9, 121.3, 124.6, 129.8,135.2,148.4,150.7,153.4,159.5 ppm. |
| 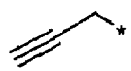 | 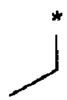 | $-CH_3$ | 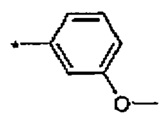 | 3-Ethyl-8-(3-methoxyphenylethyn yl)-7-methyl-1-prop-2-ynyl-3,7-dihydropurine-2,6-dione | MW 362.4, colorless crystals, m.p. 174 °C. **$^1$H NMR** ($CDCl_3$): 1.35 (t, J = 6.9 Hz, 3H, $CH_3$), 2.17 (t, J = 2.5 Hz, 1 H), 3.81 (s, 3H, $OCH_3$), 4.07 (s, 3H, $NCH_3$), 4.19 (q, J = 6.9 Hz, 2H, $NCH_2$), 4.78 (d, J = 2.5 Hz, 2H), 6.97-6.99 (m, 1 H), 7.10-7.11 (m, 1H), 7.18-7.20 (m, 1H), 7.28-7.30 (m, 1H) ppm. **$^{13}$C NMR ($CDCl_3$):** 13.5, 30.5, 33.2. 38.8, 55.4, 70.5, 76.6, 78.6, 97.3, 107.9, 116.8, 116.9, 121.3, 124.6, 129.8, 138.1, 147.8, 150.1, 153.9, 159.5 ppm. |
| 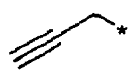 | $-CH_3$ | 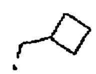 | 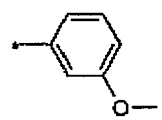 | 7-Cyclobutylmethyl-8-(3-methoxyphenylethyn yl)-3-methyl-1-prop-2-ynyl-3,7-dihydropurine-2,6-dione | MW402.5, colorless crystals, **m.p. ?** °C. **$^1$H NMR** ($CDCl_3$): 1.89 (m, 4H), 2.05 (m, 2H), 2.17 (t, J = 2.5 Hz, 1H), 2.94 (m, 1 H), 3.59 (s, 3H, $NCH_3$), 3.81 (s, 3H, $OCH_3$), 4.48 (d, J = 7.55 Hz, 2H), 4.78 (d, J = 2.5 Hz, 2H), 6.98-7.00 (m, 1H), 7.10-7.11 (m, 1H), 7.18-7.19 (m, 1 H), 7.29-7.32 (m, 1 H) ppm. **$^{13}$C NMR ($CDCl_3$):** 18.3, 25.6, 29.9, 30.6, 36.3, 51.4, 55.4, 70.5, 77.2, 78.6, 96.7, 107.4, 116.7, 116.9, 121.4, 124.5, 129.8, 135.7, 148.3, 150.7, 153.4, 159.5 ppm. |

(continued)

| R¹ | R² | R³ | R⁴ | Name | MW/ m.p.,/NMR |
|---|---|---|---|---|---|
| 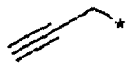 | 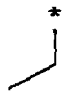 | 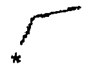 | 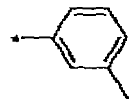 | 3,7-Diethyl-8-(3-methylphenylethynyl) -1-prop-2-ynyl-3,7-dihydropurine-2,6-dione | MW 360.4, colorless crystals, m.p. 178 °C. **$^1$H NMR ($CDCl_3$):** 1.35 (t, J = 7.25 Hz, 3H, $CH_3$), 1.51 (t, J = 6.9 Hz, 3H, $CH_3$), 2.17 (t, J = 2.5 Hz, 1H), 2.36 (s, 3H, $CH_3$), 4.19 (q, J = 6.9 Hz, 2H, $NCH_2$), 4.51 (q, J = 7.25 Hz, 2H, $NCH_2$), 4.78 (d, J = 2.5 Hz, 2H), 7.23-7.29 (m, 2H), 7.39-7.41 (m, 2H) ppm. **$^{13}$C NMR ($CDCl_3$):** 13.4, 16.1, 21.2, 30.5, 38.8, 42.0, 70.5, 76.5, 78.6, 97.2, 107.1, 120.2, 128.6, 129.2, 131.1, 132.6, 135.4, 138.5, 147.9, 150.2, 153.5 ppm. |
| 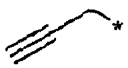 | $-CH_3$ | 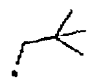 | 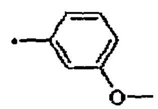 | 7-tert-Butylmethyl-8-(3-methoxyphenylethyn yl)-3-methyl-1-prop-2-ynyl-3,7-dihydropurine-2,6-dione | MW 404.5, colorless crystals, **m.p**. ? °C. **$^1$H NMR ($CDCl_3$):** 1.05 (s, 9H, $C(CH_3)_3$), 2.15 (t, J = 2.5 Hz, 1H), 3.60 (s, 3H, $NCH_3$), 3.80 (s, 3H, $OCH_3$), 4.34 (s, 2H, $CH_2$), 4.77 (d, J = 2.5 Hz, 2H), 6.97-6.99 (m, 1H), 7.08-7.09 (m, 1H), 7.15-7.17 (m, 1H), 7.28-7.31 (m, 1H) ppm. **$^{13}$C NMR ($CDCl_3$):** 27.9, 29.9, 30.7, 34.9, 55.4, 56.9, 70.5, 78.2, 78.6, 96.6, 108.3, 116.6, 116.6, 121.5, 124.4,129.8,136.7,148.2.150.6, 153.5, 159.5 ppm. |
| 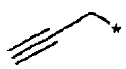 | $-CH_3$ | 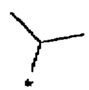 | 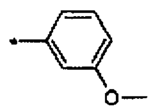 | 8-(3-Methoxyphenylethyn yl)-3-methyl-7- J isopropyl-1-prop-2-ynyl-3,7-dihydropurine-2,6-dione | MW 376.4, colorless crystals, m.p. 181°C. **$^1$H NMR ($CDCl_3$):** 1.69 (d, J = 6.95 Hz, 6H, $C(CH_3)_2$), 2.17 (t, = 2.5 Hz, 1H), 3.59 (s, 3H, $NCH_3$), 3.81 (s, 3H, $OCH_3$), 4.79 (d, J = 2.5 Hz, 2H), 5.29 (m, 1H), 6.97-6.99 (m, 1H), 7.11-7.12 (m, 1H), 7.18-7.20 (m, 1H), 7.28-7.32 (m, 1H) ppm. **$^{13}$C NMR ($CDCl_3$):** 21.7, 29.9, 30.9, 51.4, 55.4, 70.5, 77.7, 78.6, 97.2, 107.0, 116.7, 116.8, 121.5, 124.5. 129.8, 134.6, 149.0, 150.6, 153.1, 159.5 ppm. |

(continued)

| $R^1$ | $R^2$ | $R^3$ | $R^4$ | Name | MW/ m.p.,/NMR |
|---|---|---|---|---|---|
| 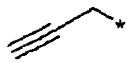 | $-CH_3$ | 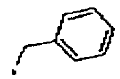 | 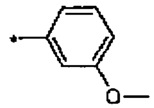 | 7-Benzyl-8-(3-methoxyphenylethyn yl)-3-methyl-1-prop-2-ynyl-3,7-dihydropurine-2,6-dione | MW 424.5, colorless crystals, **m.p.** 175 °C. **$^1$H NMR ($CDCl_3$)**: 2.17 (t, J = 2.5 Hz, 1H), 3.59 (s, 3H, $NCH_3$), 3.81 (s, 3H, $OCH_3$), 4.77 (d, J = 2.5 Hz, 2H), 5.64 (s, 2H, $CH_2$), 6.98-7.49 (m, 9H) ppm. **$^{13}$C NMR ($CDCl_3$):** 29.9, 30.6, 49.9, 55.4, 70.6, 77.3, 78.5, 97.4, 107.2, 116.8, 116.9, 121.2, 124.6, 128.3, 128.5, 128.9, 129.9, 135.3, 135.7, 148.5, 150.6, 153.5, 159.5 ppm |
| 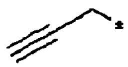 | 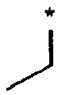 | $-CH_3$ | 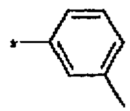 | 3-Ethyl-7-methyl-8-(3-methylphenylethynyl) -1-prop-2-ynyl-3,7-dihydropurine-2,6-dione | MW 346.4, colorless crystals, m.p. 211.7 °C. **$^1$H NMR ($CDCl_3$):** 1.35 (t, J = 6.95 Hz, 3H, $CH_3$), 2.17 (t, J = 2.5 Hz, 1H), 2.36 (s, 3H, $CH_3$), 4.07 (s, 3H, $NCH_3$), 4.18 (q, J = 6.95 Hz, 2H, $NCH_2$), 4.77 (d, J = 2.5 Hz, 2H), 7.24-7.27 (m, 2H), 7.39-7.42 (m, 2H) ppm. **$^{13}$C NMR ($CDCl_3$):** 13.4, 21.2, 30.5, 33.2, 38.8, 70.5, 76.6, 78.6, 97.7, 107.9, 120.2, 128.6, 129.2, 139.2, 132.6, 136.3, 138.5, 147.8, 150.1, 153.9 ppm. |
| 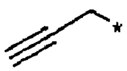 | 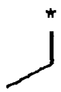 | $-CH_3$ | 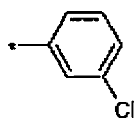 | 8-(3-Chlorophenylethynyl) -3-ethyl-7-methyl-9-prop-2-ynyl-3,7-dihydropurine-2,6-dione | MW 366.8, colorless crystals, **m.p.** 170.5 °C. **$^1$H NMR ($CDCl_3$):** 1.35 (t, J = 7.25 Hz, 3H, $CH_3$), 2.17 (t, J = 2.5 Hz, 1 H), 4.08 (s, 3H, $NCH_3$), 4.18 (q, J = 7.25 Hz, 2H, $NCH_2$), 4.78 (d, J = 2.5 Hz, 2H), 7.31-7.35 (m, 1H), 7.40-7.42 (m, 1H), 7.47-7.49 (m, 1H), 7.58-7.59 (m, 1 H) ppm. **$^{13}$C NMR ($CDCl_3$):** 13.3, 30.5, 33.3, 38.8, 70.5, 77.8, 78.5, 95.6, 108.1, 122.1, 129.9, 130.2, 130.5, 131.9, 134.6, 135.7, 147.7, 150.1, 153.9 ppm. |

(continued)

| $R^1$ | $R^2$ | $R^3$ | $R^4$ | Name | MW/ m.p.,/NMR |
|---|---|---|---|---|---|
| 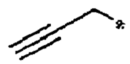 | $-CH_3$ | $-CH_3$ | 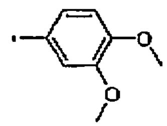 | 8-(3,4-Dimethoxyphenylethynyl)-3,7-dimethyl-1-prop-2-ynyl-3,7-dihydropurine-2,6dione | MW 378.4, colorless crystals, m.p. 235.5 °C. $^1H$ NMR (500 MHz, $CDCl_3$): δ = 2.17 (t, J = 2.52 Hz, 1H, CCH), 3.60 (s, 3H, $OCH_3$), 3.89 (s, 3H, $OCH_3$), 3.91 (s, 3H, $N3CH_3$), 4.07 (s, 3H, $N7CH_3$), 4.79 (d, J = 2.52 Hz, 2H, $\underline{CH_2}$-CCH), 6.86 (d, J = 8.52 Hz, 1 H, 6'H), 7.08 ($\bar{d}$, J =1.89 Hz, 1H, 2'H), 7.22 (dd, J = 1.89 and 8.20 Hz, 1 H, 5'H) ppm. **$^{13}C$ NMR** (125 MHz, $CDCl_3$): δ = 29.7, 30.4, 32.2, 56.0 ($OCH_3$), 56.1 ($OCH_3$), 70.5, 75.7, 78.6, 98.0, 107.4, 111.1, 112.3, 114.4, 126.1, 136.6, 148.3, 148.9, 150.7, 151.2, 153.8 ppm. |
| 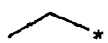 | 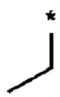 | $-CH_3$ | 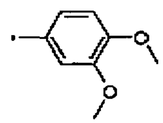 | 1,3-Diethyl-8-(3,4-dimethoxyphenylethy nyl)-7-methyl-3,7-dihydropurine-2,6-dione | MW 382.4, colorless crystals, **m.p.** 197.2 °C. **$^1H$ NMR ($CDCl_3$):** 1.24 (t, J = 6.9 Hz, 3H, $CH_3$), 1.33 (t, J = 6.9 Hz, 3H, $CH_3$), 3.88 (s, 3H, $OCH_3$), 3.90 (s, 3H, $OCH_3$), 4.07 (s, 3H, $NCH_3$), 4.06 (q, J = 6.9 Hz, 2H), 4.15 (q, J = 6.9 Hz, 2H), 6.85 (d, J = 7.2 Hz, 1 H), 7.07 (d, J = 1.9 Hz, 1 H), 7.21 (dd, J = 7.2 and 1.9 Hz, 1 H) ppm. **$^{13}C$ NMR ($CDCl_3$):** 13.3, 13.4, 33.1, 36.5, 38.5, 55.9, 56.0, 75.9, 97.5,108.0, 111.1, 112.5, 114.4, 126.0, 136.0, 147.4, 148.9, 150.6, 161.0, 154.7 ppm. |
| 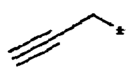 | 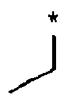 | $-CH_3$ | 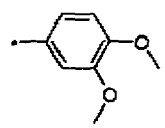 | 3-Ethyl-8-(3,4-dimethoxyphenylethy nyl)-7-methyl-1-prop-2-ynyl-3,7-dihydropurine-2,6-dione | MW 392.4, colorless crystals, m.p. 221.5 °C. **$^1H$ NMR ($CDCl_3$):** 1.35 (t, J = 7.25 Hz, 3H, $CH_3$), 2.17 (t, J = 2.5 Hz, 1H), 3.88 (s, 3H, $OCH_3$), 3.90 (s, 3H, $OCH_3$), 4.07 (s, 3H, $NCH_3$), 4.18 (q, J = 7.25 Hz, 2H, $CH_2$), 4.78 (d, J = 2.5 Hz, 2H), 6.85 (d, J = 8.2 Hz, 1H), 7.07 (d, J = 1.9 Hz, 1H), 7.22 (dd, J = 8.2 and 1.9 Hz, 1H) ppm. **$^{13}C$ NMR ($CDCl_3$):** 13.4, 30.5, 33.2, 38.8, 55.9, 56.0, 70.5, 75.8, 78.6, 97.9, 107.8, 111.1, 112.3, 114.4, 126.1, 136.5, 147.8, 148.9, 150.1, 151.1, 153.8 ppm. |

(continued)

| R¹ | R² | R³ | R⁴ | Name | MW/ m.p.,/NMR |
|---|---|---|---|---|---|
| 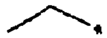 | 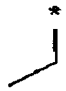 | $-CH_3$ | 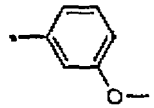 | 1,3-Diethyl-8-(3-methoxyphenylethyn yl)-7-methyl-3,7-dihydropurine-2,6-dione | MW 352.4, colorless crystals, **m.p.** 191.4 °C. **$^1$H NMR ($CDCl_3$):** 1.24 (t, J = 7.25 Hz, 3H, $CH_3$), 1.33 (t, J = 6.95 Hz, 3H; $CH_3$), 3.81 (s, 3H, $OCH_3$), 4.07 (s, 3H, $NCH_3$), 4.06 (q, J = 7.25 Hz, 2H), 4.16 (q, J = 6.9 Hz, 2H), 6.96-6.98 (m, 1H), 7.10-7.11 (m, 1H), 7.17-7.19 (m, 1 H), 7.27-7.30 (m, 1H) ppm. **$^{13}$C NMR** ($CDCl_3$): 13.3, 13.4, 33.1, 36.5, 38.5, 55.4, 76.8, 97.0, 108.2, 118.8, 121.4, 124.6, 129.8, 135.7, 147.4, 150.6, 154.7, 159.4 ppm. |
| 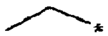 | 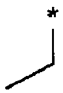 | $-CH_3$ | 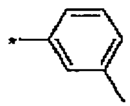 | 1,3-Diethyl-7-methyl-8-(3-methylphenylethynyl) -3,7-dihydropurine-2,6-dione | MW 336.4, colorless crystals, m.p. 154.5 °C. **$^1$H NMR ($CDCl_3$):** 1.24 (t, J = 7.25 Hz, 3H, $CH_3$), 1.33 (t, J = 7.25 Hz, 3H, $CH_3$), 2.35 (s, 3H, $CH_3$), 4.07 (s, 3H, $NCH_3$), 4.05 (q, J = 7.25 Hz, 2H), 4.16 (q, J = 7.25 Hz, 2H), 7.22-7,28 (m, 2H), 7.39-7.41 (m, 2H) ppm. **$^{13}$C NMR ($CDCl_3$):** 13.3, 13.4, 21.2, 33.1, 36.5, 38.6, 77.2, 97.4, 108.1, 120.3, 128.5, 129.2, 131.1, 132.6, 135.8, 138.5, 147.4, 150.6, 154.7 ppm. |
| 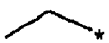 | 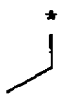 | $-CH_3$ | 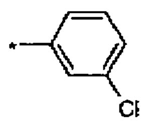 | 8-(3-Chlorophenylethynyl) -1,3-diethyl-7-methyl-3,7-dihydropurine-2,6-dione | MW 356.8, colorless crystals, m.p. 153.6 °C. **$^1$H NMR ($CDCl_3$):** 1.24 (t, J = 6.95 Hz, 3H, $CH_3$), 1.33 (t, J = 7.25 Hz, 3H, $CH_3$), 4.07 (s, 3H, $NCH_3$), 4.06 (q, J = 7.25 Hz, 2H), 4.16 (q, J = 6.95 Hz, 2H), 7.30-7.34 (m, 1 H), 7.39-7.41 (m, 1H), 7.47-7.49 (m, 1 H), 7.57-7.58 (m, 1 H) ppm. **$^{13}$C NMR ($CDCl_3$):** 13.3, 13.4, 33.2, 36.6, 38.6, 78.2, 95.2, 108.4, 122.2, 129.9, 130.2, 130.4, 131.8, 134.6, 135.2, 147.4, 150.5, 154.7 ppm. |

(continued)

| $R^1$ | $R^2$ | $R^3$ | $R^4$ | Name | MW/ m.p.,/NMR |
|---|---|---|---|---|---|
| 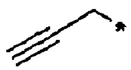 | 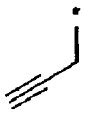 | $-CH_3$ | 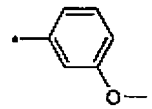 | 8-(3-Methoxyphenylethyn yl)-7-methyl-1,3-diprop-2-ynyl-3,7-dihydropurine-2,6-dione | MW 372.4, colorless crystals, **m.p.** 217.4 °C. **$^1$H NMR ($CDCl_3$):** 2.18 (t, J = 2.55 Hz, 1H), 2.55 (t, J = 2.55 Hz, 1H), 3.81 (s, 3H, $OCH_3$), 4.07 (s, 3H, $NCH_3$), 4.78 (d, J = 2.55 Hz, 2H), 4.88 (d, J = 2.55 Hz, 2H), 6.97-7.00 (m, 1H), 7.10-7.11 (m, 1H), 7.18-7.19 (m, 1H), 7.28-7.31 (m, 1H) ppm. **$^{13}$C NMR ($CDCl_3$):** 30.7, 32.7, 33.3, 55.4, 70.7, 72.2, 76.5, 77.3, 78.3, 97.6, 107.9, 116.8, 116.9, 121.2, 124.7, 129.8, 136.3, 146.9, 149.8, 153.6, 159.5 ppm. |
| 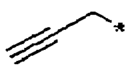 | $-CH_3$ | 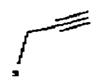 | 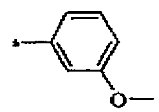 | 8-(3-Methoxyphenylethyn yl)-3-methyl-1,7-diprop-2-ynyl-3,7-dihydropurine-2,6-dione | MW 372.4, colorless crystals, **m.p.** 227.1 °C. **$^1$H NMR ($CDCl_3$):** 2:17 (t, J = 2.5 Hz, 1H), 2.42 (t, J = 2.5 Hz, 1H), 3.60 (s, 3H, $NCH_3$), 3.81 (s, 3H, $OCH_3$), 4.79 (d, J = 2.5 Hz, 2H), 5.28 (d, J = 2.5 Hz, 2H), 6.98-7.00 (m, 1H), 7.13-7.14 (m, 1H), 7.20-7.23 (m, 1 H), 7.29-7.32 (m, 1 H) ppm. **$^{13}$C NMR ($CDCl_3$):** 29.9, 30.6, 35.7, 55.4, 70.6, 74.2, 76.2, 77.2, 78.3, 98.4, 106.7, 116.9, 117.0, 121.1, 124.7, 129.8, 135.8, 148.3, 150.6, 153.4, 159.5 ppm. |
| 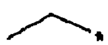 | 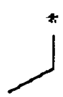 | $-CH_3$ | 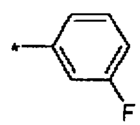 | 1,3-Diethyl-8-(3-fluorophenylethynyl)-7-methyl-3,7-dihydropurine-2,6-dione | MW 340.4, colorless crystals, m.p. 154.7 °C. **$^1$H NMR ($CDCl_3$):** 1.24 (t, J = 6.9 Hz, 3H, $CH_3$), 1.33 (t, J = 7.25 Hz, 3H, $CH_3$), 4.07 (s, 3H, $NCH_3$), 4.07 (q, J = 7.25 Hz, 2H), 4.16 (q, J = 6.9 Hz, 2H), 7.12-7.16 (m, 1H), 7.27-7.28 (m, 1 H), 7.28-7.39 (m, 2H) ppm. **$^{13}$C NMR ($CDCl_3$):** 13.3, 13.4, 33.2, 36.6, 38.6, 77.8. 95.4 (d, J = 3.49 Hz), 108.3, 117.6 (d, J = 21.2 Hz), 118.8 (d, J = 23.4 Hz), 122.3 (d, J = 9.23 Hz), 128.0 (J = 3.24 Hz), 130.4 (J = 8.48 Hz), 135.2, 147.4, 150.5, 154.7, 162.3 (d, 248 Hz) ppm. |

(continued)

| $R^1$ | $R^2$ | $R^3$ | $R^4$ | Name | MW/ m.p.,/NMR |
|---|---|---|---|---|---|
| 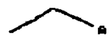 | 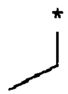 | $-CH_3$ | 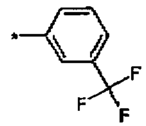 | 1,3-Diethyl-8-(3-trifluoromethylphenyl ethynyl)-7-methyl-3,7-dihydropurine-2,6-dione | MW 390.4, colorless crystals, **m.p.** 164.7 °C. **$^1$H NMR ($CDCl_3$):** 1.24 (t, J = 6.9 Hz, 3H, $CH_3$), 1.33 (t, J = 7.25 Hz, 3H, $CH_3$), 4.07 (q, J = 7.25 Hz, 2H), 4.09 (s, 3H, $NCH_3$), 4.16 (q, J = 6.9 Hz, 2H), 7.54 (dd, J = 7.89 and 7.56 Hz, 1H), 7.68 (d, J = 7.88 Hz, 1H), 7.77 (d, J = 7.88 Hz, 1 H), 7.86 (s, 1 H) ppm. **$^{13}$C NMR ($CDCl_3$):** 13.3, 13.4, 33.3, 36.6, 38.6, 78.4, 95.0, 108.4, 121.6, 122.3, 124.5, 126.6, 128.9, 129.3, 131.5, 135.0, 147.4, 150.5, 154.7 ppm. |
| 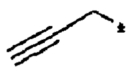 | 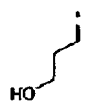 | $-CH_3$ | 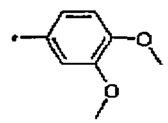 | 3-(3-Hydroxypropyl)-8-(3,4-dimethoxyphenylethy nyl)-7-methyl-1-prop-2-ynyl-3,7-dihydropurine-2,6-dione | MW 422.44, colorless crystals, **m.p.** 203.1 °C. **$^1$H NMR ($CDCl_3$):** 1.97 (tt, J = 5.6/5.7 Hz, 2H, $CH_2$), 2.17 (t, J = 2.5 Hz, 1H), 3.54 (t, J = 5.6 Hz, 2H, $CH_2$), 3.89 (s, 3H, $OCH_3$), 3.90 (s, 3H, $OCH_3$), 4.07 (s, 3H, $N7CH_3$), 4.27 (t, J = 5.7 Hz, 2H, $CH_2$), 4.78 (d, J = 2.5 Hz, 2H), 6.85 (d, J = 8.2 Hz, 1 H), 7.06 (d, J = 1.6 Hz, 1H), 7.22 (dd, J = 8.2/1.6 Hz, 1H) ppm. **$^{13}$C NMR ($CDCl_3$):** 30.6, 31.0, 33.3, 40.0, 56.0, 56.1, 58.0, 70.7, 75.4, 78.3, 98.6, 107.5, 111.1, 112.0, 114.4, 126.2, 138.5, 148.0, 148.9, 150.9, 151.3, 153.6 ppm. |
| 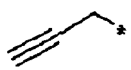 | 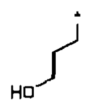 | $-CH_3$ | 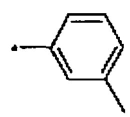 | 3-(3-Hydroxypropyl)-7-methyl-8-(3-methylphenylethynyl) -1-prop-2-ynyl-3,7-dihydropurine-2,6-dione | MW 376.42, colorless crystals, **m.p.** 184 °C. **$^1$H NMR ($CDCl_3$):** 1.97 (m, 2H, $CH_2$), 2.17 (t, J = 2.5 Hz, 1 H), 2.36 (s, 3H, $CH_3$), 3.54 (t, J = 5.4 Hz, 2H, $CH_2$), 4.07 (s, 3H, $NCH_3$), 4.28 (t, J = 6.0 Hz, 2H, $CH_2$), 4.78 (d, J = 2.5 Hz, 2H), 7.24-7.29 (m, 2H), 7.39-7.41 (m, 2H) ppm. **$^{13}$C NMR ($CDCl_3$):** 21.2, 30.6, 31.0, 33.3, 40.0, 58.0, 70.7, 76.2, 78.3, 98.3, 107.7, 120.0, 128.6, 129.3, 131.3, 132.7, 136.3, 138.6, 148.0, 150.9, 153.6 ppm. |
| | | | | | |

(continued)

| $R^1$ | $R^2$ | $R^3$ | $R^4$ | Name | MW/ m.p.,/NMR |
|---|---|---|---|---|---|
| 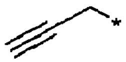 | $-CH_3$ | 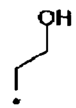 | 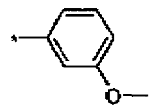 | 7-(2-Hydroxyethyl)-8-(3-methoxyphenylethyn yl)-3-mefhyl-1-prop-2-ynyl-3,7-dihydropurine-2,6-dione | MW 378.39, colorless crystals, **m.p.** 220.2 °C. **$^1$H NMR ($CDCl_3$):** 2.17 (t, J = 2.5 Hz, 1H), 3.60 (s, 3H, $NCH_3$), 3.81 (s, 3H, $OCH_3$), 4.04 (t, J = 5.35 Hz, 2H, $CH_2$), 4.63 (dt, J = 5.35 and 2.5 Hz), 4.78 (d, J = 2.5 Hz, 2H), 6.97-6.99 (m, 1H), 7.09-7.10 (m, 1H), 7.17-7.19 (m, 1 H), 7.27-7.31 (m, 1H) ppm. **$^{13}$C NMR ($CDCl_3$):** 30.0, 30.8, 48.9, 55.4, 62.0, 70.7, 76.5, 78.3, 97.4, 107.8, 116.9, 117.0, 121.1, 124.7, 129.8, 136.3, 148.6, 150.5,154.4, 159.5 ppm. |
| 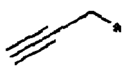 | $-CH_3$ | $-CH_3$ | 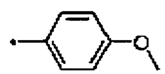 | 8-(4-Methoxyphenylethyn yl)-3,7-dimethyl-1-prop-2-ynyl-3,7-dihydropurine-2,6-dione | MW 348.3 colorless crystals: **m.p.** 245 °C; **$^1$H NMR ($CDCl_3$):** 2.17 (t, J = 2.5 Hz, 1H), 3.59 (s, 3H), 3.83 (s, 3H), 4.06 (s, 3H), 4.78 (d, J = 2.5 Hz, 2H), 6.90 (d, J = 8,8 Hz, 2H), 7.53 (d, J = 8.8 Hz, 2H) ppm. **$^{13}$C NMR ($CDCl_3$):** 29.8, 30.5, 33.2, 55.4, 70.5, 75.9, 78.6, 98.0, 107.6, 114.4, 133.9, 136.6, 148.3, 150.7, 153.7, 161.2 ppm. |
| 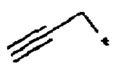 | $-CH_3$ | 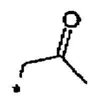 | 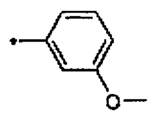 | 7-Acetonyl-8-(3-methoxyphenylethyn yl)-3-methyl-1-prop-2-ynyl-3,7-dihydropurine-2,6-dione | MW 390.4, colorless crystals, m.p. 220.3 °C. **$^1$H NMR ($CDCl_3$):** 2.16 (t, J = 2.5 Hz, 1 H), 2.33 (s, 3H, $COCH_3$), 3.61 (s, 3H, $NCH_3$), 3.81 (s, 3H, $OCH_3$), 4.78 (d, J = 2.5 Hz, 2H), 5.25 (S, 2H), 6.97-6.99 (m, 1H), 7.0 5-7.06 (m, 1H), 7.12-7.14 (m, 1 H), 7.27-7.30 (m, 1H)ppm. **$^{13}$C NMR ($CDCl_3$):** 27.2, 29.9, 30.5, 54.8, 55.4, 62.0, 70.7, 76.0, 78.3, 97.4, 107.3,116.9, 117.0, 121.0, 124.7, 129.8, 136.3, 148.2, 150.6, 153.7, 159.5, 198.7 ppm. |

(continued)

| $R^1$ | $R^2$ | $R^3$ | $R^4$ | Name | MW/ m.p.,/NMR |
|---|---|---|---|---|---|
| 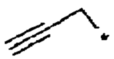 | 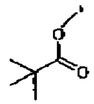 | 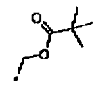 | 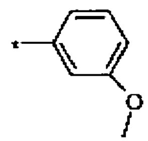 | 2,2-Dimethylpropionic acid 3-(2,2-dimethylpropionyloxy methyl)-8-(3-methoxyphenyl-ethynyl)-1-prop-2-ynyl-3,7-dihydropurine-2,6-dione-7-yl-methyl ester | MW 548.60, colorless crystals: **m.p.** 190.2 ˚C; **$^1$H NMR ($CDCl_3$):** 1.17 (s, 9H, t-$C_4H_9$), 1.19 (s, 9H, t-$C_4H_9$), 2.17 (t, J = 2.5 Hz, 1 H), 3.82 (s, 3H, $OCH_3$), 4.78 (d, J = 2.5 Hz, 2H), 6.11 (s, 2H, $NCH_2O$), 6.40 (s, 2H, $NCH_2O$), 6.98-7.01 (m, 1H), 7.15-7.16 (m, 1 H), 7.20-7.22 (m, 2H), 7.28-7.31 (m, 1 H) ppm. **$^{13}$C NMR ($CDCl_3$):** 26.9, 27.0, 30.7, 38.88, 38.93, 55.4, 66.7, 67.3, 70.9, 75.9, 77.9, 98.4, 107.0, 116.7, 117.6, 120.8, 124.9, 129.8, 136.6, 147.3, 149.7, 152.9, 159.5, 176.9, 177.4 ppm. |
| 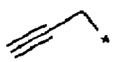 | 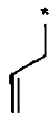 | $-CH_3$ | 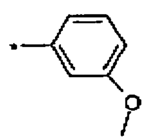 | 3-Allyl-8-(3-methoxyphenylethyn yl)-7-methyl-1-prop-2-ynyl-3,7-dihydropurine-2,6-dione | MW 374.40, colorless crystals, **m.p.** 169.8 ˚C. **$^1$H NMR ($CDCl_3$):** 2.17 (t, J = 2.2 Hz, 1 H), 3.81 (s, 3H, $OCH_3$), 4.07 (s, 3H, $N7CH_3$), 4.72 (m, 2H), 4.78 (d, J = 2.2 Hz, 2H), 5.22 (m, 1 H), 5.28 (m, 1 H), 5.98 (m, 1 H), 6.97-6.99 (m, 1H), 7.10-7.11 (m, 1H), 7.18-7.20 (m, 1H), 7.27-7.31 (m, 1 H) ppm. **$^{13}$C NMR ($CDCl_3$):** 30.5, 33.2, 45.4, 55.4, 70.6, 76.6, 78.5, 97.4, 107.8, 116.8, 118.9, 118.4, 121.3, 124.6, 129.8, 131.2, 136.2, 147.8, 150.2, 153.8, 159.5 ppm. |
| 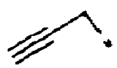 | 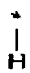 | $-CH_3$ | 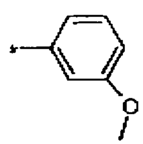 | 8-(3-Methoxyphenylethyn yl)-7-methyl-1-prop-2-ynyl-3,7-dihydropurine-2,6-dione | MW 334.3, colorless crystals, m.p. 281 ˚C. **$^1$H NMR (DMSO-$d_6$):** 3.07 (t, J = 2.5 Hz, 1H), 3.80 (s, 3H, $OCH_3$), 3.97 (s, 3H, $N7CH_3$), 4.55 (d, J = 2.5 Hz, 2H), 7.09-7.11 (m, 1 H), 7.24-7.27 (m, 2H), 7.38-7.41 (m, 1 H), 12.13 (s, 1H, N3H) ppm. **$^{13}$C NMR ($CDCl_3$) :** 29.5, 33.0, 55.6, 72.9, 77.4, 79.7, 96.0, 107.4, 116.7, 117.1, 121.0, 124.5, 130.6, 135.2, 147.0, 150.2, 153.9, 159.4 ppm. |

(continued)

| $R^1$ | $R^2$ | $R^3$ | $R^4$ | Name | MW/ m.p.,/NMR |
|---|---|---|---|---|---|
| 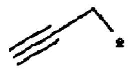 | $-CH_3$ | $-CH_3$ | 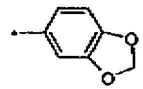 | 8-(3,4-Methylenedioxyphen ylethynyl)-3,7-dimethyl-1-prop-2-ynyl-3,7-dihydropurine-2,6-dione | MW 362.35, colorless crystals: m.p. 273.5 °C; **$^1$H NMR ($CDCl_3$):** 2.17 (t, J = 2.6 Hz, 1H), 3.59 (s, 3H), 4.05 (s, 3H), 4.78 (d, J = 2-6 Hz, 2H), 6.02 (s, 2H, $O_2CH_2$), 6.81 (d, J = 8.2 Hz, 1 H), 7.00 (d, J = 1.8 Hz, 1 H), 7.14 (dd, J = 1.6 and 8.2) ppm. **$^{13}$C NMR ($CDCl_3$):** 29.8, 30.5, 33.2, 70.5, 75.2, 78.5, 97.7, 101.8, 107.7 ($O_2CH_2$) 108.8, 111.7, 113.4, 127.6, 136.4, 147.7, 148.3, 149.7, 150.7, 153.7 ppm. |
| 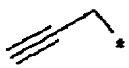 | 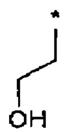 | $-CH_3$ | 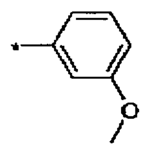 | 3-(2-Hydroxyethyl)-8-(3-methoxyphenylethyn yl)-7-methyl-1-prop-2-ynyl-3,7-dihydropurine-2,6-dione | MW 378.39, colorless crystals, m.p. 212.8 °C. **$^1$H NMR ($CDCl_3$):** 2.18 (t, J =2.5 Hz, 1H), 2.79 (t, J = 5.4 Hz, 1H, OH), 3.81 (s, 3H, $OCH_3$), 3.98 (m, 2H, $CH_2$), 4.07 (s, 3H, $N7CH_3$), 4.37 (m, 2H, $CH_2$), 4.78 (d, J = 2.5 Hz, 2H), 6.98-7.00 (m, 1H), 7.09-7.10 (m, 1 H), 7.17-7.19 (m, 1 H), 7.28-7.31 (m, 1 H) ppm. **$^{13}$C NMR ($CDCl_3$):** 30.7, 33.3, 46.2, 55.4, 61.5, 70.7, 76.4, 78.3, 97.6, 107.9, 116.8, 117.0, 121.2, 124.7, 129.8, 136.0, 148.0, 151.2, 153.6, 159.5 ppm. |
| 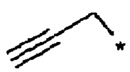 | 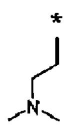 | $-CH_3$ | 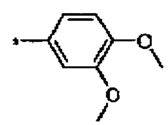 | 8-(3,4-Dimethoxyphenylethy nyl)-3-(2-dimethylaminoethyl)-7-methyl-1-prop-2-ynyl-3,7-dihydropurine-2,6-dione | MW 435.5, colorless crystals, m.p. 201.1 °C. **$^1$H NMR ($CDCl_3$):** 2.16 (t, J = 2.2 Hz, 1H), 2.30 (s, 6H, $N(CH_3)2$), 2.70 (t, J = 6.6 Hz, 2H, $CH_2$), 3.89 (s, 3H, $OCH_3$), 3.91 (s, 3H, $OCH_3$), 4.05 (s, 3H, $N7CH_3$), 4.23 (t, J = 6.6 Hz, 2H, $CH_2$), 4.77 (d, J = 2.2 Hz, 2H), 6.85 (d, J = 8.5 Hz, 1 H), 7.07 (d, J = 2.2 Hz, 1H), 7.22 (dd, J = 2.2/8.5 Hz, 1H) ppm. **$^{13}$C NMR ($CDCl_3$):** 30.5, 33.2, 41.4, 45.7, 55.99, 56.0, 56.8, 70.5, 75.9, 78.6, 97.7, 107.8, 111.1, 112.3, 114.4, 126.1, 136.4, 148.1, 148.9, 150.5, 151.1, 153.9 ppm. |

(continued)

| R[1] | R[2] | R[3] | R[4] | Name | MW/ m.p.,/NMR |
|---|---|---|---|---|---|
| 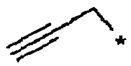 | $-CH_3$ | $-CH_3$ | 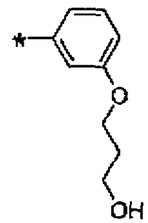 | 8-[3-(3-Hydroxypropoxy)phe nylethynyl]-3,7-dimethyl-1-prop-2-ynyl-3,7dihydropurine-2,6-dione | MW 392.4, colorless crystals, **m.p.** 199.2 °C. **$^1$H NMR ($CDCl_3$):** 1.68 (t, J = 5.4 Hz, 1H, OH), 2.04 (m, 2H, $CH_2$), 2.17 : (t, J = 2.2 Hz, 1H), 3.59 (s, 3H, $N3CH_3$), 3.85 (m, 2H, $CH_2$), 4.07 (s, 3H, $N7CH_3$), 4.12 (m, 2H, $CH_2$), 4.78 (d, J = 2.2 Hz, 2H $CH_2$), 6.97-6.99 (m, 1H), 7.11-7.12 (m, 1H), 7.17-7.19 (m, 1H), 7.27-7.30 (m, 1H) ppm. **$^{13}$CNMR ($CDCl_3$):** 29.9, 30.6, 31.9, 33.2, 60.0, 65.7, 70-6, 76.6, 78.5, 97.3,107.8,117.3,117.4,121.3, 124.7, 129.8, 136.1, 148.2, 150.7, 153.8, 158.7 ppm. |
| 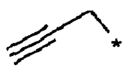 | $-CH_3$ | $-CH_3$ | | 8-[3-(3-Dimethylaminopropo xy)phenylethynyl]- | MW 419.5, colorless crystals, m.p. 135.9 °C. **$^1$H NMR ($CDCl_3$):** 1.95 (dt, J = 7 Hz, 2H, $CH_2$), 2.17 (t, J = 2.6 Hz, 1H), 2.24 (s, 6H, $N(CH_3)_2$), 2.44 (t, J =7 Hz, |
| | | | 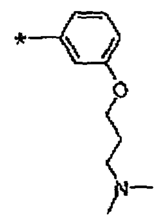 | 3,7-dimethyl-1-prop-2-ynyl-3,7dihydropurine-2,6-dione | 2H, $CH_2$), 3.59 (s, 3H, $N3CH_3$), 4.01 (t, J =7 Hz, 2H, $CH_2$), 4.07 (s, 3H, $N7CH_3$), 4.78 (d, J = 2.6 Hz, 2H $CH_2$), 6.97-6.99 (m, 1H), 7.11 (m, 1H), 7.16-7.18 (m, 1H), 7.26-7.28 (m, 1H) ppm. **$^{13}$C NMR ($CDCl_3$):** 27.4, 29.9, 30.6, 33.3, 45.5, 56.2, 66.4, 70.6, 76.5, 78.5, 97.5, 107.8, 117.4, 117.5, 121.2, 124.5, 129.8, 136.2, 148.2, 150.7, 153.8, 158.9 ppm. |
| 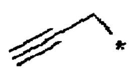 | $-CH_3$ | $-CH_3$ | 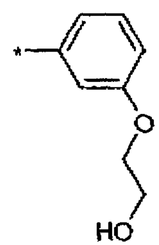 | 8-[3-(2-Hydroxyethoxy)phen ylethynyl]-3,7-dimethyl-1-prop-2-ynyl-3,7dihydropurine-2,6-dione (JH06041): | MW 378.4, colorless crystals, **m.p.** 242.7 °C. **$^1$H NMR (DMSO-$d_6$):** 3.09 (t, J = 2.6 Hz, 1H), 3.43 (s, 3H, $N3CH_3$), 3.72 (q, J = 5.1 Hz, 2H, $CH_2$), 4.00 (s, 3H, $N7CH_3$), 4.04 (t, J = 4.7 Hz, 2H, $CH_2$), 4.60 (d, J = 2.6 Hz, 2H, $CH_2$), 4.85 (t, J = 5.4 Hz, 1H, OH), 7.09-7.12 (m, 1H), 7.26-7.27 (m, 2H), 7.37-7.40 (m, 1H) ppm. **$^{13}$C NMR (DMSO-$d_6$):** 29.6, 30.3, 33.3, 59.6, 70.0, 73.1, 77.2, 79.5, 96.4, 107.5, 117.3, 117.7, 120.8, 124.4, 130.1, 135.1, 147.7, 150.2, 153.2, 158.9 ppm. |

(continued)

| $R^1$ | $R^2$ | $R^3$ | $R^4$ | Name | MW/ m.p.,/NMR |
|---|---|---|---|---|---|
| 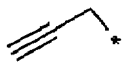 | $-CH_3$ | $-CH_3$ | 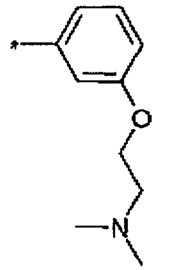 | 8-[3-(2-Dimethylaminoethoxy )phenylethynyl]-3,7-dimethyl-1-prop-2-ynyl-3,7dihydropurine-2,6-dione | MW 405.5, colorless crystals, m.p. 159.7 °C. **$^1$H NMR ($CDCl_3$):** 2.17 (t, J = 2.6 Hz, 1 H), 2.32 (s, 6H, $N(CH_3)_2$), 2.73 (t, J = 5.7 Hz, 2H, $CH_2$), 3.59 (s, 3H, $N3CH_3$), 4.06 (t, J = 5.7 Hz, 2H, $CH_2$), 4.07 (s, 3H, $N7CH_3$), 4.78 (d, J = 2.6 Hz, 2H $CH_2$), 6.99-7.02 (m, 1 H), 7.12-7.13 (m, 1H), 7.17-7.19 (m, 1H), 7.26-7.30 (m, 1 H) ppm. **$^{13}$C NMR ($CDCl_3$):** 29.9, 30.6, 33.3, 45.9, 58.1, 66.2, 70.6, 76.5, 78.5, 97.4, 107.8, 117.48, 117.52, 121.2, 124.7, 129.8, 136.1, 148.2, 150.7, 153.8, 158.7 ppm. |
| 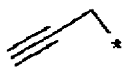 | $-CH_3$ | $-CH_3$ | 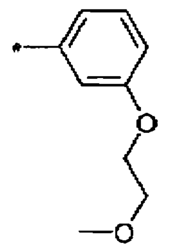 | 8-[3-(2-Methoxyethoxy)phen ylethynyl]-3,7-dimethyl-prop-2-ynyl-3,7dihydropurine-2,6-dione | MW 392.4, colorless crystals, m.p. 200.8 °C. **$^1$H NMR ($CDCl_3$):** 2.17 (t, J = 2.6 Hz, 1H), 3.44 (s, 3H, $COCH_3$), 3.59 (s, 3H, $N3CH_3$), 3.74 (t, J = 4.8 Hz, 2H, $CH_2$), 4.07 (s, 3H, $N7CH_3$), 4.12 (t, J = 4.8 Hz, 2H, $CH_2$), 4.78 (d, J = 2.6 Hz, 2H $CH_2$), 7.00-7.03 (m, 1H), 7.13 (m, 1H), 7.18-7.20 (m, 1H), 7.27-7.30 (m, 1H) ppm. $^{13}$C **NMR ($CDCl_3$):** 29.9, 30.6, 33.2, 59.3, 67.5, 70.6, 70.8, 76.5, 78.5, 97.4, 107.5, 117.5, 117.6, 121.2, 124.8, 129.8, 136.1, 148.2, 150.7, 153.8, 15.7 ppm. |

(continued)

| $R^1$ | $R^2$ | $R^3$ | $R^4$ | Name | MW/ m.p.,/NMR |
|---|---|---|---|---|---|
| 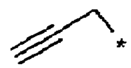 | 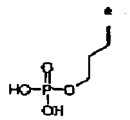 | $-CH_3$ | 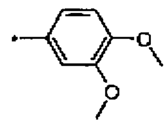 | (E)-Phosphoric Acid mono{3-[8-[(3,4-dimethoxyphenyl)eth ynyl]-7-methyl-2,6-dioxo-1-prop-2-ynyl-1,2,6,7-tetrahydropurin-3-yl] propyl} ester | MW 502.4, pale yellow solid, m.p. 182.3 °C. **$^1$H NMR** (500 MHz, DMSO-$d_6$):δ = 1.95-2.02 (m, 2H), 3.09 (t, J = 2.52 Hz, 1H, CCH), 3.81 (s, 3H, $OCH_3$), 3.82 (s, 3H, $OCH_3$), 3.90 (q, J = 6.62 Hz, 2H), 4.00 (s, 3H, $N7CH_3$), 4.06 (t, J = 6.93 Hz, 2H), 4.60 (d, J = 2.52 Hz, 2H, $CH_2$-CCH), 7.05 (d, J = 8.20 Hz, 1H, 6'H), 7.27 (d, J = 1.89 Hz, 1 H, 2'H), 7.31 (dd, J = 1.89 and 8.20 Hz, 1H, 5'H) ppm. **$^{13}$C NMR** (125 MHz, DMSO-$d_6$): δ = 28.7 (d, J = 7.23 Hz, $CH_2CH_2CH_2O$), 30.3, 33.2, 40.6, 55.8 ($OCH_3$), 55.9 ($OCH_3$), 63.3 (d, J = 4.98 Hz, $CH_2CH_2CH_2O$), 73.1, 76.3, 79.6, 97.4, 107.5, 111.6, 112.1, 114.8, 126.0, 135.6, 147.4, 148.9, 149.9, 151.1, 153.2 ppm. **$^{31}$P NMR** (202 MHz, DMSO-$d_6$): δ = -0.62 (dd, J = 4.92 and 7.38 Hz) ppm. **ESI** +Q1 m/z 503 (M + $H^+$). |
| 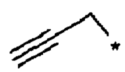 | 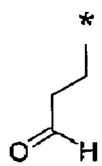 | $-CH_3$ | 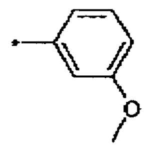 | 3-{8-[(3-Methoxyphenyl)ethyn yl]-7-methyl-2,6-dioxo-1-prop-2-ynyl-1,2,6,7-tetrahydropurin-3-yl}propanal | MW 390.4, colorless crystals, m.p. 151.2 °C. $^1$H **NMR** (500 MHz, $CDCl_3$): δ = 2.18 (t, J = 2.52 Hz, 1 H, CCH), 2.99 (dt, J = 1.58/6.94 Hz, 2H), 3.82 (s, 3H, $OCH_3$), 3.97 (s, 3H, $N7CH_3$), 4.48 (t, J = 6.94 Hz, 2H), 4.76 (d, J = 2.52 Hz, 2H, $CH_2$-CCH), 6.99 (ddd, J = 1.26/2.52/8.43 Hz, 1H, 5'-H), 7.11 (dd, J = 1.26 and 2.52 Hz, 1H, 2'H), 7.19 (dt, J = 1.26 and 7.88 Hz, 1H, 6'H), 7.30 (t, J = 7.88 Hz, 1H, 4'H), 9.84 (t, J = 1.26 Hz, 1 H, CHO) ppm. **$^{13}$C NMR** (125 MHz, $CDCl_3$): δ = 30.6, 33.3, 37.6, 42.2, 55.4 ($OCH_3$), 70.7, 76.4, 78.3, 97.5, 108.0, 116.8, 117.0, 121.2, 124.7, 129.8, 136.2, 147.4, 150.3, 153.6, 159.5, 199.6 ppm. |

(continued)

| $R^1$ | $R^2$ | $R^3$ | $R^4$ | Name | MW/ m.p.,/NMR |
|---|---|---|---|---|---|
| $-CH_3$ | $-CH_3$ | $-CH_3$ | 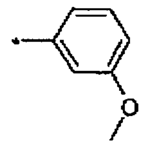 | 8-(3-Methoxyphenylethyn yl)-1,3,7-trimethyl-3,7-dihydropurine-2,6-dione | MW 324.3 colorless crystals, m.p. 210.3 °C. **$^1$H NMR ($CDCl_3$):** 3.40 (s, 3H, $N1CH_3$), 3.58 (s, 3H, $N7CH_3$), 3.81 (s, 3H, $OCH_3$), 4.07 (s, 3H, $N7CH_3$), 6.97-6.99 (m, 1H), 7.01-7.11 (m, 1H), 7.18-7.20 (m, 1H), 7.27-7.30 (m, 1 H) ppm. **$^{13}$C NMR ($CDCl_3$):** 28.0, 29.8, 33.2, 55.4, 76.7, 97.1, 107.9, 116.80, 116.82, 121.3, 124.6, 129.8, 135.7, 147.9, 151.5, 154.9, 159.5 ppm. |
| $-CH_3$ | $-CH_3$ | $-CH_3$ | 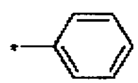 | 1,3,7-Trimethyl-8-(phenylethynyl)-3,7-dihydropurine-2,6-dione | MW 294.3, colorless crystals, **m.p.** 234.7 °C. **$^1$H NMR ($CDCl_3$):** 3.40 (s, 3H, $N1CH_3$), 3.58 (s, 3H, $N7CH_3$), 4.07 (s, 3H, $N7CH_3$), 7.37-7.41 (m, 3H), 7.58-7.60 (m, 2H) ppm. **$^{13}$C NMR ($CDCl_3$):** 28.0, 29.8, 33.2, 76.9, 97.2, 107.9, 120.4, 128.7, 130.2, 132.1, 135.8, 147.9, 151.5, 154.9 ppm. |
| $-CH_3$ | $-CH_3$ | $-CH_3$ | 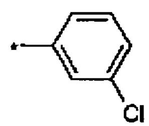 | 8-(3-Chlorphenylethynyl)-1,3,7-trimethyl-3,7-dihydropurine-2,6-dione | MW 328.8, colorless crystals, **m.p.** 233.8 °C. **$^1$H NMR ($CDCl_3$):** 3.40 (s, 3H, $N1CH_3$), 3.58 (s, 3H, $N7CH_3$), 4.07 (s, 3H, $N7CH_3$), 7.31-7.34 (m, 1H), 7.40-7.42 (m, 1H), 7.47-7.49 (m, 1H), 7.57-7.58 (m, 1H) ppm. **$^{13}$C NMR** ($CDCl_3$): 28.1, 29.8, 33.2, 77.9, 95.4, 108.1, 122.1, 130.0, 130.2, 130.5, 131.8, 134.6, 135.2, 147.9, 151.5, 154.9 ppm. |
| $-CH_3$ | $-CH_3$ | $-CH_3$ | 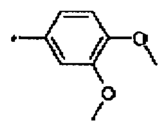 | 8-(3,4-Dimethoxyphenylethynyl)-1,3,7-trimethyl-3,7-dihydropurine-2,6-dione | MW 354.4, colorless crystals, m.p. 271.9 °C. **$^1$H NMR ($CDCl_3$):** 3.40 (s, 3H, $N1CH_3$), 3.58 (s, 3H, $N7CH_3$), 3.88 (s, 3H, $OCH_3$), 3.90 (s, 3H, $OCH_3$), 4.07 (s, 3H, $N7CH_3$), 6.85 (d, J = 8.2 Hz, 1H), 7.07 (d, J = 1.9 Hz, 1H), 7.21 (dd, J = 1.9 and 8.2 Hz, 1H) ppm. **$^{13}$C NMR ($CDCl_3$):** 28.0, 29.8, 33.2, 55.99, 56.03, 75.8, 97.7, 107.8, 111.1, 112.4, 114.4, 126.0, 136.1, 147.9, 148.9, 151.1, 151.6, 154.9 ppm. |

(continued)

| $R^1$ | $R^2$ | $R^3$ | $R^4$ | Name | MW/ m.p.,/NMR |
|---|---|---|---|---|---|
| 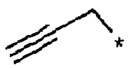 | 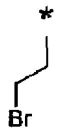 | $-CH_3$ | 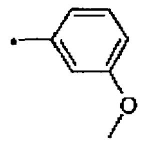 | 3-(2-Bromoethyl)-8-(3-methoxyphenylethyn yl)-7-methyl-1-prop-2-ynyl-3,7-dihydropurine-2,6-dione | MW 441.3, colorless crystals, m.p. 162.6 °C. **$^1$H NMR ($CDCl_3$):** 2.18 (t, J = 2.5 Hz, 1H), 3.69 (t, J = 7.0, 2H, $CH_2Br$), 3.82 (s, 3H, $OCH_3$), 4.07 (s, 3H, $N7CH_3$), 4.51 (t, J = 7.0, 2H, $CH_2\underline{CH_2}N$), 4.77 (d, J = 2.5 Hz, 2H), 6.98-7.00 (m, 1H), 7.10-7.11 (m, 1H), 7.19-7.20 (m, 1H), 7.27-7.31 (m, 1H) ppm. **$^{13}$C NMR ($CDCl_3$):** 27.3, 30.6, 33.3, 44.3, 55.4, 70.7, 76.5, 78.3, 97.6, 107.8, 116.8, 117.0, 121.2, 124.7, 129.8, 131.2, 147.4, 150.2, 153.6, 159.5 ppm. |
| 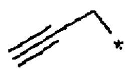 | 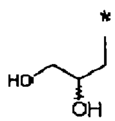 | $-CH_3$ | 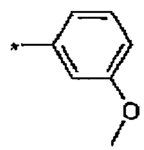 | (R/S)-3-(2,3-Dihydroxypropyl)-8-(3-methoxyphenylethyn yl)-7-methyl-1-prop-2-ynyl-3,7-dihydropurine-2,6-dione | MW 408.4, colorless crystals, **m.p.** 176.1 °C. **$^1$H NMR ($CDCl_3$):** 2.19 (t, J = 2.6 Hz, 1H), 3.28 (d, J = 5.7 Hz, 1H), 3.40 (t, J = 7.0 Hz), 3.58 (m, 2H, $CH_2$), 3.82 (s, 3H, $OCH_3$), 4.07 (s, 3H, $N7CH_3$), 4.08 (m, 1H), 4.33 (ddd, J = 5.4, 14.5 and 35.6 Hz), 6.98-7.00 (m, 1H), 7.09-7.10 (m, 1H), 7.17-7.19 (m, 1H), 7.28-7.32 (m, 1H) ppm. **$^{13}$C NMR ($CDCl_3$):** 30.8, 33.4, 45.6, 55.4, 62.9, 70.3, 70.9, 76.1, 78.1, 98.1, 107.8, 116.8, 117.1, 121.0, 124.7, 129.9, 136.1, 146.0, 151.7, 153.4, 159.5 ppm. |
| 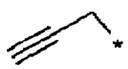 | 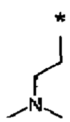 | $-CH_3$ | 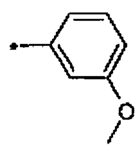 | 8-(3-Methoxyphenylethyn yl)-3-(2-dimethylaminoethyl)-7-methyl-1-prop-2-ynyl-3,7-dihydropurine-2,6-dione | MW 405.5, colorless crystals, m.p. 162 °C. **$^1$H NMR ($CDCl_3$):** 2.16 (t, J = 2.5 Hz, 1 H), 2.30 (s, 6H, $NCH_3$), 2.70 (t, J = 6.7 Hz, 2H, $CH_2$), 3.82 (s, 3H, $OCH_3$), 4.06 (s, 3H, $N7CH_3$), 4.23 (t, J = 6.7 Hz, 2H, $CH_2$), 4.77 (d, J = 2.5 Hz, 2H), 6.97-6.99 (m, 1H), 7.11 (m, 1H), 7.18-7.20 (m, 1 H), 7.28-7.31 (m, 1 H) ppm. **$^{13}$C NMR ($CDCl_3$):** 30.5, 33.2, 41.4, 45.8, 55.4, 56.8, 70.5, 77.2, 78.6, 97.2, 108.0, 116.8, 116.9, 121.4, 124.7, 129.8, 136.0, 148.0, 150.5, 153.9, 159.5 ppm. |

(continued)

| $R^1$ | $R^2$ | $R^3$ | $R^4$ | Name | MW/ m.p.,/NMR |
|---|---|---|---|---|---|
| 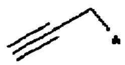 | 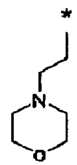 | $-CH_3$ | 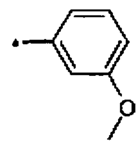 | 8-(3-M ethoxyp hen ylethyn yl)-7-methyl-3-(2-morpholin-4-ylethyl)-1-prop-2-ynyl-3,7-dihydropurine-2,6-dione | MW 447.5, colorless crystals, m.p. 196.7 °C. **$^1$H NMR ($CDCl_3$):** 2.17 (t, J = 2.6 Hz, 1H), 2.53 (s br, 4H, 2 $CH_2$) 2.72 (t, J = 6.6 Hz, 2H, $CH_2$), 3.62 (t, J = 4.7 Hz, 4H, 2 $CH_2$), 3.82 (s, 3H, $OCH_3$), 4.07 (s, 3H, N7$CH_3$), 4.25 (t, J = 6.6 Hz, 2H, $CH_2$), 4.77 (d, J = 2.6 Hz, 2H), 6.97-6.99 (m, 1H), 7.10-7.11 (m, 1H), 7.18-7.20 (m, 1 H), 7.28-7.31 (m, 1 H) ppm. **$^{13}$C NMR ($CDCl_3$):** 30.5, 33.2, 40.4, 53.7, 55.4, 55.8, 67.0, 70.5, 76.6, 78.6, 97.2, 107.9, 116.9, 121.3, 124.6, 129.8, 136.0, 148.0, 150.5, 153.9, 159.5 ppm. |
| 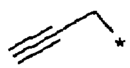 | 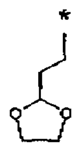 | $-CH_3$ | 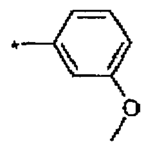 | 3-[2-(1,3-Dioxolan-2-yl)ethyl]-8-[(3-methoxyphenyl)ethyn yl]-7-methyl-1-prop-2-ynyl-3,7-dihydropurine-2,6-dione | MW 434.5, colorless crystals, **m.p.** 133.9 °C. **$^1$H NMR** (500 MHz, $CDCl_3$): δ = 2.15 (t, J = 2.52 Hz, 1 H, CCH), 2.16 - 2.20 (m, 2H), 3.79 - 3.82 (m, 2H), 3.83 (s, 3H, OCH), 3.92 - 3.96 (m, 2H), 4.07 (s, 3H, N7$CH_3$), 4.34 (t, J = 6.94 Hz, 2H), 4.79 (d, J = 2.52 Hz, 2H, C<u>H</u>$_2$-CCH), 5.01 (t, J = 4.42 Hz, 1H, CH), 6.98 (d<u>d</u>d, J = 1.26/2.52 and 8.43 Hz, 1H, 5'-H), 7.11 (dd, J =1.26 and 2.52 Hz, 1H, 2'H), 7.19 (d, J = 7.88 Hz, 1H, 6'H), 7.29 (t, J = 7.88 Hz, 1 H, 4'H) ppm. **$^{13}$C NMR** (125 MHz, $CDCl_3$): δ = 30.3, 31.4, 33.3, 38.5, 55.6 ($OCH_3$), 64.5, 73.1, 77.3, 79.5. 96.4, 101.8, 107.7, 116.7, 117.3, 120.9, 124.5, 130.3, 135.1, 147.3, 149.8, 153.2, 159.4 ppm. |
| 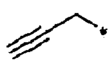 | 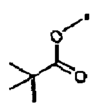 | $- CH_3$ | 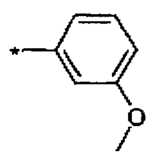 | 2,2-Dimethylpropionic acid 8-(3-methoxyphenylethyn yl)-7-methyl-1-prop-2-ynyl-3,7-dihydropurine-2,6-dione-3-ylmethyl ester | MW 448.5, colorless crystals: m.p. 219.2 °C; **$^1$H NMR ($CDCl_3$):** 1.17 (s, 9H, t-$C_4H_9$), 2.17 (t, J = 2.5 Hz, 1 H), 3.81 (s, 3H, $OCH_3$), 4.07 (s, 3H, N7$CH_3$), 4.78 (d, J = 2.5 Hz, 2H), 6.10 (s, 2H, $NCH_2O$), 6.97-7.00 (m, 1H), 7.10-7.11 (m, 1H), 7.18-7.20 (m, 2H), 7.30-7.31 (m, 1H) ppm. **$^{13}$C NMR ($CDCl_3$):** 27.0, 30.6, 33.3, 38.9, 55.4, 66.7, 70.8, 76.5, 78.1, 97.5, 107.8, 116.8, 117.0, 1.21.2, 124.6, 129.8, 136.3, 146.9, 149.8, 153.7, 159.5, 177.4 ppm. |

(continued)

| $R^1$ | $R^2$ | $R^3$ | $R^4$ | Name | MW/ m.p.,/NMR |
|---|---|---|---|---|---|
| 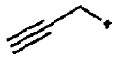 | 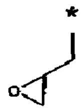 | $-CH_3$ | 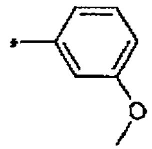 | 8-(3-Methoxyphenylethyn yl)-7-methyl-3-(2-oxiran-2-ylmethyl)-1-prop-2-ynyl-3,7-dihydropurine-2,6-dione | MW 390.4, colorless crystals, m.p. 190.5 ˚C. **$^1$H NMR ($CDCl_3$):** 2.18 (t, J = 2.6 Hz, 1H), 2.76 (dd, J = 2.6 and 5.0 Hz, 1H), 2.81 (dd, J = 3.8 and 5.0 Hz, 1H), 3.39 (m, 1H), 3.82 (s, 3H, $OCH_3$), 4.07 (s, 3H, $N7CH_3$), 4.31 (ddd, J = 5, 15 and 59 Hz, 2H), 6.97-6.99 (m, 1 H), 7.10-7.11 (m, 1 H), 7.18-7.20 (m, 1 H), 7.28-7.31 (m, 1H) ppm. **$^{13}$C NMR ($CDCl_3$):** 30.6, 33.3, 45.1, 46.3, 59.0, 55.4, 70.7, 76.6, 78.4, 97.5, 107.9, 116.8, 116.9, 121.2, 124.7, 129.8, 136.2, 147.8, 150.5, 153.7, 159.5 ppm. |
| 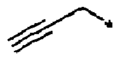 | 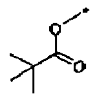 | $-CH_3$ | 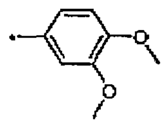 | {8-[(3,4-Dimethoxyphenyl)eth ynyl]-7-methyl-2,6-dioxo-1-prop-2-ynyl-1,2,6,7-tetra hydropurin-3-yl}methyl pivalate | MW 478.5, colorless crystals, **m.p.** 201.1 ˚C. **$^1$H NMR** (500 (MHz, $CDCl_3$): $\delta$ = 1.18 (s, 9H, $C(CH_3)_3$), 2:17 (t, J = 2.52 Hz, 1H, CCH), 3.89 (s, 3H, $OCH_3$), 3.91 (s, 3H, $OCH_3$), 4.07 (s, 3H, $N7CH_3$), 4.78 (d, J = 2.52 Hz, 2H, $C\underline{H}_2$-CCH), 6.10 (s, 2H, $CH_2O$), 6.86 (d, J = 8.51 Hz, 1 H, 6'H), 7.08 (d, J = 1.89 Hz, 1H, 2'H), 7.22 (dd, J =1,89 and 8.20 Hz, 1H, 5'H) ppm. **$^{13}$C NMR** (125 MHz, $CDCl_3$):$\delta$ = 27.0 ($C(\underline{C}H_3)_3$), 30.6, 33.2, 38.9, 56.0 ($OCH_3$), 56.1 ($OCH_3$), 66.7, 70.8, 75.7, 78.2, 98.2, 107.7, 111.1, 112.2, 114.4, 126.1, 136.7, 147.0, 148.9, 149.8, 151.2, 153.6, 177.4 ppm. |

(continued)

| R1 | R2 | R3 | R4 | Name | MW/ m.p.,/NMR |
|---|---|---|---|---|---|
| 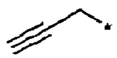 | 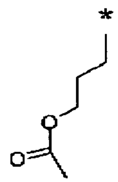 | $-CH_3$ | 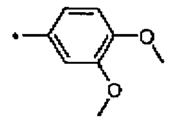 | 3-{8-[(3,4-Dimethoxyphenyl)eth ynyl]-7-methyl-2,6-dioxo-1-prop-2-ynyl-1,2,6,7-tetrahydropurine-3-yl}propyl acetate | MW 464.5, colorless crystals, m.p. ?? ˚C. **$^1$H NMR** (500 MHz, $CDCl_3$): $\delta$ = 2.03 (s, 3H, $CO_2CH_3$), 2.13 (t, J = 6.62 Hz, 2H), 2.17 (t, J = 2.21 Hz, 1H, CCH), 3.89 (s, 3H, $OCH_3$), 3.91 (s, 3H, $OCH_3$), 4.06 (s, 3H, N7$CH_3$), 4.12 (t, J = 6.31 Hz, 2H), 4.24 (t, J = 6.93 Hz, 2H), 4.77 (d, J = 2.21 Hz, 2H, C$\underline{H_2}$-CCH), 6.86 (d, J = 8.19 Hz, 1H, 6'H), 7.07 (d, J = 1.89 Hz, 1H, 2'H), 7.22 (dd, J = 1.89 and 8.19 Hz, 1 H, 5'H) ppm. **$^{13}$C NMR** (125 MHz, $CDCl_3$): $\delta$ = 20.9, 27.1, 30.5, 33.2, 40.8, 56.0 ($OCH_3$), 56.1 ($OCH_3$), 61.8 70.6, 75.7, 78.5, 98.0, 107.7, 111.1, 112.2, 114.4, 126.1, 136.5, 147.9, 148.9, 150.3, 151.2, 153.7, 171.0 ppm. |
| 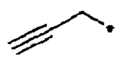 | 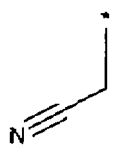 | $-CH_3$ | 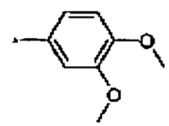 | {8-[(3,4-Dimethoxyphenyl)eth ynyl]-7-methyl-2,6-dioxo-1-prop-2-ynyl-1,2,6,7-tetrahydropurin-3-yl}acetonitrile | MW 403.4, colorless crystals, **m.p.** 265.9 ˚C. **$^1$H NMR** (500 MHz, $CDCl_3$): ($\delta$ = 2.20 (t, J = 2.21 Hz, 1 H, CCH), 3.90 (s, 3H, $OCH_3$), 3.92 (s, 3H, $OCH_3$), 4.08 (s, 3H, N7$CH_3$), 4.77 (d, J = 2.21 Hz, 2H, C$\underline{H_2}$-CCH), 5.00 (s, 2H, $CH_2CN$), 6.87 (d, J = 8.51 Hz, 1H, 6'H), 7.07 (d, J = 1.89 Hz, 1H, 2'H), 7.23 (dd, J = 1.89 and 8.20 Hz, 1H, 5'H) ppm. **$^{13}$C NMR** (125 MHz, $CDCl_3$): $\delta$ = 30.5, 30.8, 33.4, 56.0 ($OCH_3$), 56.1 ($OCH_3$), 71.2, 75.4, 77.9, 98.8, 107.7, 111.2, 111.9, 113.8, 114.4, 126.2, 136.9, 146.0, 148.9, 149.6, 151.4, 153.1 ppm. |

(continued)

| R¹ | R² | R³ | R⁴ | Name | MW/ m.p.,/NMR |
|---|---|---|---|---|---|
| 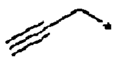 | 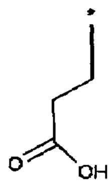 | - $CH_3$ | 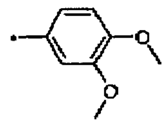 | 3-{8-[(3,4-Dimethoxyphenyl)eth ynyl]-7-methyl-2,6-dioxo-1-prop-2-ynyl-1,26,7-tetrahydropurin-3-yl}propionic acid | MW 436.4, colorless crystals, **m.p.** 261.3 °C. **$^1$H NMR** (500 MHz, DMSO-$d_6$): δ = 2.65 (t, J = 7.88 Hz, 2H), 3.10 (t, J = 1.62 Hz, 1H, CCH), 3.81 (s, 3H, $OCH_3$), 3.82 (s, 3H, $OCH_3$), 4.00 (s, 3H, N7$CH_3$), 4.20 (t, J = 7.88 Hz, 2H), 4.60 (d, J = 1.58 Hz, 2H, C<u>H</u>$_2$-CCH), 7.01 (d, J = 8.20 Hz, 1H, 6'H), 7.21 (s, 1 H, 2'H), 7.23 (dd, J =1.89 and 8.20 Hz, 1H, 5'H), 12.41 (s, 1H, OH) ppm. **$^{13}$C NMR** (125 MHz, DMSO-$d_6$): δ = 30.3, 32.2, 33.2, 39.1, 55.8 ($OCH_3$), 55.9 ($OCH_3$), 73.2, 76.2, 79.5, 97.4, 107.4, 111.5, 112.1, 114.8, 126.0, 135.6, 147.2, 148.9, 149.8, 151.2, 153.1, 172.1 ppm. |
| 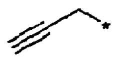 | 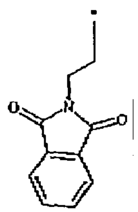 | - $CH_3$ | 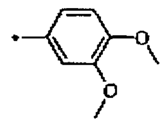 | 8-[(3,4-Dimethoxyphenyl)eth ynyl]-3-[2-(1,3-dioxo-1,3-dihydro-2*H*-isoindol-2-yl)ethyl]-7-methyl-1-prop-2-ynyl-3,7-dihydropurine-2,6-dione | MW 537.5, colorless crystals, m.p. 252.2 °C. **$^1$H NMR** (500 MHz, $CDCl_3$): δ = 1.99 (t, J = 2.52 Hz, 1H, CCH), 3.90 (s, 3H, $OCH_3$), 3.92 (s, 3H, $OCH_3$), 3.99 (s, 3H, N7$CH_3$), 4.09 - 4.12 (m, 2H), 4.43 - 4.47 (m, 2H), 4.62 (d, J = 2.52 Hz, 2H, C<u>H</u>$_2$-CCH), 6.86 (d, J = 8.52 Hz, 1H, 6'H), 7.01 (d, J = 1.89 Hz, 1H, 2'H), 7.16 (dd, J = 1.89 and 8.20 Hz, 1H, 5'H), 7.61 (dd, J = 3.15 and 5.36 Hz, 2H), 7.76 (dd, J = 3.15 and 5.36 Hz, 2H) ppm. **$^{13}$C NMR** (125 MHz, $CDCl_3$): δ = 30.4, 33.2, 36.4, 42.0, 56.0 ($OCH_3$), 56.1 ($OCH_3$), 70.3, 75.5, 78.3, 97.6, 107.8, 111.1, 112.3, 114.4, 123.2, 126.0, 132.1, 133.7, 136.3, 147.7, 148.9, 150.7, 151.1, 153.7, 168.3 ppm. |

(continued)

| $R^1$ | $R^2$ | $R^3$ | $R^4$ | Name | MW/ m.p.,/NMR |
|---|---|---|---|---|---|
| 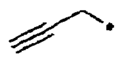 | 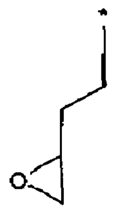 | - $CH_3$ | 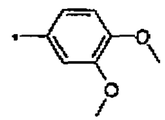 | 8-[(3,4-Dimethoxyphenyl)eth ynyl]-7-methyl-3-(2-oxiran-2-ylethyl)-1-prop-2-ynyl-3,7-dihydropurine-2,6-dione | MW 434.5, colorless crystals, **m.p.** 197.4 ˚C. $^1$H **NMR** (500 MHz, $CDCl_3$): δ = 1.96 - 2.09 (m, 2H), 1.99 (t, J = 2.52 Hz, 1H, CCH), 2.42 (dd, J = 2.52 and 5.04 Hz, 1 H), 2.69 (dd, J = 3.78 and 489 Hz, 1H), 3.01-3.07 (m, 1H), 3.89 (s, 3H, $OCH_3$), 3.91 (s, 3H, $OCH_3$), 4.06 (s, 3H, N7$CH_3$), 4.25 - 4.32 (m, 1 H), 4.32 - 4.40 (m, 1H), 4.78 (d, J = 2.52 Hz, 2H, C<u>H</u>$_2$-CCH), 6.86 (d, J = 8.52 Hz, 1H, 6'H), 7.07 (d, J = 1.89 Hz, 1H, 2'H), 7.22 (dd, J = 1.89 and 8.20 Hz, 1H, 5'H) ppm. **$^{13}$C NMR** (125 MHz, $CDCl_3$): δ = 30.5, 31.2, 33.2, 40.8, 46.4, 50.0, 56.0 ($OCH_3$), 56.1 ($OCH_3$), 70.5, 75.8, 78.5, 98.0, 107.8, 111.1, 112.3, 114.4, 126.1, 136.5, 147.8, 148.9, 150.4, 151.4, 153.8 ppm. |
| 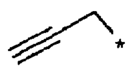 | 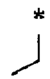 | 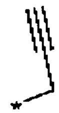 | 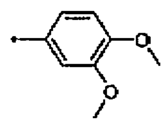 | 8-(3,4-Dimethoxyphenylethy nyl)-3-ethyl-1,7-di-prop-2-ynyl-3,7-dihydropurine-2,6-dione | MW 416.4, colorless crystals, **m.p**. 183.6 ˚C. **$^1$H NMR ($CDCl_3$):** 1.36 (t, J = 7.3 Hz, 3H, CH3), 2.17 (t, J = 2.5 Hz, 1 H), 2.43 (t, J = 2.2 Hz, 1 H), 3.88 (s, 3H, $OCH_3$), 3.91 (s, 3H, $OCH_3$), 4.18 (q, J = 7.3 Hz, 2H), 4.78 (d, J = 2.5 Hz, 2H), 5.27 (d, J = 2.2 Hz, 2H), 6.86 (d, J = 8.6 Hz, 1H), 7.10 (d, J = 1.9 Hz, 1H), 7.25 (dd, J = 1.918.6 Hz, 1H) ppm. **$^{13}$C NMR ($CDCl_3$):** 13.4, 30.5, 35.6, 38.9, 56.01, 56.04, 70.6, 74.0, 75.5, 76.4, 78.5, 98.9, 106.7, 111.2, 112.2, 114.5, 126.2, 136.2, 147.9, 148.9, 150.1, 151.3, 153.5 ppm. |

(continued)

| R¹ | R² | R³ | R⁴ | Name | MW/ m.p.,/NMR |
|---|---|---|---|---|---|
| 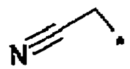 | 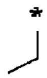 | 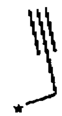 | 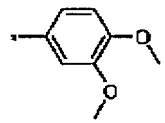 | [8-(3,4-Dimethoxyphenylethy nyl)-3-ethyl-7-prop-2-ynyl-3,7-dihydropurin-2,6-dione-1-yl] acetonitrile | MW 417.4, colorless crystals, m.p. 236.1 °C. **$^1$H NMR ($CDCl_3$):** 1.37 (t, J = 7.25 Hz, 3H, $CH_3$), 2.44 (t, J = 2.55 Hz, 1 H), 3.89 (s, 3H, $OCH_3$), 3.92 (s, 3H, $OCH_3$), 4.19 (q, J = 7.25 Hz, 2H, $CH_2$), 4.89 (s, 2H, $NCH_2CN$), 5.24 (d, J = 2.55 Hz, 2H, $CH_2$) 6.87 (d, J = 8.2 Hz, 1 H), 7.10 (d, J = 1.85 Hz, 1H), 7.26 (dd, J = 1.85/8.2 Hz, 1H) ppm. **$^{13}$C NMR ($CDCl_3$) :** 13.3, 28.4, 29.8, 35.8, 39.2, 56.0, 56.1, 74.3, 75.3, 76.1, 99.5, 106.3, 111.2, 111.9, 114.5, 114.7, 126.3, 136.9, 148.3, 148.9, 149.6, 151.4, 152.7 ppm. |
| 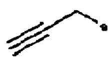 | -$NH_2$ | -H | 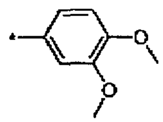 | 3-Amino-8-[(3-methoxyphenyl)ethyn y)]-1-prop-2-ynyl-3,7-dihydropurine-2,6-dione | MW 335.3, colorless crystals, **m.p. ??** °C. **$^1$H NMR** (500 MHz, $CDCl_3$): δ = 3.09 (t, J = 2.5 Hz, 1H, CCH), 3.80 (s, 3H, $OCH_3$), 4.62 (d, J = 2.5 Hz, 2H, C<u>H</u>$_2$CCH), 5.35-5.64 (s br, 2H, $N3NH_2$), 7.08 (ddd, J = 1.2/2.8 and 12.4 Hz, 1 H, 5'H), 7.14 (dd, J = 1.2 and 2.8 Hz, 1 H, 2'H), 7.18 (dt, J = 1.2 and 7.6 Hz, 1H, 6'H), 7.38 (t, J = 7.7 Hz, 1H, 4'H), 14.05-14.34 (s br, 1 H, N7NH) ppm. **$^{13}$C NMR** (125 MHz, $CDCl_3$): δ = 30.7, 55.5 ($OCH_3$), 72.8, 73.1, 79.8, 94.5, 108.0, 116.5, 116.6, 121.4, 124.2, 130.4, 133.0, 150.3, 151.2, 151.5, 159.4 ppm. |
| 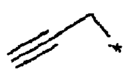 | 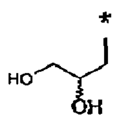 | -$CH_3$ | 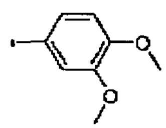 | (R/S)-3-(2,3-Dihydroxypropyl)-8-(3,4-dimethoxyphenytethy nyl)-7-methyl-1-prop-2-ynyl-3,7-dihydropurine-2,6-dione | M 438.4, colorless crystals, **m.p.** 204.6 °C. **$^1$H NMR ($CDCl_3$):** 2.18 (t, J = 2.5 Hz, 1 H), 3.58 (m, 2H, $CH_2$), 3.90 (s, 3H, $OCH_3$), 3.91 (s, 3H, $OCH_3$), 4.07 (s, 3H, $N7CH_3$), 4.08 (m, 1H), 4.33 (ddd, J = 5/15/36 Hz), 4.77 (d, J = 2.5 Hz, 2H), 6.86 (d, J = 9 Hz, 1 H), 7.06 (d, J = 2 Hz, 1 H), 7.22 (dd, J = 2/9 Hz, 1 H) ppm. **$^{13}$C NMR ($CDCl_3$):** 30.8, 33.4, 45.7, 56.0, 56.1, 62.8, 70.3, 70.9, 75.3, 78.1, 98.9, 107.6, 111.2, 111.9, 114.3, 126.2, 136.4, 148.0, 148.9, 151.4, 151.6, 153.4 ppm. |

(continued)

| R[1] | R[2] | R[3] | R[4] | Name | MW/ m.p.,/NMR |
|---|---|---|---|---|---|
| 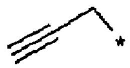 | 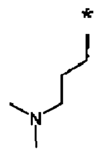 | $-CH_3$ | 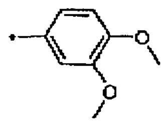 | 8-(3,4-Dimethoxyphenylethy nyl)-3-(3-dimethylaminopropyl) -7-methyl-1-prop-2-ynyl-3,7-dihydropurine-2,6-dione | M = 449.5, colorless crystals, m.p. 186.1 ˚C. **$^1$H NMR ($CDCl_3$):** 2.01 (tt, J = 6.9/7.3, $CH_2$), 2.16 (t, J = 2.6 Hz, 1H), 2.30 (s, 6H, $N(CH_3)_2$), 2.49 (t, J = 6.9 Hz, 2H, $CH_2$), 3.89 (s, 3H, $OCH_3$), 3.91 (s, 3H, $OCH_3$), 4.06 (s, 3H, $N7CH_3$), 4.18 (t, J = 7.3 Hz, 2H, $CH_2$), 4.77 (d, J = 2.6 Hz, 2H), 6.85 (d, J = 8.5 Hz. 1H), 7.07 (d, J = 1.9 Hz, 1 H), 7.22 (dd, J = 1.9/8.5 Hz, 1H) ppm. **$^{13}$C NMR ($CDCl_3$):** 25.6, 30.5, 33.2, 41.8, 45.0, 56.0, 56.1, 56.6, 70.5, 75.8, 78.6, 97.9, 107.8, 111.1, 112.3, 114.4, 126.1, 136.5, 147.9, 148.9, 150.4, 151.1,153.8 ppm. |
| 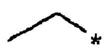 | -H | -H | 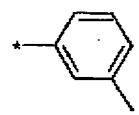 | 1-Ethyl-8-(3-methylphenylethynyl) -3,7-dihydropurine-2,6-dione | colorless solid: **m.p. > 250˚C.** $^1$**H NMR** (DMSO-$d_6$): 1.11 (t , J = 6.95 Hz, 3H, $CH_3$), 2.33 (s, 3H, $ArCH_3$), 3.87 (q, J = 6.95 Hz, 2H), 7.31-7.42 (m, 4H), 11.88 (s, 1 H, NH), 14.05 (s, 1H, NH) ppm. $^{13}$C **NMR (DMSO-$d_6$):** 13.3, 20.8, 35.2, 79.5, 91.7, 107.6, 120.2, 129.0, 129.1, 131.0, 132.2, 133.1, 138.6, 147.0, 1501.8, 154.3 ppm. |
| 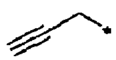 | -H | -H | 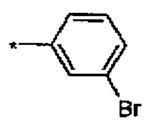 | 8-(3-Bromophenylethynyl) -1-prop-2-ynyl-3,7-dihydropurine-2,6-dione | colorless solid: **m.p.** > 250 ˚C **$^1$H NMR (DMSO-$d_6$):** 3.07 (t, J = 2.2 Hz, 1 H), 4.56 (d, J = 2.2 Hz, 2H), 7.42-7.45 (m, 1H), 7.61-7.63 (m, 1 H), 7.70-7.73 (m, 1 H), 7.80-7.81 (m, 1 H), 12.12 (s, 1H, NH), 14.26 (s, 1H, NH) ppm. $^{13}$C **NMR (DMSO-$d_6$):** 29.7, 72.9, 79.8, 80.8, 90.0, 107.5, 122.0, 122.5, 131.0, 131.2, 133.0, 133.3, 134.0, 147.3, 150.3, 153.6 ppm. |
| 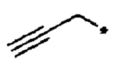 | -H | -H | 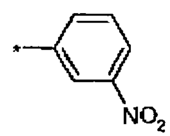 | 8-(3-Nitrophenylethynyl-1-prop-2-ynyl-3,7-dihydropurine-2,6-dione | light grey solid (yield: 95 %): m.p. > 250 ˚C. **$^1$H NMR (DMSO-$d_6$):** 3.08 (t, J = 2.2 Hz, 1 H), 4.56 (d, J = 2.2 Hz, 2H), 7.76-7.79 (m, 1H), 8.03-8.06 (m, 1H), 8.31-8.34 (m, 1 H), 8.36-8.37 (m, 1H), 12.15 (s, 1 H, NH), 14.34 (s, 1H, NH) ppm. **$^{13}$C NMR (DMSO-$d_6$):** 29.7, 72.9, 79.7, 81.4, 89.3, 107.7, 121.8, 124.9, 126.4, 130.9, 132.6, 137.9, 147.2, 148.1, 150.3, 153.7 ppm. |

(continued)

| $R^1$ | $R^2$ | $R^3$ | $R^4$ | Name | MW/ m.p.,/NMR |
|---|---|---|---|---|---|
| 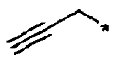 | - $CH_3$ | -H | 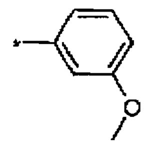 | 8-(3-Methoxyphenylethyn yl)-3-methyl-1-prop-2-ynyl-3,7-dihydropurine-2,6-dione | light yellow solid: **m.p.:** > 250 °C; **$^1$H NMR[1] (DMSO-*d$_6$*):** 3.08 (t, J = 1.6 Hz, 1H), 3.45 (s, 3H), 3.79 (s, 3H), 4.60 (d, J = 1.6 Hz, 2H), 7.08-7.10 (m, 1H), 7.14-7.16 (m, 1H), 7.18-7.20 (m, 1H), 7.37-7.41 (m, 1 H), 14.36 (s, 1 H, NH) ppm. **$^{13}$C NMR (DMSO-*d$_6$*):** 30.0, 30.4, 55.5, 73.0, 79.3, 79.6, 92.0, 107.5, 116.6, 116.9, 121.2, 124.3, 130.4, 133.2, 148.2, 150.4, 152.9, 153.4 ppm. |
| 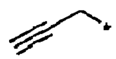 | - $CH_3$ | -H | 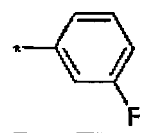 | 8-(3-Fluorophenylethynyl)-3-methyl-1-prop-2-ynyl-3,7- dihydropurine-2,6- dione | colorless solid (yield: 85 %), **m.p.** > 250 °C. **$^1$H NMR (DMSO-*d$_6$*):** 3.08 (t, J = 2.5 Hz, 1H), 3.44 (s, 3H), 4.60 (d, J = 2.5 Hz, 2H), 7.36-7.40 (m, 1H), 7.46-7.55 (m, 3H), 14.4 (s, 1 H, NH) ppm. **$^{13}$C NMR (DMSO-*d$_6$*):** 30.0, 30.4, 73.0, 79.6, 80.2, 90.6, 107.6, 117.7 (d), 118.5 (d), 122.1 (d), 128.4 (d), 131.4 (d), 132.8, 148.1, 150.3, 152.9, 161.9 (d) ppm. |
| 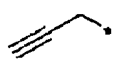 | -$CH_3$ | -H | 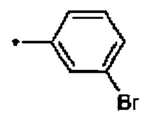 | 8-(3-Bromophenylethynyl) -3-methyl-1-prop-2-ynyl-3,7-dihydropurine-2,6-dione | light yellow solid (yield: 80 %), **m.p.** > 250 °C. **$^1$H NMR (DMSO-*d$_6$*) :** 3.08 (t, J = 2.5 Hz, 1H), 3.44 (s, 3H), 4.60 (d, J = 2.5 Hz, 2H), 7.43-7.46 (m, 1H), 7.62-7.65 (m, 1 H), 7.71-7.73 (m, 1 H), 7.82-7.83 (m, 1H), 14.4 (s, 1H, NH) ppm. **$^{13}$C NMR (DMSO-*d$_6$*)**: 30.0, 30.4, 73.0, 79.6, 80.7, 90.2, 107.7, 122.0, 122.4, 131.0, 131.3, 133.3, 133.4, 134.0, 148.2, 150-4, 153.0 ppm. |
| 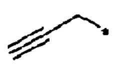 | - $CH_3$ | -H | 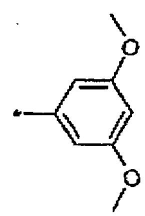 | 8-(3,5-Dimethoxyphenylethy nyl)-3-methyl-1-prop-2-ynyl-3,7-dihydropurine-2,6-dione | colorless solid, **m.p.** > 250 °C °C. **$^1$H NMR (DMSO-*d$_6$*):** 3.08 (t, J = 1.9 Hz, 1H), 3.44 (s, 3H), 3.78 (s, 6H), 4.60 (d, J = 1.9 Hz, 2H), 6.63 (m, 1 H), 6.76 (m, 2H), 14.36 (s, 1H, NH) ppm. **$^{13}$C NMR (DMSO-*d$_6$*):** 29.9, 30.4, 55.7, 73.0, 79.0, 79.6, 92.1, 103.4, 107.4, 109.6, 121.5, 133.2, 148.2, 150.4, 152.8, 160.7 ppm. |

(continued)

| $R^1$ | $R^2$ | $R^3$ | $R^4$ | Name | MW/ m.p.,/NMR |
|---|---|---|---|---|---|
| 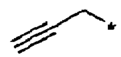 | $-CH_3$ | -H | 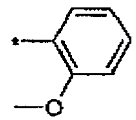 | 8-(2-Methoxyphenylethyn yl)-3-methyl-1-prop-2-ynyl-3,7-dihydropurine-2,6-dione | colorless solid, m.p. > 250 ˚C. **$^1$H NMR (DMSO-$d_6$):** 2.95 (t, J = 2.5 Hz, 1 H), 3.47 (s, 3H), 3.89 (s, 3H), 4.63 (d, J = 2.5 Hz, 2H), 7.00-7.04 (m, 1H), 7.13-7.14 (m, 1 H), 713-7.54 (m, 2H), 14.1 (s, 1H, NH) ppm. **$^{13}$C NMR (DMSO-$d_6$):** 29.6, 30.1, 55.8, 72.3, 79.4, 82.7, 91.9, 109.2, 111.8, 120.6, 131.7, 133.4, 150.2, 160.4 ppm. |
| 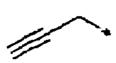 | $-CH_3$ | -H | 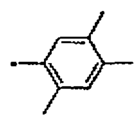 | 3-Methyl-8-(2,4,5-trimethylphenylethyn yl)-1-prop-2-ynyl-3,7-dihydropurine-2,6-dione | colorless solid, m.p. ?? ˚C. **$^1$H NMR (DMSO-$d_6$):** 2.18 (s, 3H), 2.21 (s, 3H), 2.38 (s, 3H), 3.08 (t, J = 2.3 Hz, 1H), 3.43 (s, 3H), 4.60 (d, J = 2.3 Hz, 2H), 7.10 (s, 1 H), 7.31 (s, 1 H), 14.03 (s, 1H, NH) ppm. **$^{13}$C NMR (DMSO-$d_6$):** 18.7, 19.5, 19.6, 29.9, 30.4, 73.0, 79.7, 82.3, 91.7, 107.2, 131.2, 132.8, 133.8, 134.3, 138.0, 139.2, 148.2, 150.4, 152.8 ppm. |
| 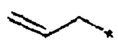 | $- CH_3$ | -H | | 1-Allyl-8-(3-methoxyphenylethyn yl)-3,9-dimethyl-3,7-dihydropurine-2,6-dione | colorless crystals **$^1$H NMR ($CDCl_3$):** 3.76 (s, 3H, $N_9CH_3$)), 3.79 (s, 3H, $OCH_3$), 4.03 (s, $N3CH_3$), 4.61 (d, J = 5.65 Hz, 2H, $NCH_2$), 5.20 (m, 2H), 5.88 (m, 1H), 6.93-6.95 (m, 1H), 7.04 (s, 1 H), 7.11-7.12 (m, 1H), 7.25-7.27 (m, 1H) ppm. **$^{13}$C NMR ($CDCl_3$):** 29.7, 31.4, 34.0, 43.9, 55.4, 77.0, 95.1, 116.4, 116.7, 117.8, 117.9, 121.7, 124.3, 129.7, 131.9, 139.2, 150.9, 156.1, 159.4 ppm. |
| 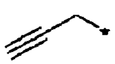 | -H | -H | 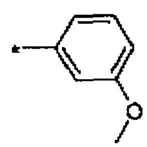 | | MW 320.3, colorless solid, **m.p.** ?? ˚C. **$^1$H NMR (DMSO-$d_6$):** 3.03 (t, J = 2.5 Hz, 1H), 3.80 (s, 3H, $OCH_3$), 4.56 (d, J = 2.5 Hz, 2H), 7.07 (ddd, J = 0.9/ 2.5 and 8.4 Hz, 1H, 5'H), 7.13 (dd, J = 1.3 and 2.5, Hz, 1H, 2'H), 7.18 (d, J = 7.9 Hz, 1H, 6'H), 7.38 (t. J = 7.9 Hz, 1 H, 4'-H), 12.08 (s, 1 H, N7H), 14.16 (s, 1H, N3H) ppm. **$^{13}$C NMR (DMSO-$d_6$):** 29.6, 55.5, 72.8, 79.5, 79.8, 91.6, 116.2, 116.5, 116.9, 121.3, 124.3, 130.4, 133.3, 150.3, 150.8, 153.7, 159.4 ppm. |

(continued)

| $R^1$ | $R^2$ | $R^3$ | $R^4$ | Name | MW/ m.p.,/NMR |
|---|---|---|---|---|---|
| 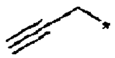 | -H | $-CH_3$ | 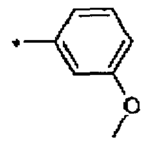 | 8-[(3-Methoxyphenyl)ethyn yl]-7-methy)-1-prop-2-ynyt-3,7-dihydro-1H-purine-2,6-dione | MW 334.3, colorless solid, m.p. 281 ˚C. $^1$**H NMR** (500 MHz, DMSO-$d_6$): δ = 3.07 (t, J = 2.2 Hz, 1 H, CCH), 3.80 (s, 3H, $OCH_3$), 3.98 (s, 3H, $N7CH_3$), 4.56 (d, J = 2.5 Hz, 2H, $CH_2$-CCH), 7.10 (ddd, J = 0.9 / 2.5 and 8.2 Hz, 1H, 5'H), 7.25 (dd, J = 1.3 and 2.5 Hz, 1H, 2H), 7.26 (dt, J = 1.3 and 7.6 Hz, 1H, 6'H), 7.40 (dd, J = 7.5 and 8.2 Hz, 1H, 4'H), 12.13 (s br, 1H, N3H) ppm. $^{13}$**C NMR** (125 MHz, DMSO-$d_6$):δ = 29.5, 33.0, 55.6 ($OCH_3$), 72.9, 77.4, 79.7, 96.0, 107.4, 116.7, 117.1, 121,0, 124.5, 130.4, 135.2, 147.0, 150.2, 153.9, 159.4 ppm. **ESI** +Q1 m/z 335 ($M+H^+$). |
| 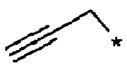 | - H | -H | 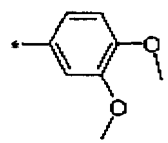 | | MW 350.3, colorless solid, m.p. ?? ˚C. $^1$**H MMR (DMSO-*d₆*):** 3.06 (t, J = 2.5 Hz, 1H), 3.80 (s, 3H, $OCH_3$), 3.81 (s, 3H, $OCH_3$), 4.55 (d, J = 2.5 Hz, 2H), 7.04 (d, J = 8.2 Hz, 1H, 6'H), 7.14 (d, J = 1.9 Hz, 1H, 2'H), 7.20 (dd, J = 1.9 and 8.2 Hz, 1H, 5'H), 12.07 (s, 1 H, N7H), 14.05 (s, 1H, N3H) ppm. $^{13}$**C NMR (DMSO-$d_6$):** 29.6, 55.80, 55.81, 72.8, 78.4, 79.8, 92.6, 107.1, 112.0, 112.2, 114.6, 125.7, 134.0, 147.3, 148.9, 150.4, 150.9, 153.5 ppm. |
| 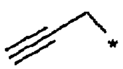 | -H | $-CH_3$ | 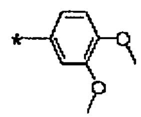 | 8-[(3,4-Dimethoxyphenyl)eth ynyl]-7-methyl-1-prop-2-ynyl-3,7-dihydro-1*H*-purine-2,6-dione | MW 364.4, colorless solid, **m.p.** 279.7 ˚C. $^1$**H NMR** (500 MHz, DMSO-$d_6$): δ = 3.07 (t, J = 2.2 Hz, 1H, CCH), 3.80 (s, 3H, $OCH_3$), 3.81 (s, 3H, $OCH_3$), 3.96 (s, 3H, $N7CH_3$), 4.55 (d, J = 2.2 Hz, 2H, $CH_2$-CCH), 7.05 (d, J = 8.5 Hz, 1 H, 6'H), 7.24 (d, J = 1.9 Hz, 1 H, 2'H), 7.28 (dd, J = 1.9 and 8.2 Hz, 1H, 5'H) ppm. $^{13}$**C NMR** (125 MHz, DMSO-$d_6$): δ = 29.4, 33.0, 55.8 ($OCH_3$), 55.9 ($OCH_3$), 72.9, 76.4, 79.7, 96.9, 107.2, 111.7, 112.1, 114.8, 125.9, 135.7, 147.0, 148.9, 150.2, 151.1, 153.9 ppm. |

Table 2:

| Structure | Human $A_1$ AR (CHO-cells) [3H]CCPA Ki [nM] (Inh. at 10 μM) | Human $A_{2A}$ AR (CHO-cells) [3H]MSX-2 Ki [nM] | Human $A_3$ AR (CHO-cells) [3H]PSB-11 Ki [nM] (Inh, at 10 μM) |
|---|---|---|---|
| 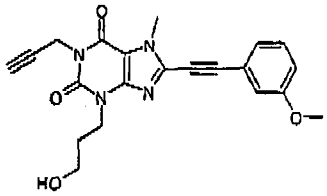 | ≥3000 (38 %) | 5.5 ± 1.4 | |
| 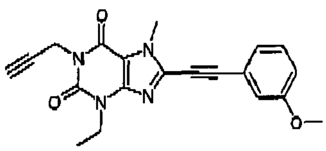 | >> 3000 (6 %) | 64.8 ± 12.0 | |
| 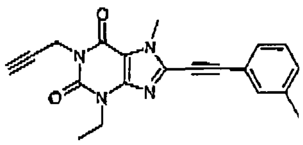 | >> 3000 (3 %) | 89.7 ± 16.1 | |
| 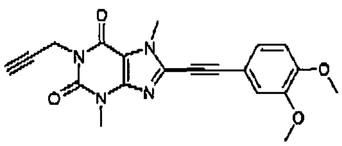 | >> 3000 (2 %) | 36.4 ± 0.6 | |
| 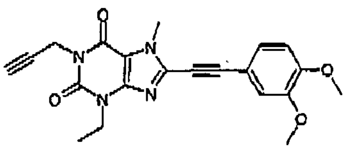 | >> 3000 (5%) | 17.7 ± 0.7 | |

Table 3:

| Structure | Rat $A_1$ AR rat cortex $[^3H]$CCPA $K_i$ [nM] (Inh. at 10 μM) | Rat $A_{2A}$ AR rat striatum $[^3H]$MSX-2 $K_i$ [nM] | Human $A_3$ AR (CHO-cells) $[^3H]$PSB-11 $K_i$ [nM] (Inh. at 10 μM) |
|---|---|---|---|
| 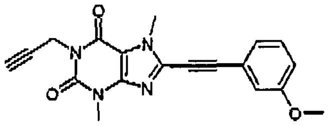 | > 10000 (8%) | 30.9 ± 8.0 | > 10000 (23 %) |
| 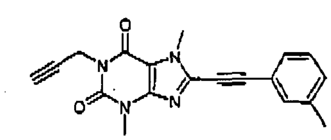 | > 10000 (28 %) | 43.4 ± 11.6 | |

(continued)

| Structure | Rat $A_1$ AR rat cortex [$^3$H]CCPA $K_i$ [nM] (Inh. at 10 $\mu$M) | Rat $A_{2A}$ AR rat striatum [$^3$H]MSX-2 $K_i$ [nM] | Human $A_3$ AR (CHO-cells) [$^3$H]PSB-11 $K_i$ [nM] (Inh. at 10 $\mu$M) |
|---|---|---|---|
| 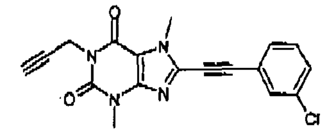 | > 10000 (27%) | 46.0 ± 9.0 | |
| 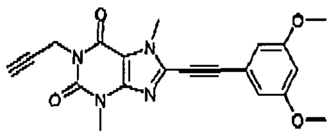 | > 10000 (28%) | 190 ± 50 | > 10000 (8%) |
| 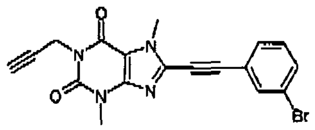 | ≥10000 (37%) | 151 ± 19 | |
| 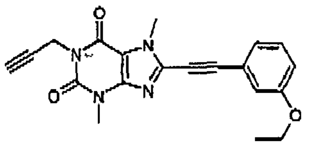 | ca. 10000 (47%) | 174 ± 42 | |
| 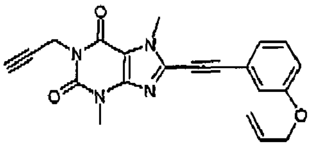 | ≥10000 | 152 ± 63 | |
| 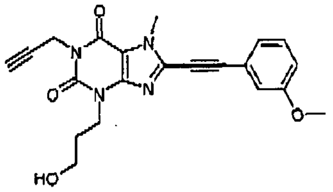 | 142 ± 8 | 11.8 ± 1.64 | |
| 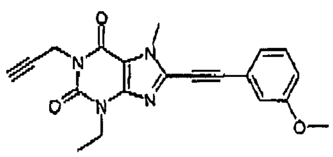 | > 10000 (28%) | 49.9 ± 9.7 | |
| 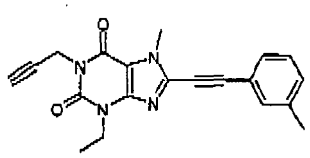 | > 10000 (22 %) | 76.3 ± 17.1 (102 % Inh, at 3 $\mu$M), | |
| 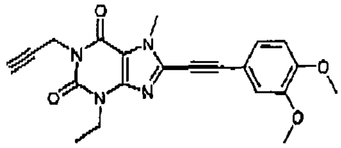 | >> 3000 (-70%) | 49.6 ± 14.3 | |

(continued)

| Structure | Rat $A_1$ AR rat cortex [$^3$H]CCPA $K_i$ [nM] (Inh. at 10 $\mu$M) | Rat $A_{2A}$ AR rat striatum [$^3$H]MSX-2 $K_i$ [nM] | Human $A_3$ AR (CHO-cells) [$^3$H]PSB-11 $K_i$ [nM] (Inh. at 10 $\mu$M) |
|---|---|---|---|
| 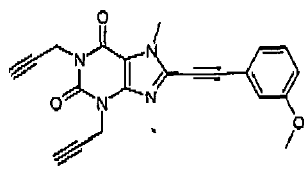 | > 10000 (17%) | 80.7 $\pm$ 21.1 | |
| 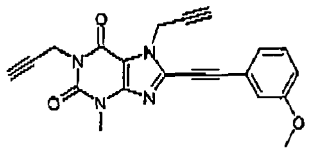 | ≥10000 (37 %) | 90.0 $\pm$ 35.7 | |
| 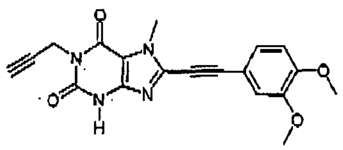 | ≥10000 | 13 | |
| 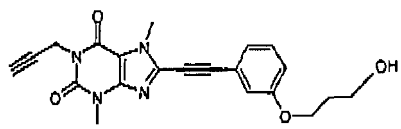 | ≥10000 | 211 | |
| 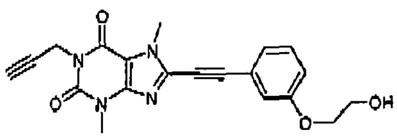 | ≥10000 | 117 | |
| 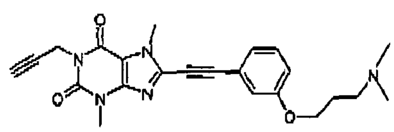 | 186 | 40 | |
| 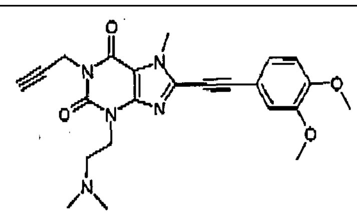 | 810 | 54 | |
| 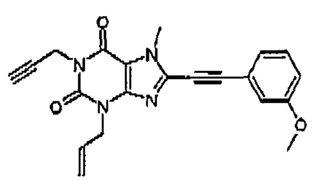 | | 36 | |
| 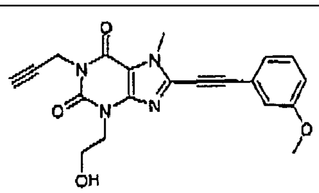 | | 103 $\pm$ 14.9 | |

(continued)

| Structure | Rat $A_1$ AR rat cortex [$^3$H]CCPA $K_i$ [nM] (Inh. at 10 $\mu$M) | Rat $A_{2A}$ AR rat striatum [$^3$H]MSX-2 $K_i$ [nM] | Human $A_3$ AR (CHO-cells) [$^3$H]PSB-11 $K_i$ [nM] (Inh. at 10 $\mu$M) |
|---|---|---|---|
| 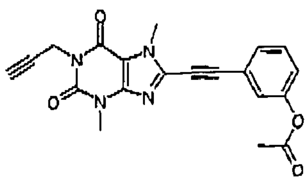 | 1170 $\pm$ 731 | 140 $\pm$ 12 | |
| 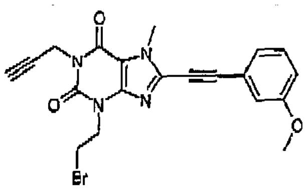 | > 1000 | 90 | |
| 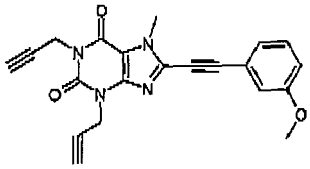 | > 10000 | 81 | |
| 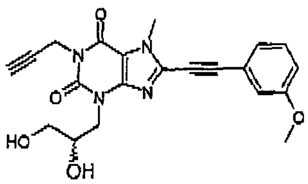 | > 1000 | 70 | |
| 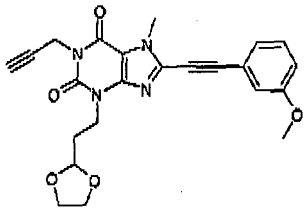 | 48% Inh. at 1 $\mu$M | 120 | |
| 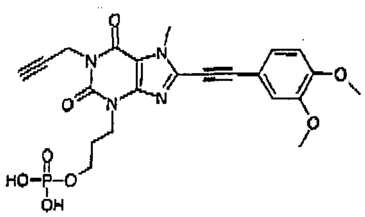 | 136 | 37 | |
| 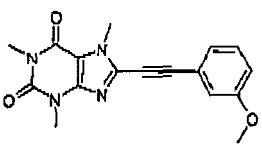 | > 10000 | 185 | |
| 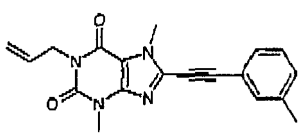 | > 10000 (4%) | 527 $\pm$ 73 | |

(continued)

| Structure | Rat $A_1$ AR rat cortex [$^3$H]CCPA $K_i$ [nM] (Inh. at 10 $\mu$M) | Rat $A_{2A}$ AR rat striatum [$^3$H]MSX-2 $K_i$ [nM] | Human $A_3$ AR (CHO-cells) [$^3$H]PSB-11 $K_i$ [nM] (Inh. at 10 $\mu$M) |
|---|---|---|---|
| 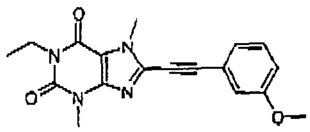 | > 10000 (3%) | 370 ± 56 | |
| 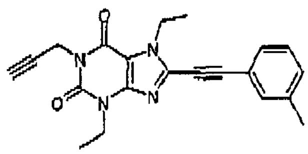 | > 10000 (27 %) | 343 ± 56 | |
| 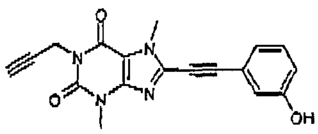 | 425 ± 99 | 173 ± 35 | |
| 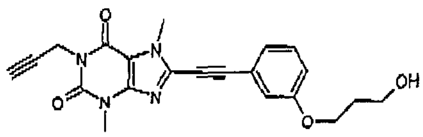 | ≥ 10000 | 211 | |
| 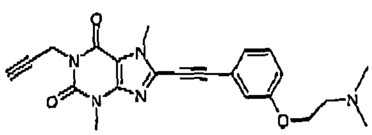 | 194 | 49 | |

**Table 4:**

| Structure | $A_1$ AR | $A_{2A}$ AR | $A_{2B}$ AR | $A_3$ AR |
|---|---|---|---|---|
| 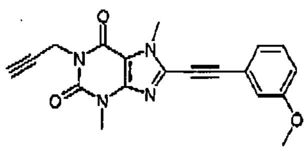 | 5 | 81 | 19 | 24 |
| 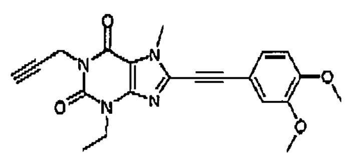 | 4 | 95 | 14 | 38 |
| 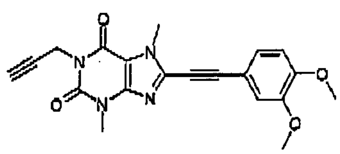 | 10 | 85 | 19 | 16 |

(continued)

| Structure | $A_1$ AR | $A_{2A}$ AR | $A_{2B}$ AR | $A_3$ AR |
|---|---|---|---|---|
| 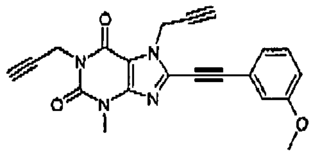 | 24 | 86 | 22 | 37 |
| 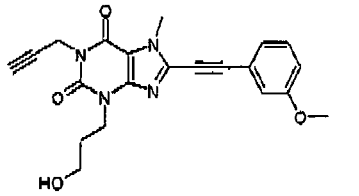 | 41 | 93 | 6 | 37 |
| 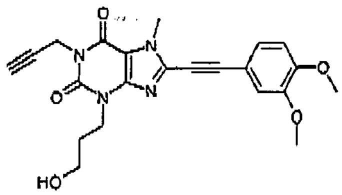 | 34 | 97 | 14 | 36 |
| 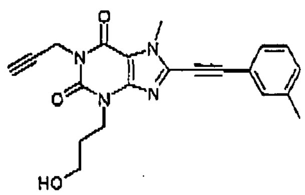 | 43 | 92 | 17 | 54 |

Table 5:

| Structure | Ki [nM] rA2A rat striatum [3H]MSX-2 (n = 3) | Ki [nM] (+100 mM NaCl) rA2A rat striatum [3H] MSX-2 (n = 3) | Sodium Chlorid-Shift Ki (+NaCl)/Ki |
|---|---|---|---|
| 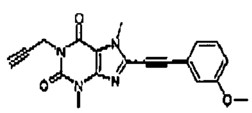 | 30,9 ± 8,0 | 33,2 ± 9,9 | 0,97 ± 0.03 |
| 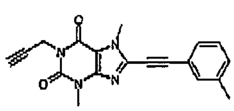 | 43,4 ± 11,6 | 53,1 ± 5,9 | 1,19 ± 0,16 |
| 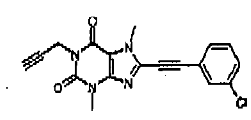 | 46,0 ± 9,0 | 39,5 ± 13,1 | 0,82 ± 0,13 |

**Table 6:**

| Structure | $A_1$ AR $IC_{50}$ μM | $A_1$ AR Imax % | $A_1$ AR Ki [μM] | $A_{2A}$ AR $IC_{50}$ μM | $A_{2A}$ AR Imax% | $A_{2A}$ AR $K_i$ [μM] |
|---|---|---|---|---|---|---|
| 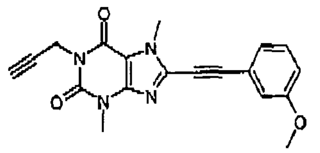 | No significant activity detected | No significant activity detected | No significant activity detected | 1,9 | 90 | 0.178 |
| 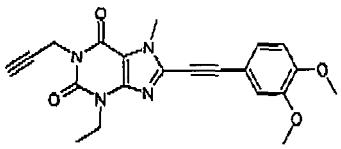 | 3,9 | 49 | 0.854 | 0,2 | 97 | 0.023 |
| 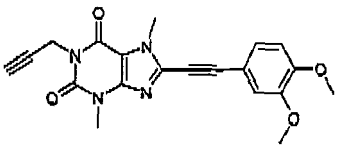 | No significant activity detected | No significant activity detected | No significant activity detected | 0,6 | 95 | 0.058 |
| 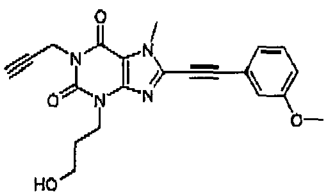 | 1,7 | 90 | 0.370 | 0,9 | 117 | 0.082 |
| 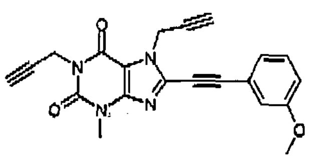 | 2,3 | 55 | 0.501 | 0,5 | 86 | 0.047 |
| 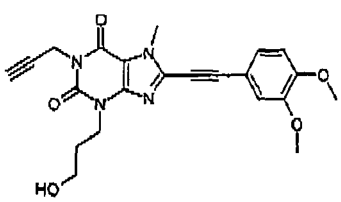 | 1,8 | 92 | 0.401 | 0,3 | 117 | 0.026 |
| 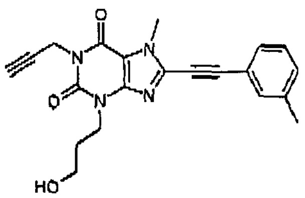 | 1,3 | 102 | 0.289 | 0,4 | 109 | 0.040 |
| CPX ($A_1$ receptor antagonist) | 14 nM | 100 | 3.1 | | | |
| 5-Amino-7-(β-phenylethyl)-2-(8-furyl)pyrazolo(4,3-e)-1,2,4-triazolo(1,5-c)pyrimidme ($A_{2A}$ receptor antagonist) | | | | 6,7 nM | 100 | 0.6 |

**Table 7:**

| FOB Method | Parameter | Recorded as | |
|---|---|---|---|
| Home cage | Body posture | Score | - Normal<br>- Sleeping<br>- Hunchback, relieving posture<br>- Lying on side<br>- Flat body posture |
| Handling reaction | Handling reaction | Score | - Easy<br>- Difficult<br>- Freezing |
| Body temperature | Rectal temperature | Temperature | ˚C |
| Viewing jar | Palpebral closure | Score | - Open<br>- Half / ¾ shut<br>- Shut |
| | Lacrimation | Score | - none<br>- moisture around eyes<br>- moisture flows from eyes |
| | Salivation | Score | - None - Moisture around mouth - Moisture flows from mouth |
| | Rearing | Number | N |
| | Scratching (non-stereotype) | Number | N |
| | Jumps | Number | N |
| | Touch reactivity | Score | - Normal (head turning or no interest) Retreat, twitch<br>- Freezing<br>- Biting, attack |
| | Fear reaction | Score | - Approach<br>- None<br>- Retreat, twitch<br>- Freezing<br>- Biting, attack |
| | Startle response | Score | - Twitch<br>- Jump<br>- None |
| Walking alley | Crossings | Number | N |
| | Akinesia | Occurrence | No / yes |
| | Gait characterization | Score | -Normal<br>- Staggering<br>-Atactic<br>- Retraction of hindlimbs<br>- Sneaking<br>-n/a |
| Inclined Plane | Righting reflex | Direction | - Upstairs<br>- Downstairs<br>- None |
| | Leaving | Time | s |
| Bar test | Catalepsy | Time | s |

(continued)

| FOB Method | Parameter | Recorded as | |
|---|---|---|---|
| Hand-held tests | Muscle tone | Score | - Normal<br>- Soft<br>- Hard |
| | Lid closing reflex (right/left eye) | Occurrence | Yes / no |
| | Struggle | Occurrence | Yes / no |
| Tail flick test | Analgesia | Time | s |
| Grip-strength test | Grip-strength - fore paws | Score | - Grasp and pull<br>- Grasp without pull<br>- No grasp |
| | Grip-strength - hind paws | Score | - Grasp and pull<br>- Grasp without pull<br>- no grasp |
| Throughout the test | Piloerection | Occurrence | No / yes |
| | Stereotypies<br>- Licking<br>- Scanning<br>- Scratching<br>- Chewing | Occurrence | No / yes |
| | Tremor | Occurrence | No / yes |
| | Convulsion | Occurrence | No / yes |
| | Head twitches / Wet dog shakes | Occurrence | No / yes |
| | Fore paw treading | Occurrence | No / yes |
| | Flat body posture | Occurrence | No / yes |
| | Diarrhoea | Occurrence | No / yes |
| | Vocalization<br>- Spontaneously<br>- During touching | Occurrence | No / yes |
| | Trembling | Occurrence | No / yes |
| | Hind leg abduction | Occurrence | No / yes |
| | Straub tail | Occurrence | No / yes |
| | Sneezing | Occurrence | No / yes |
| | Coughing | Occurrence | No / yes |
| | Death | Occurrence | No / yes |
| | Respiration | Score | - Normal<br>- Slow<br>- Slow and flat<br>- Slow and intermittent<br>- Fast<br>- Fast and flat |
| | Remarkable miscellaneous behaviour | Type, number and situation | |

**Table 8:**

| SAC Method | Parameter | Recorded as | |
|---|---|---|---|
| Home cage | Body posture | Score | - Normal<br>- Sleeping<br>- Hunchback, relieving posture<br>- Lying on side<br>- Flat body posture |
| Throughout the test | Piloerection | Occurrence | No / yes |
| | Stereotypies<br>- Licking<br>- Scanning<br>- Scratching<br>- Chewing | Occurrence | No / yes |
| | Tremor | Occurrence | No / yes |
| | Convulsion | Occurrence | No / yes |
| | Head twitches /<br>Wet dog shakes | Occurrence | No / yes |
| | Fore paw treading | Occurrence | No / yes |
| | Flat body posture | Occurrence | No / yes |
| | Diarrhoea | Occurrence | No / yes |
| | Vocalization<br>- Spontaneously<br>- During touching | Occurrence | No / yes |
| | Trembling | Occurrence | No / yes |
| | Hind leg abduction | Occurrence | No / yes |
| | Straub tail | Occurrence | No / yes |
| | Sneezing | Occurrence | No / yes |
| | Coughing | Occurrence | No / yes |
| | Death | Occurrence | No / yes |
| | Remarkable miscellaneous behaviour | Type, number and situation | |
| Bar test | Catalepsy | Time | s |

**Table 9:**

| FU Method | Parameter | Recorded as | |
|---|---|---|---|
| Home cage | Body posture | Score | - Normal<br>- Sleeping<br>- Hunchback, relieving posture<br>- Lying on side<br>- Flat body posture |

(continued)

| FU Method | Parameter | Recorded as | |
|---|---|---|---|
| Handling reaction | Handling reaction | Score | - Easy<br>- Difficult<br>- Freezing |
| Bar test | Catalepsy | Time | s |
| Body weight | Body weight | Weight | g |
| Body temperature | Rectal temperature | Temperature | ˚C |
| Throughout the test | Piloerection | Occurrence | No / yes |
| | Stereotypies<br>- Licking<br>- Scanning<br>- Scratching<br>- Chewing | Occurrence | No / yes |
| | Tremor | Occurrence | No / yes |
| | Convulsion | Occurrence | No / yes |
| | Head twitches / Wet dog shakes | Occurrence | No / yes |
| | Fore paw treading | Occurrence | No / yes |
| | Flat body posture | Occurrence | No / yes |
| | Diarrhoea | Occurrence | No / yes |
| | Vocalization<br>- Spontaneously<br>- During touching | Occurrence | No / yes |
| | Trembling | Occurrence | No / yes |
| | Hind leg abduction | Occurrence | No / yes |
| | Straub tail | Occurrence | No / yes |
| | Sneezing | Occurrence | No / yes |
| | Coughing | Occurrence | No / yes |
| | Death | Occurrence | No / yes |
| | Respiration | Score | -Normal<br>- Slaw<br>- Slow and flat<br>- Slow and intermittent<br>- Fast<br>- Fast and flat |
| | Remarkable miscellaneous behaviour | Type, number and situation | |

Table 10:

| Structure / Patterns | 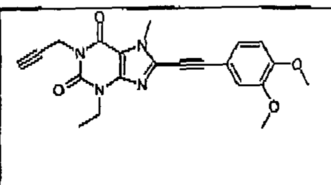 | 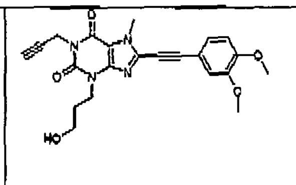 | 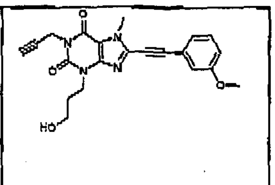 |
|---|---|---|---|
| Activity | +++ | ++ | + |
| Motor Coordination | 0 | - | 0 |
| Catalepsy | 0 | 0 | 0 |
| Hypersensitivity | ++ | 0 | + |
| Temperature | + | - | 0 |
| Respiration | +++ | +++ | ++ |
| Tremor | 0 | + | 0 |
| Stereotypies | ++ | + | 0 |

+++ = very strong effect, ++ = strong effect, + = average effect, 0 = no effect, - = negative effect

## Claims

1. A compound represented by formula (I),

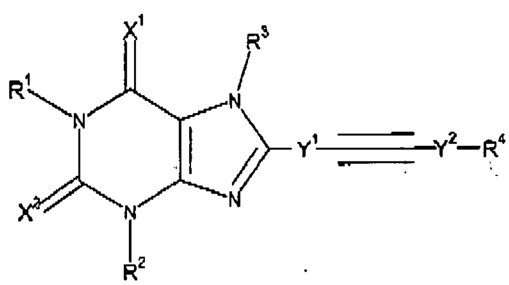

wherein,

$X^1$, $X^2$ are independently S, O or $(C_1\text{-}C_6)$-alkyl,
$Y^1$, $Y^2$ are independently a direct bond or $(C_1\text{-}C_3)$-alkyl,
$R^1$ and $R^3$ are each independently hydrogen, optionally substituted heterocyclyl with 3 to 18 ring atoms, optionally substituted aryl, optionally substituted heteroaryl with 5 to 18 ring atoms, -C(O)$R^5$, -C(O)O$R^5$ , -O$R^5$, -OC(O)$R^5$, or
$(C_1\text{-}C_{10})$-alkyl, $(C_2\text{-}C_{10})$-alkenyl, $(C_2\text{-}C_{10})$-alkinyl, $(C_3\text{-}C_{18})$-cycloalkyl, each of which can optionally be substituted in one or more places, in the same way or differently, with halogen, hydroxyl, cyano, amino, nitro, optionally substituted $(C_3\text{-}C_{18})$-cycloalkyl, optionally substituted heterocyclyl with 3 to 18 ring atoms, optionally substituted aryl, optionally substituted heteroaryl with 5 to 18 ring atoms, -OC(O)$R^5$ or -C(O)$R^5$, -O$R^5$,
$R^2$ hydrogen, optionally substituted heterocyclyl with 3 to 18 ring atoms, optionally substituted aryl, optionally

substituted heteroaryl with 5 to 18 ring atoms, $-NR^6R^7$, $-OR^5$, $-C(O)R^5$, $-C(O)OR^8$,
or
$(C_1-C_{10})$-alkyl, $(C_2-C_{10})$-alkenyl, $(C_2-C_{10})$-alkinyl, $(C_3-C_{18})$-cycloalkyl, each of which can optionally be substituted in one or more places, in the same way or differently with halogen, hydroxyl, cyano, nitro, optionally substituted $(C_3-C_{18})$-cycloalkyl, optionally substituted heterocyclyl with 3 to 18 ring atoms, optionally substituted aryl, optionally substituted heteroaryl with 5 to 18 ring atoms, $-NR^6R^7$, $-OR^8$, $-C(O)R^5$ or $-C(O)OR^8$,
$R^4$ is an optionally substituted heterocyclyl with 3 to 18 ring atoms, optionally substituted $(C_3-C_{18})$-cycloalkyl or optionally substituted heteroaryl with 5 to 18 ring atoms,
or
an aryl optionally substituted with one or more groups selected from the group consisting of halogen, amino, nitro, cyano, $-OR^9$,$-C(O)R^9$, $-OC(O)R^8$, optionally substituted heterocyclyl with 3 to 18 ring atoms, optionally substituted $(C_3-C_{18})$-cycloalkyl, optionally substituted aryl or optionally substituted heteroaryl with 5 to 18 ring at oms,
or
an aryl optionally substituted with $(C_1-C_{10})$-alkyl, $(C_2-C_{10})$-alkenyl, $(C_2-C_{10})$-alkinyl, $(C_3-C_{18})$-cycloalkyl, each of which can optionally be further substituted in one or more places, in the same way or differ ently with halogen, hydroxy, cyano, amino, nitro, $(C_1-C_8)$alkyl, $(C_1-C_6)$-alkoxy, $(C_1-C_6)$-alkoxy-$(C_1-C_6)$-alkoxy, heterocyclyl with 3 to 18 ring atoms, $(C_3-C_{18})$-cycloalkyl, aryl, a heteroaryl with 5 to 18 ring atoms, $-C(O)R^5$, or $-OC(O)R^5$,
with the proviso, that
if the aryl is a phenyl, the phenyl is at least once substituted with a group other than hydrogen,
$R^5$ is a hydrogen, $(C_1-C_6)$-alkyl, $(C_2-C_6)$-alkenyl, $(C_2-C_6)$-alkinyl,
or
$(C_1-C_6)$-alkyl substituted in one or more places, in the same way or differently, with hydroxyl, $(C_1-C_6)$-alkoxy, aryl or $-NR^6R^7$,
$R^6$ and $R^7$ are independently hydrogen, $(C_1-C_6)$-alkyl, $(C_2-C_6)$-alkenyl or $(C_2-C_6)$-alkinyl,
$R^8$ hydrogen, $(C_1-C_{10})$-alkyl, $(C_2-C_{10})$-alkenyl, $(C_2-C_{10})$-alkinyl,
or
$(C_1-C_6)$-alkyl substituted in one or more places, in the same way or differently, with hydroxyl, $(C_1-C_6)$-alkoxy, aryl or $-NR^6R^7$,
or
optionally substituted $(C_3-C_{18})$-cycloalkyl, optionally substituted heterocyclyl with 3 to 18 ring atoms, optionally substituted aryl, optionally substituted heteroaryl with 5 to 18 ring atoms or carbohydrate,
or
formyl, optionally substituted $(C_1-C_{10})$-alkylcarbonyl, optionally substituted $(C_3-C_{18})$-cycloalkylcarbonyl, optionally substituted arylcarbonyl, optionally substituted heteroarylcarbonyl in which the heteroaryl part has 5 to 18 ring atoms, optionally substituted heterocyclylcarbonyl in which the heterocyclyl part has 3 to 18 ring atoms, hydroxycarbonyl, hydroxyl-$(C_1-C_6)$-alkylcarbonyl, $(C_1-C_{10})$-alkoxycarbonyl, optionally substituted aryloxycarbonyl, benzoylacyl, benzoylglycyl, optionally substituted amino acid residue,
or
is selected from the group

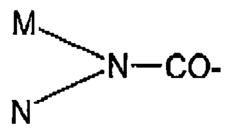
or
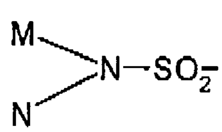

$R^9$ wherein M and N independently represent hydrogen, $(C_1-C_{10})$-alkyl, optionally substituted aryl, or phenoxy$(C_1-C_6)$-alkyl and wherein M and N may form a ring together with the amine nitrogen,
or
an ester moiety of inorganic acids,
or
an ester moiety of ascorbic acid,
or
$-SiR_k,R_j,R_u$, wherein $R_k$, $R_j$, $R_u$ are independently selected from $(C_1-C_6)$-alkyl or aryl,
hydrogen, $(C_1-C_{10})$-alkyl, $(C_2-C_{10})$-alkenyl, $(C_2-C_{10})$-alkinyl,
or

($C_1$-$C_6$)-alkyl substituted in one or more places, in the same way or differently, with hydroxyl, ($C_1$-$C_6$)-alkoxy or $-NR^6R^7$,

or

optionally substituted ($C_3$-$C_{18}$)-cycloalkyl, optionally substituted heterocyclyl with 3 to 18 ring atoms or carbohydrate,

or

formyl, optionally substituted ($C_1$-$C_{10}$)-alkylcarbonyl, optionally substituted ($C_3$-$C_{18}$)-cycloalkylcarbonyl, optionally substituted heterocyclylcarbonyl in which the heterocyclyl part has 3 to 18 ring atoms, hydroxycarbonyl, hydroxyl-($C_1$-$C_6$)-alkylcarbonyl, ($C_1$-$C_{10}$)-alkoxycarbonyl or an optionally substituted amino acid residue,

or

is selected from the group

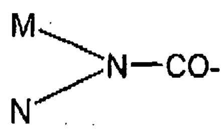

or

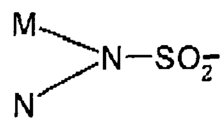

wherein M and N independently represent hydrogen or ($C_1$-$C_{10}$)-alkyl, and wherein M and N may form a non-aromatic ring together with the amine nitrogen,

or

an ester moiety of inorganic acids,

or

an ester moiety of ascorbic acid,

or

$-SiR_k,R_j,R_u$, wherein $R_k$, $R_j$, $R_u$ are independently selected from ($C_1$-$C_6$)-alkyl or aryl,

or pharmaceutically acceptable salts, isomers, diastereomers or enantiomers thereof.

**2.** A compound represented by formula (I) according to claim 1, wherein

$Y^1$, $Y^2$ are independently a direct bond,

or pharmaceutical acceptable salts, isomers, diastereomers or enantiomers thereof.

**3.** A compound represented by formula (I) according to claim 1 or 2, wherein

$X^1$, $X^2$ are independently O,

or pharmaceutically acceptable salts, isomers, diastereomers or enantiomers.

**4.** A compound represented by formula (I) according to one of the preceding claims, wherein

$R^4$ is heterocyclyl with 3 to 7 ring atoms or a heteroaryl with 5 to 10 ring atoms,

or

an aryl optionally substituted with one or more groups selected from the group consisting of halogen, amino, nitro, cyano, $-OR^5$, $-C(O)R^6$ $-OC(O)R^6$, ($C_3$-$C_6$)-cycloalkyl, aryl, heterocyclyl with 3 to 7 ring atoms or a heteroaryl with 5 to 10 ring atoms

or

an aryl optionally substituted with ($C_1$-$C_8$)-alkyl, ($C_2$-$C_8$)-alkenyl, ($C_2$-$C_8$)-alkinyl, ($C_3$-$C_6$)-cycloalkyl, each of which can optionally be further substituted in one or more places, in the same way or differently with halogen, hydroxy, cyano, amino, nitro, ($C_1$-$C_8$)-alkyl, ($C_1$-$C_6$)-alkoxy, ($C_1$-$C_6$)-alkoxy-($C_1$-$C_6$)-alkoxy, ($C_3$-$C_6$)-cycloalkyl, heterocyclyl with 3 to 7 ring atoms, aryl, $-C(O)R^6$, or $-OC(O)R^6$,

$R^5$ is a hydrogen, $(C_1\text{-}C_6)$-alkyl, $(C_2\text{-}C_6)$-alkenyl, $(C_2\text{-}C_6)$-alkinyl,
or
$(C_1\text{-}C_6)$-alkyl substituted in one or more places, in the same way or differently, with hydroxyl, aryl or $-NR^6R^7$,
$R^6$ and $R^7$ are independently hydrogen or a $(C_1\text{-}C_6)$-alkyl group,

or pharmaceutically acceptable salts, isomers, diastereomers or enantiomers thereof.

**5.** A compound represented by formula (I) according to one of the preceding claims, wherein

$R^1$ and $R^3$ are each independently hydrogen,
or
$(C_1\text{-}C_8)$-alkyl, $(C_2\text{-}C_8)$-alkenyl, $(C_2\text{-}C_8)$-alkinyl, $(C_3\text{-}C_6)$-cycloalkyl, each of which can optionally be substituted in one or more places, in the same way or differently, with halogen, hydroxyl, cyano, amino, nitro, $(C_3\text{-}C_6)$-cycloalkyl, heterocyclyl with 3 to 7 ring atoms, aryl, heteroaryl with 5 to 10 ring atoms, $-OC(O)R^6$ or $-C(O)R^6$,

or pharmaceutically acceptable salts, isomers, diastereomers or enantiomers thereof.

**6.** A compound represented by formula (I) according to one of the preceding claims, wherein

$R^2$ is a hydrogen, amino, $(C_1\text{-}C_6)$-alkyl-N-, $(C_1\text{-}C_6)$-alkyl-C(O)O-,
or
$(C_1\text{-}C_6)$-alkyl, $(C_2\text{-}C_8)$-alkenyl, $(C_2\text{-}C_8)$-alkinyl, $(C_3\text{-}C_6)$-cycloalkyl, each of which can optionally be substituted in one or more places, in the same way or differently with hydroxyl, halogen, cyano, nitro, -CO(OH), -C(O)H, $(C_3\text{-}C_6)$-cycloalkyl, heterocyclyl with 3 to 7 ring atoms, aryl, heteroaryl with 5 to 10 ring atoms, $-NR^6R^7$, or $-OR^8$,
$R^8$ hydrogen, $(C_1\text{-}C_6)$-alkyl, $(C_1\text{-}C_6)$-alkoxy-$(C_1\text{-}C_6)$-alkyl, $(C_3\text{-}C_6)$-cycloalkyl, benzyl, allyl or carbohydrate,
or
formyl, $(C_1\text{-}C_6)$-alkylcarbonyl, $(C_3\text{-}C_6)$-cycloalkylcarbonyl, arylcarbonyl,
or
hydroxycarbonyl, $(C_1\text{-}C_6)$-alkoxycarbonyl, aryloxycarbonyl, benzoylacyl, benzoylglycyl, amino acid residue, or is selected from the group

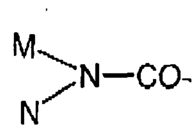

or

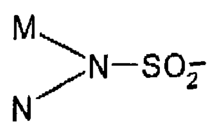

wherein M and N independently represent hydrogen, $(C_1\text{-}C_6)$-alkyl, aryl, benzyl, or phenoxy$(C_1\text{-}C_6)$-alkyl and wherein M and N may form a ring from 3 to 6 atoms together with the amine nitrogen,
or
an ester moiety of inorganic acids,
or
an ester moiety of ascorbic acid,
or
$-SiR_k,R_j,R_u$, wherein $R_k$, $R_j$, $R_u$ are independently selected from $(C_1\text{-}C_4)$-alkyl or aryl,

or pharmaceutically acceptable salts, isomers, diastereomers or enantiomers thereof.

**7.** A compound represented by formula (I) according to one of the preceding claims, wherein

$R^1$ is hydrogen, $(C_1\text{-}C_6)$-alkyl, allyl, propargyl, or a $(C_1\text{-}C_6)$-alkyl substituted with one or more cyano or $(C_3\text{-}C_6)$-cycloalkyl groups,
$R^2$ is a hydrogen or an allyl, propargyl, amino, $(C_1\text{-}C_4)$-alkyl-C(O)O-,
or

a $(C_1-C_6)$-alkyl-group optionally substituted with one or more groups selected from the group consisting of hydroxyl, halogen, cyano, $-NR^6R^7$, -C(O)OH, -C(O)H, $(C_1-C_3)$-alkoxy, $(C_1-C_3)$-alkoxy-$(C_1-C_3)$-alkoxy, $(C_1-C_3)$-alkyl-C(O)O-, a heterocyclyl with 3 to 6 ring atoms, phosphate ester, or carbohydrate ester,
$R^3$ is a hydrogen, $(C_1-C_8)$-alkyl, propargyl,
or
a $(C_1-C_3)$-alkyl group substituted with one or more groups selected from the group consisting of hydroxyl, halogen, cyano, phenyl, $(C_3-C_6)$-cycloalkyl, $-OC(O)R^6$ and $-C(O)R^6$,
$R^4$ is a heterocyclyl with 5 ring atoms selected from the group consisting of furyl, thienyl, pyrrolyl, pyrrolinyl, pyrrolidinyl, dioxolanyl, oxazolyl, thiazolyl, imidazolyl, imidazolinyl, pyrazolyl, pyrazolinyl, pyrazolidinyl, isoxazolyl, isothiazolyl, oxadiazolyl, triazolyl and thiadia zolyl,
or
a phenyl substituted with $R^a$ and $R^b$, wherein $R^a$ and $R^b$ are connected to adjacent carbonate atoms of the phenyl ring and form together a carbocyclic or heterocyclic ring from 3 to 6 atoms,
or
an aryl optionally substituted with one or more groups selected from the group consisting of $(C_1-C_4)$-alkyl, halogenyl, nitro, amino, trifluormethyl, $-OR^5$ and $-OC(O)R^6$,
$R^5$ is a hydrogen, $(C_1-C_4)$-alkyl, phenyl-$(C_1-C_3)$-alkyl, hydroxyl-$(C_1-C_4)$-alkyl, $R^6R^7$-N-$(C_1-C_4)$-alkyl, or allyl,
$R^6$ and $R^7$ are independently hydrogen or a $(C_1-C_3)$-alkyl group,

or pharmaceutically acceptable salts, isomers, diastereomers or enantiomers thereof.

**8.** A compound represented by formula (1) according to claim 7,
wherein

$R^1$ is a $(C_1-C_4)$-alkyl, cyanomethyl, allyl, propargyl or a cyclobutyl methyl group,
$R^2$ is hydrogen or a methyl, ethyl, allyl, amino, bromethyl, cyano-$(C_2-C_3)$-alkyl, propargyl, butyl-C(O)O-, butyl-C(O)O-methyl-, methyl-C(O)O-propyl-, $R^6R^7N$-$(C_2-C_3)$-alkyl-, epoxide-ethyl, 1,3-dioxo-1,3-dihydro-2H-isoindol-ethyl, dioxolane-propyl, morpholine-ethyl, $(OH)_2OP(O)$-propyl, H-C(O)-ethyl, HO-C(O)-ethyl, hydroxyl-$(C_2-C_3)$-alkyl, dihydroxypropyl, glucosyl-O-propyl, methoxyethoxy-ethyl-, or a trihydroxypentyl-group,
$R^3$ is hydrogen or a $(C_1-C_5)$-alkyl, hydroxyethyl, propargyl, methyl-C(O)-methyl, phenyl-methyl, cyclobutylmethyl, or butyl-C(O)O-methyl-group,
$R^4$ is a thiophene or benzodioxole,
or
a phenyl substituted with one or more groups selected from the group consisting of methyl, halogen, nitro, amino, trifluormethyl-, $-OC(O)CH_3$ and $-OR^5$,
$R^5$ is a hydrogen, methyl, ethyl, hydroxyl-$(C_2-C_3)$-alkyl, $(CH_3)_2$-N-$(C_2-C_3)$-alkyl, phenylmethyl or allyl,
$R^6$ and $R^7$ are independently hydrogen or a methyl group,

or pharmaceutically acceptable salts, isomers, diastereomers or enantiomers thereof.

**9.** A compound represented by formula (I) according to claim 8,
wherein

$R^2$ is a hydrogen or an amino, methyl, ethyl, allyl, bromethyl, cyanomethyl, propargyl, butyl-C(O)O-, butyl-C(O)O-methyl, methyl-C(O)O-propyl-, $(CH_3)_2$-N-$(C_2-C_3)$-alkyl, epoxide-ethyl, 3-dioxo-1,3-dihydro-2H-isoindol-ethyl, dioxolane-propyl, morpholine-ethyl, $(OH)_2$ OP(O)-propyl, H-C(O)-ethyl, HO-C(O)-ethyl, hydroxyethyl, hydroxypropyl, or a dihydroxypropyl group,
$R^4$ is a benzodioxole,
or
a phenyl substituted with one or more groups selected from the group consisting of methyl-, halogen, nitro, amino, trifluormethyl, $-OC(O)CH_3$ and $-OR^5$,

or pharmaceutically acceptable salts, isomers, diastereomers or enantiomers thereof.

**10.** A compound represented by formula (I) according to one of the preceding claims,
wherein

$R^2$ is a hydrogen, methyl, ethyl, allyl, bromethyl, propargyl, hydroxyethyl, hydroxypropyl, dihydroxypropyl,

$(CH_3)_2$-N-ethyl, $(OH)_2OP(O)$-propyl, or a dioxolane-propyl group

or pharmaceutically acceptable salts, isomers, diastereomers or enantiomers thereof.

**11.** A compound represented by formula (I) according to claim 9 or 10, wherein

$R^1$ is a ethyl, allyl or propargyl group,
$R^3$ is a methyl, ethyl or progargyl group,
$R^4$ is phenyl substituted with one or more groups selected from the group consisting of methyl, halogen, -OC(O)$CH_3$ and -$OR^5$,
$R^5$ is a hydrogen, methyl, ethyl, allyl, hydroxyl-($C_2$-$C_3$)-alkyl, or $(CH_3)_2$-N-($C_2$-$C_3$)-alkyl,

or pharmaceutically acceptable salts, isomers, diastereomers or enantiomers thereof.

**12.** A compound represented by formula (I) according to claim 11, wherein

$R^1$ is a propargyl group,
$R^2$ is a hydrogen, methyl, ethyl, allyl, propargyl, hydroxyethyl or hydroxypropyl group,
$R^3$ is a methyl or progargyl group,
$R^5$ is a methyl, ethyl, allyl, or hydroxylethyl,

or pharmaceutically acceptable salts, isomers, diastereomers or enantiomers thereof.

**13.** A compound represented by formula (I) according to one of the claims 1 to 10, wherein

$R^1$ is a propargyl or methyl group

or pharmaceutically acceptable salts, isomers, diastereomers or enantiomers thereof.

**14.** A compound represented by formula (I) according to one of the preceding claims, wherein

$R^2$ is hydrogen, methyl, ethyl, or allyl,

or pharmaceutically acceptable salts, isomers, diastereomers or enantiomers thereof.

**15.** A compound represented by formula (I) according to one of the preceding claims, wherein

$R^3$ is a methyl or propargyl group,

or pharmaceutically acceptable salts, isomers, diastereomers or enantiomers thereof.

**16.** A compound represented by formula (I) according to one of the preceding claims, wherein

$R^4$ is phenyl substituted with one or more groups selected from the group consisting of methyl, and methoxy,

or pharmaceutically acceptable salts, isomers, diastereomers or enantiomers thereof.

**17.** A compound represented by formula (I) according to one of the claims 9 to 13 or claim 15, wherein

$R^1$ is a propargyl group,
$R^2$ is a methyl, ethyl or hydroxypropyl group,

$R^4$ is a phenyl substituted with one or more methoxy groups or a meta-methylphenyl group,

or pharmaceutically acceptable salts, isomers, diastereomers or enantiomers thereof.

**18.** A compound represented by formula (I) according to claim 17, wherein

$R^1$ is a propargyl group,
$R^3$ is a methyl group,
$R^4$ is phenyl substituted with one or more methoxy groups,

or pharmaceutically acceptable salts, isomers, diastereomers or enantiomers thereof.

**19.** A compound represented by formula (I) according to claim 18, wherein

$R^1$ is a propargyl group,
$R^2$ is an ethyl or hydroxypropyl group,
$R^4$ is phenyl substituted with two methoxy groups,

or pharmaceutically acceptable salts, isomers, diastereomers or enantiomers thereof.

**20.** A compound selected from the group consisting of:

3-Ethyl-8-(3,4-dimethoxyphenylethynyl)-7-methyl-1-prop-2-ynyl-3,7-dihydropurine-2,6 dione,
3-(3-Hydroxypropyl)-8-(3,4-dimethoxyphenylethynyl)-7-methyl-1-prop-2-ynyl-3,7-dihydropurine-2,6-dione,
8-[(3,4-Dimethoxyphenyl)ethynyl]-7-methyl-1-prop-2-ynyl-3,7-dihydro-1H-purine-2,6-dione.

**21.** A compound represented by formula (II)

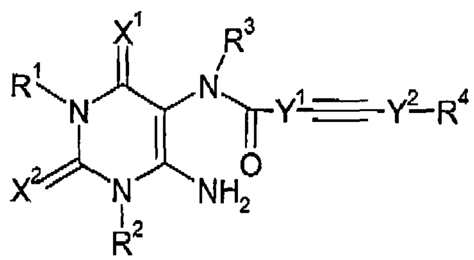

in which $X^1$, $X^2$, $Y^1$, $Y^2$, $R^1$, $R^2$, $R^3$ and $R^4$ have the meanings indicated in one of the claims 1 to 20, as well as their or pharmaceutically acceptable salts, isomers, diastereomers or enantiomers.

**22.** A compound according to claim 21, wherein $R^2$ and/or $R^3$ is a hydrogen.

**23.** Use of a compound according to claim 21 or 22 for the production of a compound according to one of the claims 1 to 20.

**24.** A taumeric form of formula (I) according to formula (III),

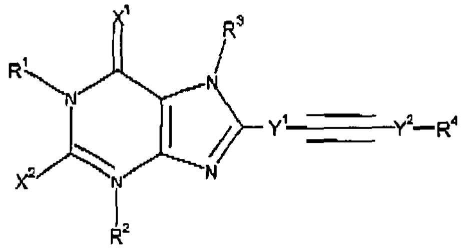

wherein, $X^1$, $Y^1$, $Y^2$, $R^1$, $R^2$, $R^3$ and $R^4$ have the meanings indicated in one of the claims 1 to 20, and wherein

$X^2$ is halogen, $(C_1-C_6)$-alkyl, $-OR^5$ or $-SR^5$, and
$R^5$ is a hydrogen, $(C_1-C_6)$-alkyl, $(C_2-C_6)$-alkenyl, $(C_2-C_6)$-alkinyl,
or
$(C_1-C_6)$-alkyl substituted in one or more places, in the same way or differently, with hydroxyl, $(C_1-C_6)$-alkoxy, aryl or-$NR^6R^7$,

or pharmaceutically acceptable salts, isomers, diastereomers or enantiomers thereof.

**25.** Process for the production of compound according to general formula (I)

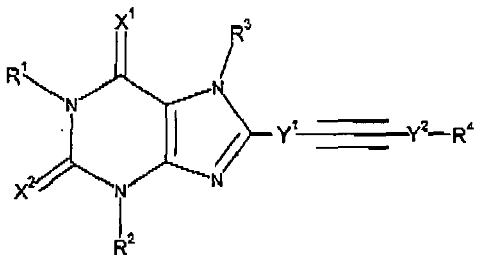

by closing the ring of an intermediate according to general formula (II)

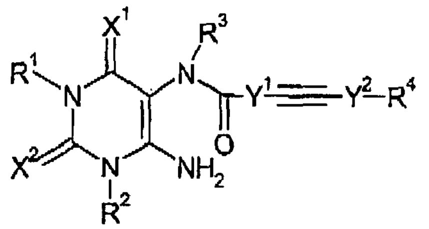

with an excess of phosphorus pentoxide at elevated temperatures,
wherein $X^1$, $X^2$, $Y^1$, $Y^2$, $R^1$ and $R^4$ have the meanings indicated in one of the claims 1 to 20, and wherein $R^2$ and/or $R^3$ are hydrogen.

**26.** Process for the production of a compound according to one of the claims 1 to 20 having at least of one substitution other than hydrogen at position $R^3$ comprising the following steps:

a) Reaction of an intermediate of general formula (I), wherein
$X^1$, $X^2$, $Y^1$, $Y^2$, $R^1$ and $R^4$ have the meanings indicated in one of the claims 1 to 20, and wherein
$R^2$ and $R^3$ are independently hydrogen,
in presence of 1.6 eq $R^3$ substituted sulfonic acid methylester, wherein
$R^3$ has the meaning indicated in one of the claims 1 to 20 and is not hydrogen, in presence of a base,
b) Optional purification of the product obtained through step a), preferably by column chromatography,
c) Optional addition of $R^2$ with an $R^2$ substituted halogenide or compound with another suitable leaving group, wherein $R^2$ has the meaning indicated in one of the claims 1 to 20 and is not hydrogen, in presence of a base.

**27.** Use of a compound according to one of the claims 1 to 20 and/or claim 24 as a medicament.

**28.** Use of a compound according to one of the claims 1 to 20 and/or claim 24 for the production of a pharmaceutical agent for preventing and/or treating diseases such as Parkinson's disease, catalepsy, dystonia, dyskinetic syndrome, restless legs syndrome, migraine, pain, dementia, neurodegenerative disorders, alcohol withdrawal and/or ischemic conditions such as e.g. stroke or cardiac ischemia.

**29.** A pharmaceutical composition comprising a compound according to one of the claims 1 to 20 and/or claim 24 and optionally comprising additional suitable pharmaceutically acceptable carriers.

**30.** A pharmaceutical composition according to claim 29 for preventing and/or treating diseases such as Parkinson's disease, catalepsy, dystonia, dyskinetic syndrome, restless legs syndrome, migraine, pain, dementia, neurodegenerative disorders, alcohol withdrawal and/or ischemic conditions such as e.g. stroke or cardiac ischemia.

**31.** Use of a compound according to one of the claims 1 to 20 and/ or claim 24 as antagonists of the $A_{2A}$ adenosine receptor.

**32.** Use of a compound according to claim 31, wherein the compounds exhibit more than 3-fold selectivity versus the $A_1$ adenosine receptor.

**33.** Use of a compound according to one of the claims 1 to 20 and/or claim 24 in the form of a pharmaceutical preparation for enteral, parenteral or oral administration.

**Figure 1:**

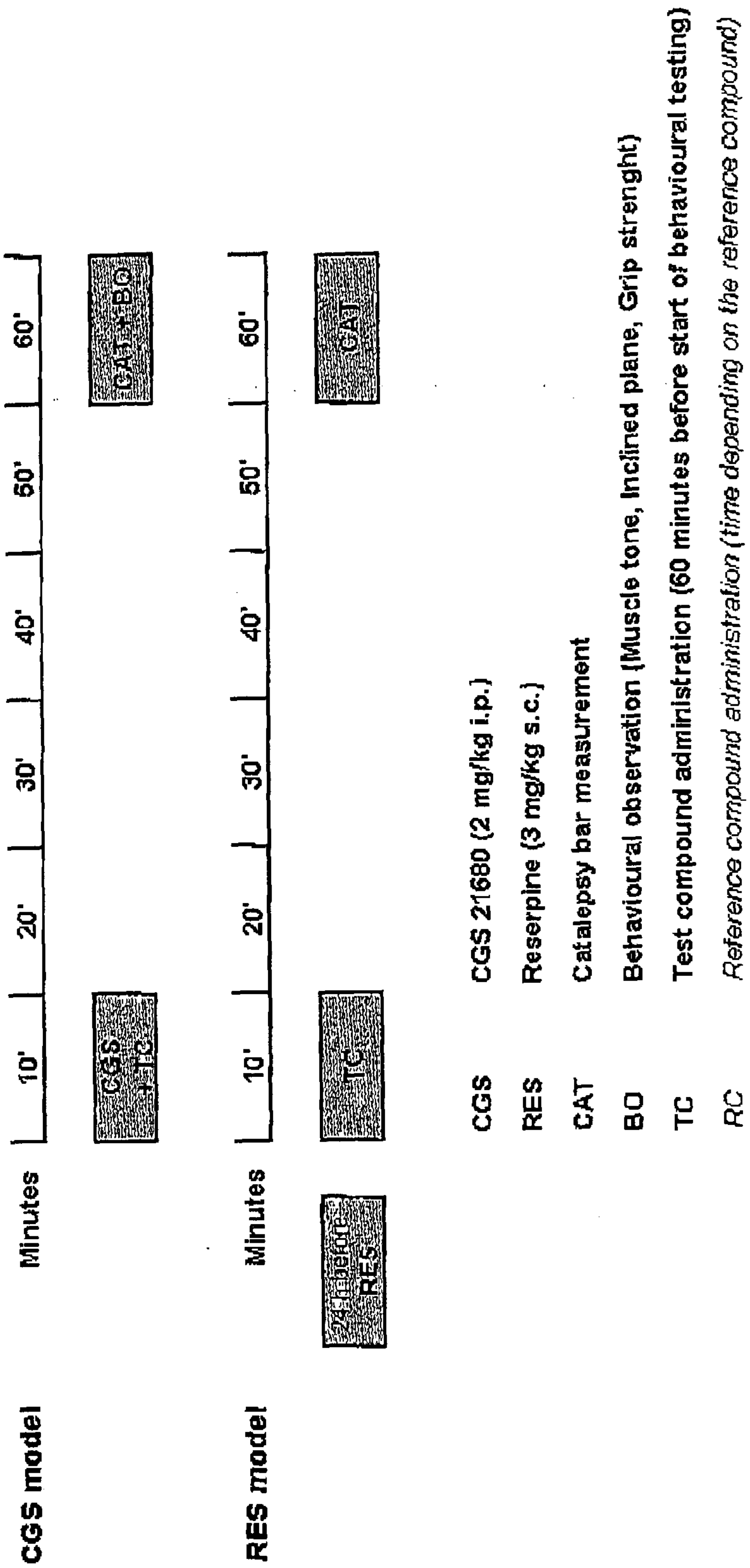
CGS model
RES model
Minutes
10'
20'
30'
40'
50'
60'
CGS +TC
CAT + BO
24h before RES
TC
CAT
CGS CGS 21680 (2 mg/kg i.p.)
RES Reserpine (3 mg/kg s.c.)
CAT Catalepsy bar measurement
BO Behavioural observation (Muscle tone, Inclined plane, Grip strenght)
TC Test compound administration (60 minutes before start of behavioural testing)
RC Reference compound administration (time depending on the reference compound)

Figure 2:

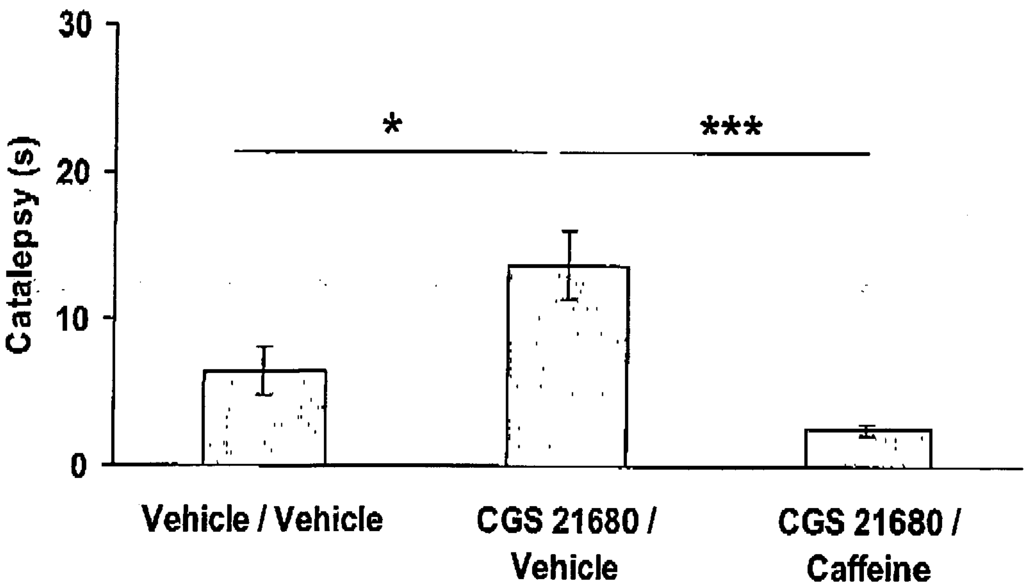
30
20
10
0
Catalepsy (s)
*
***
Vehicle / Vehicle
CGS 21680 / Vehicle
CGS 21680 / Caffeine

Figure 3:

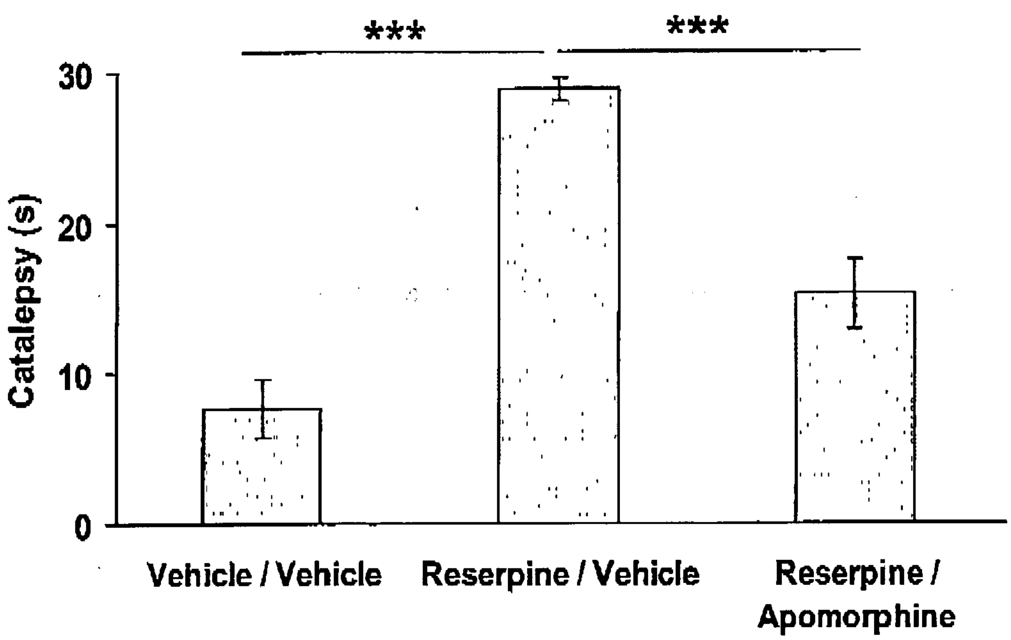
***
***
30
20
10
0
Catalepsy (s)
Vehicle / Vehicle
Reserpine / Vehicle
Reserpine / Apomorphine

**Figure 4a:**

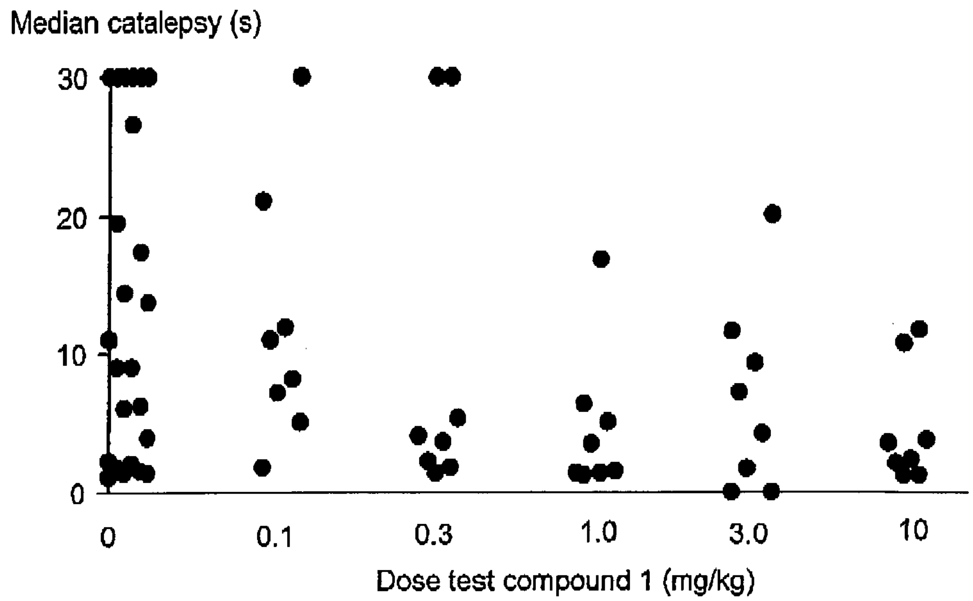


**Figure 4b:**

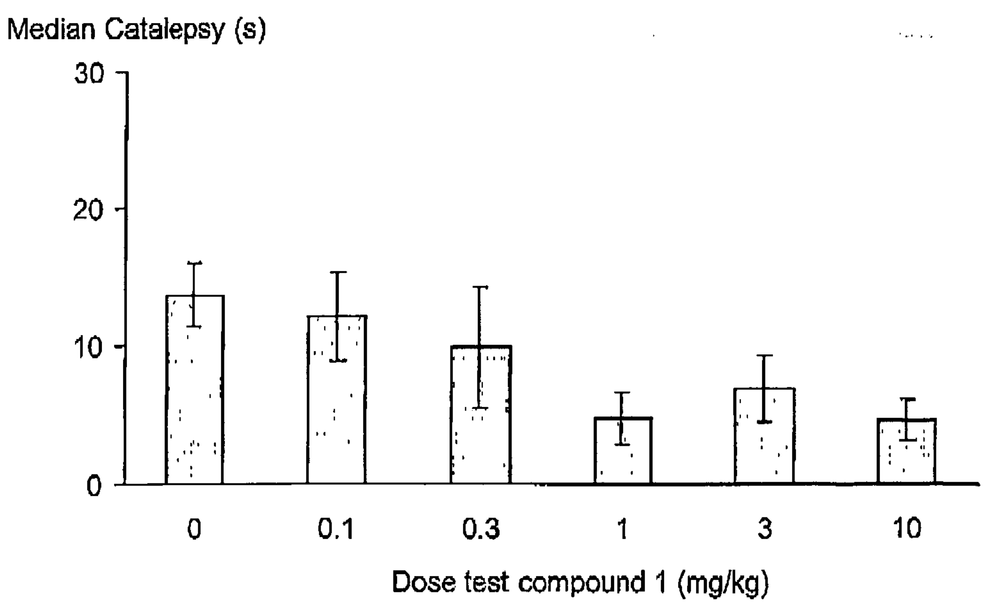

**Figure 5a:**

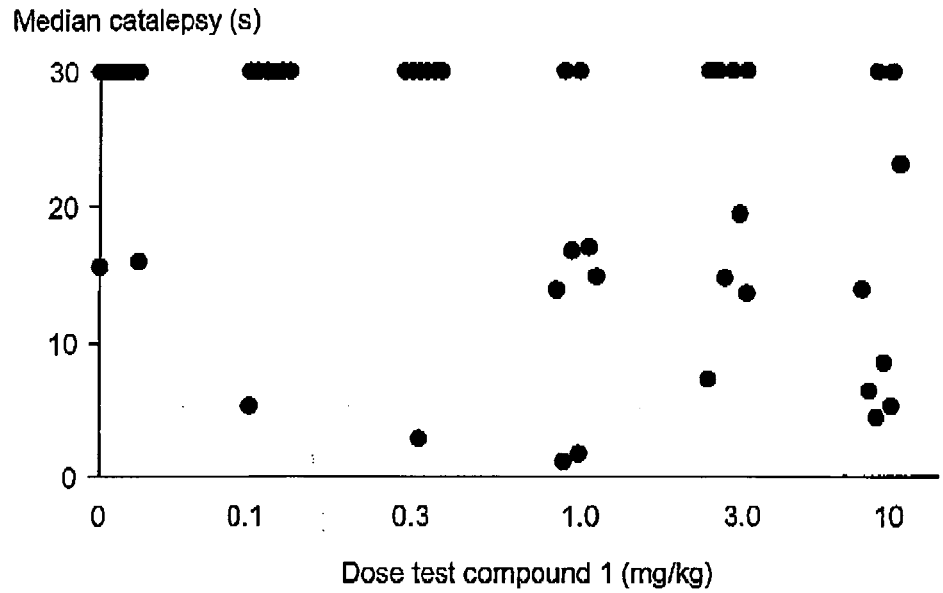


**Figure 5b:**

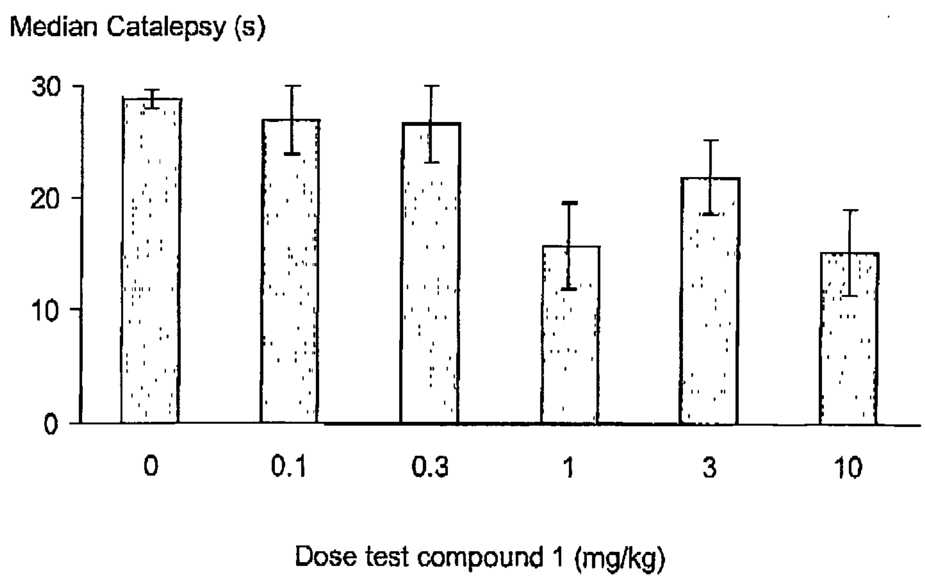

Figure 6a:

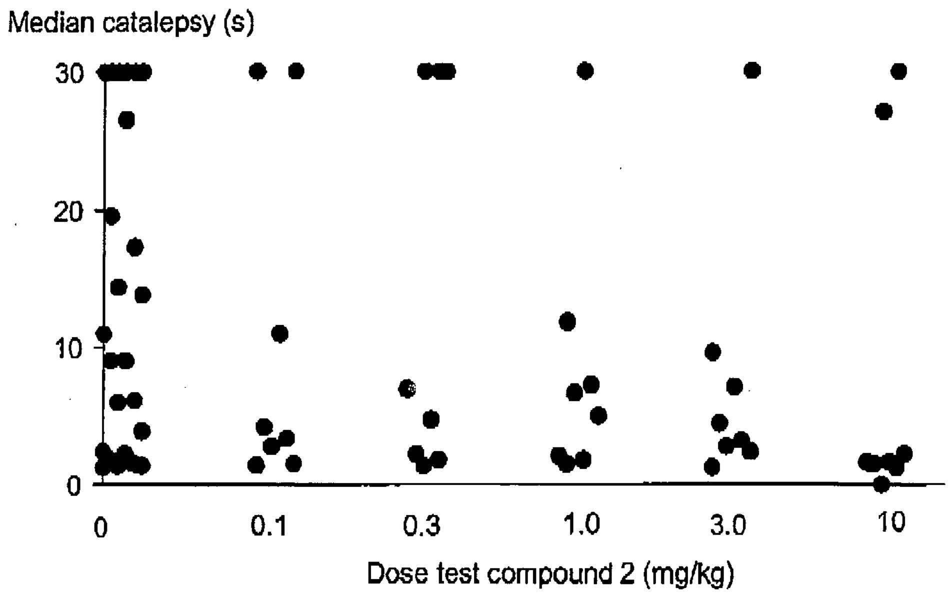


Figure 6b:

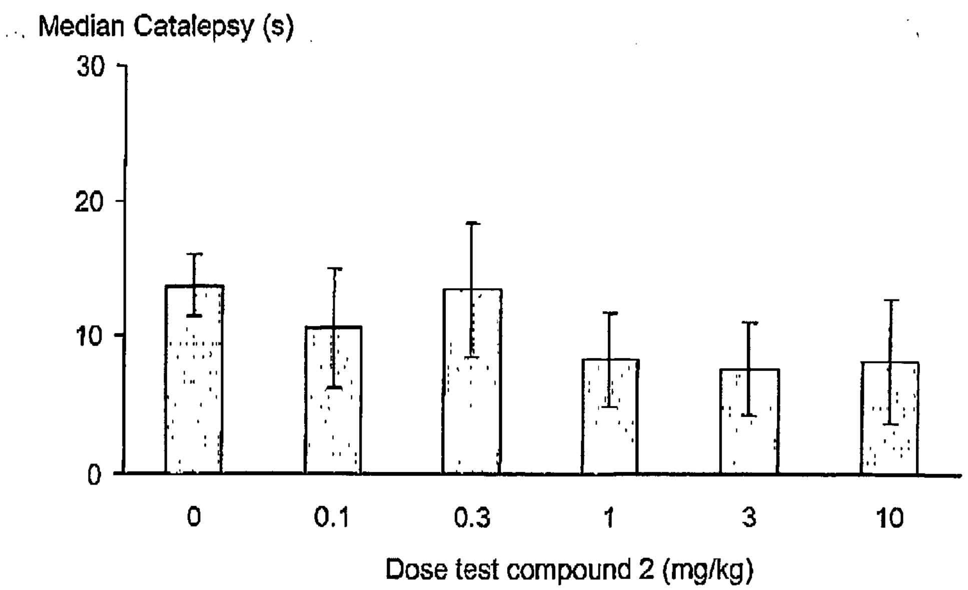

**Figure 7a:**

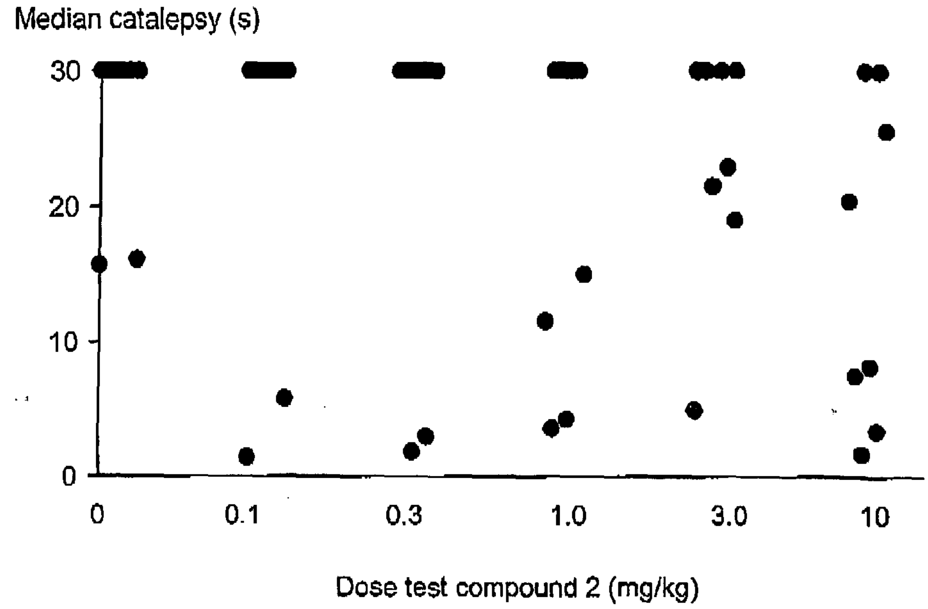


**Figure 7b:**

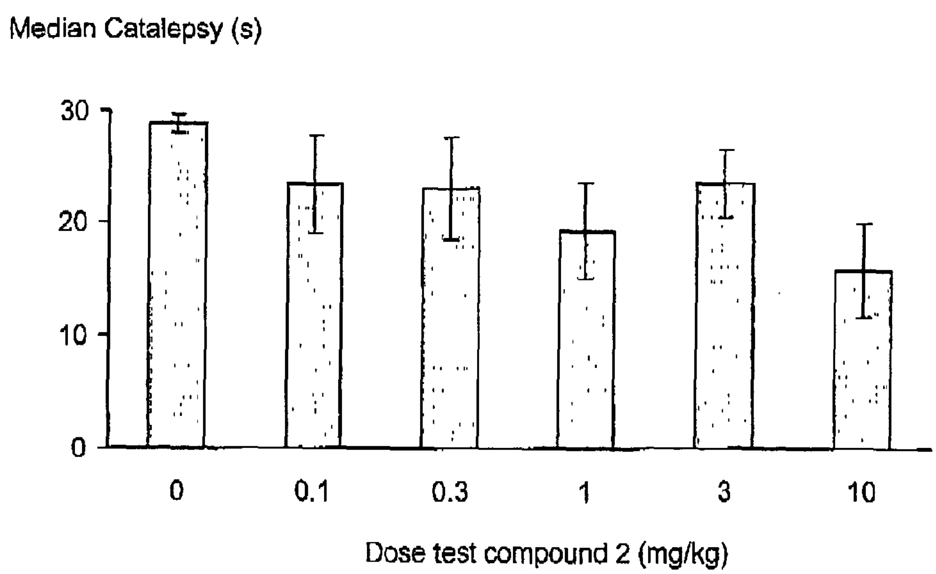

European Patent Office

# PARTIAL EUROPEAN SEARCH REPORT

which under Rule 45 of the European Patent Convention shall be considered, for the purposes of subsequent proceedings, as the European search report

Application Number
EP 06 02 6739

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MULLER C ET AL: "Configurationally stable analogs of styrylxanthines as A2A adenosine receptor antagonists" EUROPEAN JOURNAL OF MEDICINAL CHEMISTRY, EDITIONS SCIENTIFIQUE ELSEVIER, PARIS, FR, vol. 32, no. 9, September 1997 (1997-09), pages 709-719, XP004094067 ISSN: 0223-5234 * abstract; compound 21 * ----- | 1-35 | INV. C07D473/06 C07D473/04 A61K31/495 A61P25/00 |
| X | WO 2006/091896 A (ADENOSINE THERAPEUTICS LLC [US]; WANG GUOQUAN [US]; RIEGER JAYSON M [U) 31 August 2006 (2006-08-31) p. 27, 3rd structural formula ----- | 1-6, 27-30,33 | |

TECHNICAL FIELDS SEARCHED (IPC)

C07D
A61K
A61P

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| The Hague | 20 February 2007 | Fritz, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 06 02 6739

Although claims 27-28 and 31-33 are directed to a method of treatment of the human/animal body (Article 52(4) EPC), the search has been carried out and based on the alleged effects of the compounds.

-----

## ANNEX TO THE EUROPEAN SEARCH REPORT ON EUROPEAN PATENT APPLICATION NO. EP 06 02 6739

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2007

| Patent document cited in search report | Publication date | Patent family member(s) | Publication date |
|---|---|---|---|
| WO 2006091896 A | 31-08-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DALY et al.** *Cell. Mol. Neurobiol.,* 1983, vol. 3, 67
- **ONGINI et al.** *Trends Pharmacol. Sci,* 1996, vol. 17, 364
- **SHIMADA et al.** *J. Med. Chem.,* 1992, vol. 36, 2343
- **MULLER et al.** *Curr. Pharm. Des,* 1996, vol. 2, 501
- **BARALDI et al.** *Curr. Med. Chem,* 1995, vol. 2, 707
- **KIEC-KONONOWICZ et al.** *Pure and Appl. Chem,* 2001, vol. 73, 1411
- **HAUSER et al.** *Neurology,* 2003, vol. 61, 297
- **WEISS et al.** *Neurology,* 2003, vol. 61, 101
- **DEL GIUDICE et al.** *Eur. J. Med. Chem.,* 1996, vol. 31, 59
- **MULLER et al.** *J. Med. Chem.,* 1997, vol. 40, 4396
- **MULLER et al.** *Eur. J. Med. Chem.,* 1997, vol. 32, 709
- **MULLER et al.** *Drugs of the Fut,* 2000, vol. 25 (10), 1043
- **MULLER et al.** *Bioorg. Med. Chem,* 1998, vol. 6, 707
- **MULLER et al.** *Drug Dev. Res,* 1998, vol. 45, 190
- **SAUER et al.** *J. Med. Chem.,* 2000, vol. 43, 440
- **ASPINAL.** The Polysaccarides. Academic Press, 1982
- **FERRÉ S. et al.** *Neurosci. Let,* 1991, vol. 130, 162
- **FERRÉ S. et al.** *Neuroscience,* 1992, vol. 51, 501
- **KAFKA S.H. et al.** *Eur. J. Pharma col,* 1996, vol. 295, 147
- **RIMONDINI R. et al.** *Neuropsychopharmacology,* 1997, vol. 17, 82
- **MULLER et al.** *Synthesis,* 1998, 1428
- **MULLER C.E. et al.** *Eur. J. Med. Chem.,* 1997, vol. 32, 709-719
- **MULLER C.E et al.** *Eur. J. Med. Chem.,* 1997, vol. 32, 709-719
- **BURBIEL et al.** *Beilstein J. Org. Chem.,* 2006, vol. 6, 1375
- **MULLER.** *Tetrahedron Lett.,* 1991, vol. 32, 6539
- **MULLER et al.** *J. Med. Chem.,* 1993, vol. 36, 3341
- **HOCKEMEYER et al.** *J. Org. Chem.,* 2004, vol. 69, 3308
- **MULLER et al.** *Curr. Pharm Des,* 1996, vol. 2, 501
- **WEYLER et al.** *ChemMedChem,* 2006, vol. 1, 891
- **RIVKEES S.A et al.** *J. Biol. Chem.,* 1995, vol. 270, 20485
- **LUTHIN D.R. et al.** *Mol. Pharmacol.,* 1995, vol. 47
- **STEHLE J.H et al.** *Mol. Endocrinol,* 1992, vol. 6, 384
- **SALVATORE C.A. et al.** *Proc. Natl. Acad. Sci. USA,* 1993, vol. 90, 10365
- **GAO et al.** *Biochem. Pharmacol.,* 2000, vol. 60, 669
- **IRWIN S.** *Psychopharmacologia,* 1968, vol. 13, 222
- **WARBURTON D.M.** *Psychopharmacology,* 2002, vol. 163, 4
- **HAGGERTY G. C et al.** *J. Amer. Coll Toxicol,* 1991, vol. 10, 677
- **MATTSSON J.L. et al.** *J., Amer. Coll. Toxicol,* 1996, vol. 15, 239
- **KAFKA S.H. et al.** *Eur. J. Pharmacol.,* 1996, vol. 295, 147
- **RIMONDINI R. et al.** *Neuropsychopharma cology,* 1997, vol. 17, 82